(19) Europäisches Patentamt
European Patent Office
Office européen des brevets

(11) EP 3 220 956 B1

(12) # EUROPEAN PATENT SPECIFICATION

(45) Date of publication and mention of the grant of the patent:
**09.08.2023 Bulletin 2023/32**

(21) Application number: **15782051.5**

(22) Date of filing: **08.10.2015**

(51) International Patent Classification (IPC):
***A61K 47/60*** *(2017.01)* ***A61K 47/68*** *(2017.01)*
***A61P 35/00*** *(2006.01)*

(52) Cooperative Patent Classification (CPC):
**A61K 31/33; A61K 47/60; A61K 47/68; A61K 47/6851; A61K 47/6889; A61P 35/00**

(86) International application number:
**PCT/GB2015/052953**

(87) International publication number:
**WO 2016/063006 (28.04.2016 Gazette 2016/17)**

(54) **CONJUGATES AND CONJUGATING REAGENTS**
KONJUGATE UND KONJUGIERUNGSREAGENZIEN
CONJUGUÉS ET RÉACTIFS DE CONJUGAISON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2014 GB 201418986**
**24.10.2014 GB 201418989**
**24.10.2014 GB 201418984**

(43) Date of publication of application:
**27.09.2017 Bulletin 2017/39**

(73) Proprietor: **Abzena (UK) Limited**
**Cambridge, Cambridgeshire CB22 3AT (GB)**

(72) Inventors:
- **GODWIN, Antony**
**Cambridge CB22 3AT (GB)**
- **FRIGERIO, Mark**
**Cambridge CB22 3AT (GB)**

(74) Representative: **Abel & Imray LLP**
**Westpoint Building**
**James Street West**
**Bath BA1 2DA (GB)**

(56) References cited:
**WO-A1-99/45964**
**WO-A1-2011/077067**
**WO-A1-2014/064423**
**WO-A2-2013/190292**
**US-A1- 2013 338 231**

- **SIMONE KRAKERT ET AL: "Adjustment of the bioresistivity by electron irradiation: self-assembled monolayers of oligo(ethyleneglycol)-terminated alkanethiols with embedded cleavable group", PHYSICAL CHEMISTRY CHEMICAL PHYSICS., vol. 12, no. 2, 1 January 2010 (2010-01-01), pages 507-515, XP055237477, GB ISSN: 1463-9076, DOI: 10.1039/B915036F**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 220 956 B1

## Description

### Field of Invention

This invention relates to conjugates and conjugating reagents as defined in the claims.

### Background of the invention

Much research has been devoted in recent years to the conjugation of a wide variety of payloads, for example therapeutic, diagnostic and labelling agents, to peptides and proteins for a wide range of applications. The protein or peptide itself may have therapeutic properties, and/or it may be a binding protein.

Peptides and proteins have potential use as therapeutic agents, and conjugation is one way of improving their properties. For example, water soluble, synthetic polymers, particularly polyalkylene glycols, are widely used to conjugate therapeutically active peptides or proteins.

These therapeutic conjugates have been shown to alter pharmacokinetics favourably by prolonging circulation time and decreasing clearance rates, decreasing systemic toxicity, and in several cases, displaying increased clinical efficacy. The process of covalently conjugating polyethylene glycol, PEG, to proteins is commonly known as "PEGylation". The PEG chain may carry a payload, for example a therapeutic, diagnostic or labelling agent.

Binding proteins, particularly antibodies or antibody fragments, are frequently conjugated. The specificity of binding proteins for specific markers on the surface of target cells and molecules has led to their extensive use either as therapeutic or diagnostic agents in their own right or as carriers for payloads which may include therapeutic, diagnostic or labelling agents. Such proteins conjugated to labels and reporter groups such as fluorophores, radioisotopes and enzymes find use in labelling and imaging applications, while conjugation to drugs such as cytotoxic agents and chemotherapy drugs to produce antibody-drug conjugates (ADCs) allows targeted delivery of such agents to specific tissues or structures, for example particular cell types or growth factors, minimising the impact on normal, healthy tissue and significantly reducing the side effects associated with chemotherapy treatments. Such conjugates have extensive potential therapeutic applications in several disease areas, particularly in cancer. Conjugates containing binding proteins frequently contain PEG.

Many methods of conjugating proteins and peptides have been reported in the literature. Probably the most commonly used process involves the use of conjugating reagents based on maleimides. Such reagents are described in many publications, for example WO 2004/060965. An alternative approach which leads to more homogeneous products is described by Liberatore et al, Bioconj. Chem 1990, 1, 36-50, and del Rosario et al, Bioconj. Chem. 1990, 1, 51-59, which describe the use of reagents which may be used to cross-link across the disulfide bonds in proteins, including antibodies. WO 2005/007197 describes a process for the conjugation of polymers to proteins, using novel conjugating reagents having the ability to conjugate with both sulfur atoms derived from a disulfide bond in a protein to give novel thioether conjugates, while WO 2009/047500 describes the use of the same conjugating reagents to bond to polyhistidine tags attached to the protein. WO 2010/000393 describes reagents capable of forming a single carbon bridge across the disulfide bond in a protein. Other documents relating to the conjugation of proteins include WO 2014/064423, WO 2013/190292, WO 2013/190272, EP 2260873, US 2013/338231 and WO 2011/077067.

WO 2014/064424 describes specific ADCs in which the drug is a maytansine and the antibody is bonded by cross-linking across a disulfide bond. WO 2014/064423 describes specific ADCs in which the drug is an auristatin and the antibody is bonded by cross-linking across a disulfide bond. The linkers illustrated in the Examples of these documents contain a PEG portion in which one end of the PEG chain is attached via a further portion of the linker to the drug, while the other end of the PEG chain is attached via a further portion of the linker to the antibody. This is a common structural pattern for ADCs.

Over recent years, the importance of the linker which links a payload to the protein or peptide in a conjugate, has become apparent. Often, the key decision to be taken whether it is desired to have a cleavable linker, i.e. a linker which, on administration of the conjugate, degrades to release the free payload, or a non-cleavable linker. Another key decision is whether or not to include PEG in the linker. Subject to these considerations, in principle, any linker may be used. In practice, however, changes in structure of the linker may lead to differences in the properties either of the conjugating reagent or of the resulting conjugate.

One problem frequently found is that conjugates may be less storage stable than desired. This is particularly true when a cleavable linker is used, when it is desired that the conjugate should have a long shelf-life before administration but should then rapidly cleave on application, but it can be true for any linker. There is a need to increase the storage stability of conjugates. In addition, improved stability *in vivo* is desirable, as this can lead to increased biological activity. We have now found that for a particular class of conjugate, the use of PEG-containing linkers of a particular structure gives increased storage stability. Further, and very surprisingly, the conjugates have increased biological activity.

## Summary of the invention

The invention provides a conjugate of a protein or peptide with a therapeutic agent, said conjugate containing a protein or peptide bonding portion and having a linker which connects the therapeutic agent to the protein or peptide bonding portion, said linker including a polyethylene glycol portion; in which said protein or peptide bonding portion has the general formula:

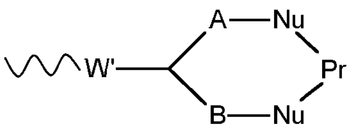

(I)

in which:

Pr represents said protein or peptide;

each Nu represents a sulfur atom or an amine group provided by a cysteine, lysine or histidine residue present in the protein or peptide Pr; or each Nu represents an imidazole group present in a polyhistidine tag attached to the protein or peptide Pr;

each of A and B independently represents a $C_{1-4}$alkylene or alkenylene chain; and

W' represents a keto group or a CH.OH group;

said polyethylene glycol portion is or includes a pendant polyethylene glycol chain which has a terminal end group of formula $-CH_2CH_2OR$, in which R represents a hydrogen atom, an alkyl group, or an optionally substituted aryl group, and wherein said pendant polyethylene glycol chain has a number average molecular weight of up to 75,000 g/mole;

the linker includes the grouping:

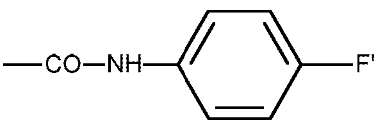

(Va) or

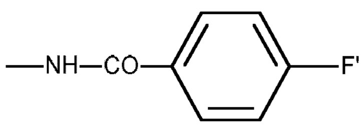

(Vb)

in which F' represents said protein or peptide bonding portion of formula I; and the protein or peptide is a receptor or ligand binding protein, an antibody or antibody fragment.

The invention also provides a conjugating reagent capable of reacting with a protein or peptide, and including a therapeutic agent, said reagent having a linker which connects the therapeutic agent to a functional grouping, said linker including a polyethylene glycol portion; said functional grouping having the formula:

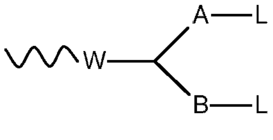

(II) or

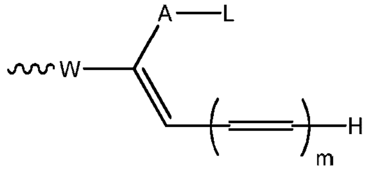

(II')

in which:

W represents a keto group or a CH.OH group;

each of A and B independently represents a $C_{1-4}$alkylene or alkenylene chain;

each L independently represents a leaving group;

said polyethylene glycol portion is or includes a pendant polyethylene glycol chain which has a terminal end group of formula $-CH_2CH_2OR$, in which R represents a hydrogen atom, an alkyl group, or an optionally substituted aryl group, and wherein

said pendant polyethylene glycol chain has a number average molecular weight of up to 75,000 g/mole;

the linker includes the grouping:

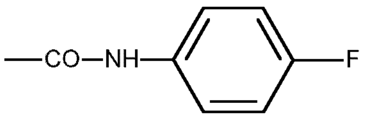

(VIa) or

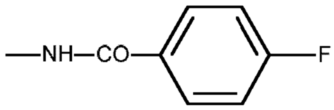

(VIb)

in which F represents the functional grouping of formula II or II'.

The invention also provides a process for the preparation of a conjugate according to the invention, which comprises reacting a protein or peptide with a conjugating reagent according to the invention.

**Detailed description of the invention**

The conjugate of the invention may be represented schematically by the formula:

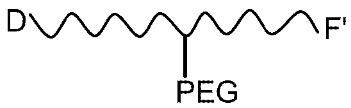

(III)

in which D represents the therapeutic, diagnostic or labelling agent, F' represents the group of formula I, and PEG represents the pendant polyethylene glycol chain having a terminal end group of formula $-CH_2CH_2OR$.

The reagent of the invention may be represented schematically by the formula:

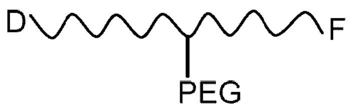

(IV)

in which D represents the therapeutic, diagnostic or labelling agent, F represents the group of formula II or II', and PEG represents a pendant polyethylene glycol chain having a terminal end group of formula $-CH_2CH_2OR$. The functional grouping F is capable of reacting with two nucleophiles present in a protein or peptide as explained below.

**The polyethylene glycol portion**

A polyethylene glycol (PEG) portion of the conjugates and reagents of the invention is or includes a pendant PEG chain which has a terminal end group of formula $-CH_2CH_2OR$ in which R represents a hydrogen atom, an alkyl group, for example a $C_{1-4}$alkyl group, especially a methyl group, or an optionally substituted aryl group, especially a phenyl group, especially an unsubstituted phenyl group. Preferably R is a methyl group or a hydrogen atom.

The PEG portion may include a single pendant PEG chain as defined above, or it may include two or more, for example two or three, pendant PEG chains.

The overall size of the PEG portion will of course depend on the intended application. For some applications, high molecular weight PEGs may be used, for example the number average molecular weight may be up to around 75,000, for example up to 50,000, 40,000 or 30,000 g/mole. For example, the number average molecular weight may be in the range of from 500 g/mole to around 75,000. However, smaller PEG portions may be preferred for some applications.

In one preferred embodiment, all of the PEG in the PEG portion is present in one or more pendant PEG chains. In another embodiment, PEG may also be present in the backbone of the molecule, and this is discussed in more detail below.

As with the PEG portion, the size of the pendant PEG chain or chains will depend on the intended application. For some applications, high molecular weight pendant PEG chains may be used, for example the number average molecular weight may be up to around 75,000, for example up to 50,000, 40,000 or 30,000 g/mole. For example, the number average molecular weight may be in the range of from 500 g/mole to around 75,000. However, for many applications, smaller pendant PEG chains may be used. For example said PEG chain may have a molecular weight up to 3,000 g/mole. However, very small oligomers, consisting of discrete PEG chains with, for example, as few as 2 repeat units, for example from 2 to 50 repeat units, are useful for some applications, and are present as a pendant PEG chain in one preferred embodiment of the invention. A pendant PEG chain may be straight-chain or branched. PEG chains, for example straight-chain or branched chains with 12, 20, 24, 36, 40 or 48 repeat units may for example be used.

**The payload**

The conjugates and reagents disclosed herein carry a payload which is a therapeutic, diagnostic or labelling agent. A single molecule of a therapeutic, diagnostic or labelling agent may be present, or two or more molecules may be present. The inclusion of one or more drug molecules, for example a cytotoxic agent or a toxin, is preferred. Auristatins and maytansinoids are typical cytotoxic drugs. It is often preferred that drug conjugates, particularly antibody drug conjugates, should contain multiple copies of the drug. Labelling agents (which should be understood to include imaging agents) may for example include a radionuclide, a fluorescent agent (for example an amine derivatised fluorescent probe such as 5-dimethylaminonaphthalene-1-(N-(2-aminoethyl))sulfonamide-dansyl ethylenediamine, Oregon Green® 488 cadaverine (catalogue number 0-10465, Molecular Probes), dansyl cadaverine, N-(2-aminoethyl)-4-amino-3,6-disulfo-1,8-naphthalimide, dipotassium salt (lucifer yellow ethylenediamine), or rhodamine B ethylenediamine (catalogue number L 2424, Molecular Probes), or a thiol derivatised fluorescent probe for example BODIPY® FL L-cystine (catalogue number B-20340, Molecular Probes). Biotin may also be used.

Our copending application GB 1418984 provides a conjugate comprising a protein, peptide and/or polymer attached to a maytansine-containing payload via a linker, a conjugating reagent useful in forming such conjugates and a maytansine-containing compound for use as payload. The maytansine-containing payloads and compounds consist of at least two maytansine moieties linked to each other through a non-degradable bridging group. These maytansines may be used as payloads in the present invention, and the reagents and conjugates form one aspect of the present invention. Our copending application discloses the following:

"The present invention provides in a first aspect a maytansine-containing compound, in which at least two maytansine moieties (D) are linked to each other through a bridging group (Bd). The bridging group (Bd) is non-degradable under physiological conditions. Advantageously, the bridging group (Bd) has at least 3 chain carbon atoms and optionally contains polyethylene glycol) spacers in addition to the 3 chain carbon atoms. Advantageously, no two heteroatoms are adjacent to one another in the bridging group. Advantageously, the bridging group does not include the moiety: $-C(O)-CH(NR^1X)-(CH_2)_b-C(O)-$, where b is 1, 2 or 3, $R^1$ is selected from hydrogen and $C_1$ to $C_6$ alkyl, and X is any group. The maytansine-containing compound of the first aspect, in which two maytansine moieties are present, may be represented by the following formula (I):

D-Bd-D (I)

In a second aspect, the invention provides a conjugating reagent, which contains a functional group capable of reaction with a peptide or protein and/or a functional group capable of reacting with a polymer, the payload being attached to the functional group(s) *via* one or more linkers, characterised in that the conjugation reagent comprises a maytansine-containing payload consisting of at least two maytansine moieties linked to each other through a non-degradable bridging group, with the proviso that when the conjugating reagent comprises a functional group capable of reaction with at a peptide or protein, the linker attaching the payload to the functional group capable of reaction with at a peptide or protein is degradable. When the conjugating reagent contains a functional group capable of reaction with at least one nucleophile present in a peptide or protein, the functional group including at least one leaving group which is lost on reaction with said nucleophile, the maytansine-containing payload consisting of at least two maytansine moieties linked to each other through a non-degradable bridging group is advantageously attached to the functional group capable of reaction with at least one nucleophile present in a peptide or protein *via* a degradable linker. The conjugating reagent optionally contains a functional group capable of reaction with at least one nucleophile present in a peptide or protein, the functional group advantageously including at least one leaving group which is lost on reaction with said nucleophile, characterised in that the conjugation reagent comprises a

maytansine-containing payload consisting of at least two maytansine moieties, especially two maytansine moieties, linked to each other through a non-degradable bridging group, and in that the payload is attached to the functional group capable of reaction with at least one nucleophile present in a peptide or protein *via* a linker, especially a degradable linker. The linker is suitable for linking the bridging group to a protein or peptide capable of binding to a partner or target. Preferably, the bridging group (Bd) of the maytansine-containing payload ($D_2Bd$) is connected to a degradable linker ($Lk^d$) that includes a degradable group which breaks under physiological conditions. The degradable group may, for example, be sensitive to hydrolytic conditions, especially acidic conditions; be susceptible to degradation under reducing conditions; or be susceptible to enzymatic degradation.

The maytansine-containing conjugating reagent of the second aspect, in which two maytansine moieties are present, may be represented by the following formula (II):

$$D_2Bd\text{-}Lk\text{-}F \qquad (II)$$

in which $D_2Bd$ represents a maytansine-containing payload consisting of two maytansine moieties linked to each other through a non-degradable bridging group, Lk is a linker, especially a degradable linker ($Lk^d$), and F represents a functional group capable of reaction with a peptide or protein and/or a functional group capable of reacting with a polymer.

The present invention further provides a process for the conjugation of a peptide, protein and/or a polymer, which comprises reacting said peptide, protein and/or a polymer with a conjugating reagent of the second aspect of the invention. When the conjugating reagent is reacted with a peptide or polymer, said conjugating reagent is advantageously capable of reaction with at least one nucleophile present in said peptide or protein, said reagent advantageously containing at least one leaving group which is lost on reaction with said nucleophile.

The invention also provides in a third aspect a conjugate comprising a protein, peptide and/or polymer attached to a maytansine-containing payload *via* a linker, characterised in that the maytansine-containing payload consists of at least two maytansine moieties linked to each other through a non-degradable bridging group. When the conjugate comprises a protein or peptide, the linker attaching the payload to the protein or peptide is advantageously degradable. The conjugate of the third aspect of the invention may, for example may, for example, comprise a maytansine-containing drug moiety ($D_2Bd$) linked *via* a linker (Lk), especially a degradable linker ($Lk^d$), to a protein or peptide (Ab) capable of binding to a partner or target, wherein the maytansine-containing drug moiety ($D_2Bd$) comprises at least two maytansine moieties (D) linked to each other through a non-degradable bridging group (Bd). A maytansine-containing conjugate of the third aspect of the invention, in which two maytansine moieties (D) are present, may be represented by the following formula (III):

$$D_2Bd\text{-}Lk\text{-}Ab \qquad (III)$$

The linker (Lk) is advantageously a degradable linker ($Lk^d$) that includes a degradable group which cleaves under physiological conditions separating the maytansine-containing drug moiety ($D_2Bd$) comprising at least two maytansine moieties (D) linked to each other through a bridging group (Bd) from the protein or peptide (Ab) capable of binding to a partner or target. The degradable group may, for example, be sensitive to hydrolytic conditions, especially acidic conditions; be susceptible to degradation under reducing conditions; or be susceptible to enzymatic degradation. The non-degradable bridging group (Bd) contains no groups that are susceptible to cleavage under the same conditions as those under which the degradable group in the degradable linker cleaves.

The maytansine-containing compound of the first aspect of the invention, may, for example, comprise or consist of at least two maytansine moieties (D), especially two maytansine moieties, linked to each other through a bridging group (Bd) having at least 3 chain carbon atoms, especially at least 7 chain carbon atoms, and optional polyethylene glycol) units in addition to the chain carbon atoms, with the proviso that no two heteroatoms are adjacent to one another in the bridging group and with the proviso that the bridging group does not include the moiety: $-C(O)-CH(NR^1X)-(CH_2)_b-C(O)-$, where b is 1, 2 or 3, $R^1$ is selected from hydrogen and $C_1$ to $C_6$ alkyl, and X is any group. Optionally, the bridging group incorporates from 0 to 8 carbonyl groups, especially from 2 to 8 carbonyl groups. The bridging group optionally incorporates from 0 to 4 unsaturated carbon-carbon double bonds; and/or from 0 to 4 $C_3$ to $C_{10}$ aryl or heteroaryl groups in the chain. Optionally, the chain is interspersed with from 0 to 11, especially from 2 to 11, chain heteroatoms selected from N, O and S, with the proviso that no two heteroatoms are adjacent to one another. Advantageously, a chain carbon atoms in the bridging group is substituted with a pendant connecting group selected from amine, carboxy, alkyne, azide, hydroxyl or thiol. Advantageously the bridging group

includes at least one amide linkage in the chain."

Preferably the payload is a therapeutic agent, especially one of those mentioned above.

**The protein**

For convenience in this section and elsewhere, "protein" should be understood to include "protein and peptide" except where the context requires otherwise.

Suitable proteins which may be present in the conjugates of the invention include for example peptides, polypeptides, antibodies, antibody fragments, enzymes, cytokines, chemokines, receptors, blood factors, peptide hormones, toxin, transcription proteins, or multimeric proteins.

Enzymes include carbohydrate-specific enzymes, proteolytic enzymes and the like, for example the oxidoreductases, transferases, hydrolases, lyases, isomerases and ligases disclosed by US 4,179,337. Specific enzymes of interest include asparaginase, arginase, adenosine deaminase, superoxide dismutase, catalase, chymotrypsin, lipase, uricase, bilirubin oxidase, glucose oxidase, glucuronidase, galactosidase, glucocerbrosidase, glucuronidase, and glutaminase.

Blood proteins include albumin, transferrin, Factor VII, Factor VIII or Factor IX, von Willebrand factor, insulin, ACTH, glucagen, somatostatin, somatotropins, thymosin, parathyroid hormone, pigmentary hormones, somatomedins, erythropoietin, luteinizing hormone, hypothalamic releasing factors, antidiuretic hormones, prolactin, interleukins, interferons, for example IFN-$\alpha$ or IFN-$\beta$, colony stimulating factors, hemoglobin, cytokines, antibodies, antibody fragments, chorionicgonadotropin, follicle-stimulating hormone, thyroid stimulating hormone and tissue plasminogen activator.

Other proteins of interest are allergen proteins disclosed by Dreborg et al Crit. Rev. Therap. Drug Carrier Syst. (1990) 6 315-365 as having reduced allergenicity when conjugated with a polymer such as poly(alkylene oxide) and consequently are suitable for use as tolerance inducers. Among the allergens disclosed are Ragweed antigen E, honeybee venom, mite allergen and the like.

Glycopolypeptides such as immunoglobulins, ovalbumin, lipase, glucocerebrosidase, lectins, tissue plasminogen activator and glycosylated interleukins, interferons and colony stimulating factors are of interest, as are immunoglobulins such as IgG, IgE, IgM, IgA, IgD and fragments thereof.

Of particular interest are receptor and ligand binding proteins and antibodies and antibody fragments which are used in clinical medicine for diagnostic and therapeutic purposes. Antibody-drug conjugates, especially where the drug is a cytotoxic drug, for example an auristatin or a maytansinoid, are an especially preferred embodiment of the invention.

The protein may be derivatised or functionalised if desired. In particular, prior to conjugation, the protein, for example a native protein, may have been reacted with various blocking groups to protect sensitive groups thereon; or it may have been previously conjugated with one or more polymers or other molecules. It may contain a polyhistidine tag, which during the conjugation reaction can be targeted by the conjugating reagent.

Bonding of the protein or peptide, and conjugating reagents

The conjugating reagents of the invention are of the general type disclosed in WO 2005/007197 and WO 2010/000393. The functional groupings II and II' are chemical equivalents of each other. When a reagent containing a group II reacts with a protein, a first leaving group L is lost to form *in situ* a conjugating reagent containing a group II' which reacts with a first nucleophile. The second leaving group L is then lost, and reaction with a second nucleophile occurs. Thus as an alternative to using a reagent containing the functional grouping II as starting material, reagents containing the functional grouping II' may be used as starting material.

A leaving group L may for example be -SP, -OP, $-SO_2P$, $-OSO_2P$, $-N^+PR^2R^3$, halogen, or -OØ, in which P represents a hydrogen atom or an alkyl (preferably $C_{1-6}$alkyl), aryl (preferably phenyl), or alkyl-aryl (preferably $C_{1-6}$alkyl-phenyl) group, or is a group which includes a portion $-(CH_2CH_2O)_n-$ in which n is a number of two or more, and each of $R^2$ and $R^3$ independently represents a hydrogen atom, a $C_{1-4}$alkyl group, or a group P, and Ø represents a substituted aryl, especially phenyl, group, containing at least one electron withdrawing substituent, for example -CN,$-NO_2$, $-CO_2R^a$, -COH, $-CH_2OH$, $-COR^a$, $-OR^a$, $-OCOR^a$, $-OCO_2R^a$, $-SR^a$, $-SOR^a$, $-SO_2R^a$, -NHCO $R^a$, $-NR^a$, $COR^a$, $-NHCO_2R^a$, $-NPCO_2R^a$, -NO, -NHOH, $-NR^a$ OH, $-C=N-NHCOR^a$, $-C=N-NR^a$ $COR^a$, $-N^+R^a{}_3$, $-N^+HR^a{}_2$, $-N^+H_2R^a$, halogen, especially chlorine or, especially, fluorine, $-C\equiv CR^a$, $-C=CR^a{}_2$ and $-C=CHR^a$, in which each $R^a$ represents a hydrogen atom or an alkyl (preferably $C_{1-6}$alkyl), aryl (preferably phenyl), or alkyl-aryl (preferably $C_{1-6}$alkyl-phenyl) group.

Conjugating reagents in which P represents a group which includes a portion $-(CH_2CH_2O)_n-$ in which n is a number of two or more are the subject of our copending application GB 1418186. This application discloses the following:

"The leaving group may for example include $-(CH_2CH_2O)_n-R^1$ where $R^1$ is a capping group. A very wide range of

capping groups may be used. $R^1$ may for example be a hydrogen atom, an alkyl group, especially a $C_{1-4}$alkyl group, particularly a methyl group, or an optionally substituted aryl group, for example an optionally substituted phenyl group, for example a tolyl group. Alternatively, the capping group may include a functional group such as a carboxyl group or an amine group. Such capping groups may for example have the formula $-CH_2CH_2CO_2H$ or $-CH_2CH_2NH_2$, and may be prepared by functionalising the terminal unit of a $-(CH_2CH_2O)_n-$ chain. Alternatively, rather than being terminated by a capping group, the $-(CH_2CH_2O)_n-$ group may have two points of attachment within the conjugating reagent such that chemically the equivalent of two leaving groups are present, capable of reacting with two nucleophiles.

The $-(CH_2CH_2O)_n-$ portion of the leaving group is based on PEG, polyethylene glycol. The PEG may be straight-chain or branched, and it may be derivatised or functionalised in any way. n is a number of 2 or more, for example 2, 3, 4, 5, 6, 7, 8, 9 or 10. For example, n may be from 5 to 9. Alternatively, n may be a number of 10 or more. There is no particular upper limit for n. n may for example be 150 or less, for example 120 or less, for example 100 or less. For example n may be from 2 to 150, for example from 7 to 150, for example from 7 to 120. The PEG portion $-(CH_2CH_2O)_n-$ of a leaving group may for example have a molecular weight of from 1 to 5 kDa; it may for example be 1kDa, 2kDa, 3kDa, 4kDa or 5kDa. A leaving group may if desired contain two or more portions $-(CH_2CH_2O)_n-$ separated by one or more spacers.

A leaving group in a conjugating reagent according to the invention is suitably of the formula -SP, -OP, $-SO_2P$, $-OSO_2P$, $-N^+PR^2R^3$, in which P is a group which includes a portion $-(CH_2CH_2O)_n-$ and each of $R^2$ and $R^3$ independently represents a hydrogen atom, a $C_{1-4}$alkyl group, or a group P. Preferably each of $R^2$ and $R^3$ represents a $C_{1-4}$alkyl group, especially a methyl group, or, especially, a hydrogen atom. Alternatively, the conjugating reagent may include a group of formula -S-P-S-; -O-P-O-; $-SO_2-P-SO_2-$; $-OSO_2-P-OSO_2-$; and - $N^+R^2R^3-P-N^+R^2R^3-$. Specific groups of this type include $-S-(CH_2CH_2O)_n-S-$, $-O-(CH_2CH_2O)_n-O-$; $-SO_2-(CH_2CH_2O)_n-SO_2-$; $-OSO_2-(CH_2CH_2O)_n-OSO_2-$; or $-N^+R^2R^3-(CH_2CH_2O)_n-N^+R^2R^3-$. They can also include groups of the type:

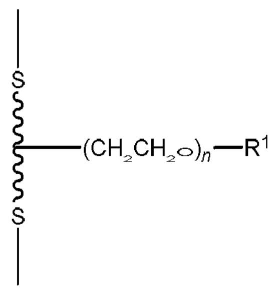
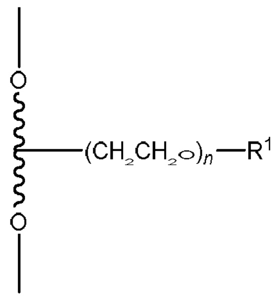
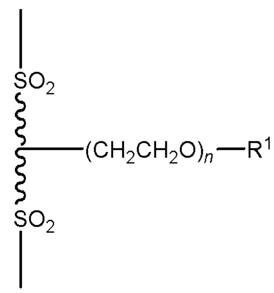

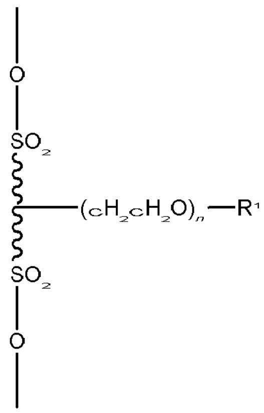
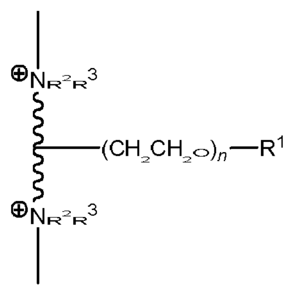

where the $-(CH_2CH_2O)_n-$ group is carried by any suitable linking group, for example an alkyl group. These divalent groups are chemically equivalent to two leaving groups capable of reacting with two nucleophiles."

An especially preferred leaving group L present in a novel conjugating reagent according to the present invention is -SP or $-SO_2P$, especially $-SO_2P$. Within this group, one preferred embodiment is where P represents a phenyl or, especially, a tosyl group. Another preferred embodiment is where P represents a group which includes a portion

$-(CH_2CH_2O)_n-$.

Preferably W represents a keto group, and preferably W' represents a keto group.

Preferably the groupings F' and F have the formula:

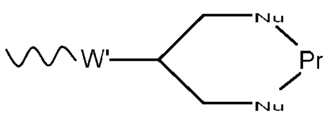

(Ia)

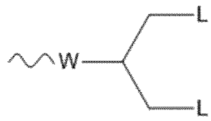

(IIa) or

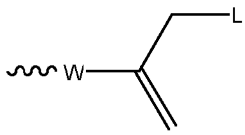

(II'a)

especially

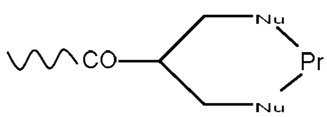

(Ib)

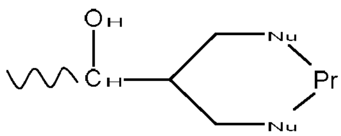

(Ic)

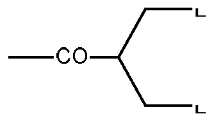

(IIb)

or

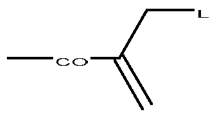

(II'b).

Nucleophilic groups in proteins are for example provided by cysteine, lysine or histidine residues, and Nu may for example be a sulfur atom or an amine group. In one preferred embodiment of the invention, each Nu represents a sulfur atom present in a cysteine residue present in the protein. Such structures may be obtained by reduction of a disulfide bond present in the protein. In another embodiment, each Nu represents an imidazole group present in a histidine residue present in a polyhistidine tag attached to said protein.

## Conjugating processes

Conjugating reagents according to the invention may be reacted with a protein or peptide to form a conjugate according to the invention, and such a reaction forms a further aspect of the invention. Thus, a conjugating reagent including the functional grouping II or II' is reacted with a protein or peptide to form a conjugate including the grouping I. The immediate product of the conjugation process is a conjugate which contains an electron-withdrawing group W. However, the conjugation process is reversible under suitable conditions. This may be desirable for some applications, for example where rapid release of the protein is required, but for other applications, rapid release of the protein may be undesirable. It may therefore be desirable to stabilise the conjugates by reduction of the electron-withdrawing moiety W to give a moiety which prevents release of the protein. Accordingly, the process described above may comprise an additional optional step of reducing the electron withdrawing group W in the conjugate. The use of a borohydride, for example sodium borohydride, sodium cyanoborohydride, potassium borohydride or sodium triacetoxyborohydride, as reducing agent is particularly preferred. Other reducing agents which may be used include for example tin(II) chloride, alkoxides such as aluminium alkoxide, and lithium aluminium hydride.

Thus, for example, a moiety W containing a keto group may be reduced to a moiety containing a CH(OH) group; an ether group $CH.OR^a$ may be obtained by the reaction of a hydroxy group with an etherifying agent; an ester group $CH.O.C(O)R^a$ may be obtained by the reaction of a hydroxy group with an acylating agent; an amine group $CH.NH_2$, $CH.NHR^a$ or $CH.NR^a_2$ may be prepared from a ketone by reductive amination; or an amide $CH.NHC(O)R^a$ or $CH.N(C(O)R^a)_2$ may be formed by acylation of an amine. A sulfone may be reduced to a sulfoxide, sulfide or thiol ether.

A key feature of using conjugating reagents of the invention is that an $\alpha$-methylene leaving group and a double bond are cross-conjugated with an electron withdrawing function that serves as a Michael activating moiety. If the leaving group is prone to elimination in the cross-functional reagent rather than to direct displacement and the electron-withdrawing group is a suitable activating moiety for the Michael reaction then sequential intramolecular bis-alkylation can occur by consecutive Michael and retro Michael reactions. The leaving moiety serves to mask a latent conjugated double

bond that is not exposed until after the first alkylation has occurred to give a reagent including the functional grouping II' and bis-alkylation results from sequential and interactive Michael and retro-Michael reactions. The cross-functional alkylating agents may contain multiple bonds conjugated to the double bond or between the leaving group and the electron withdrawing group.

Where bonding to the protein is via two sulfur atoms derived from a disulfide bond in the protein, the process may be carried out by reducing the disulfide bond following which the reduced product reacts with the reagent according to the invention. Preferably the disulfide bond is reduced and any excess reducing agent is removed, for example by buffer exchange, before the conjugating reagent is introduced. The disulfide bond can be reduced, for example, with dithiothreitol, mercaptoethanol, or tris-carboxyethylphosphine using conventional methods.

Conjugation reactions may be carried out under similar conditions to known conjugation processes, including the conditions disclosed in WO 2005/007197, WO 2009/047500, WO 2014/064423 and WO 2014/064424. The process may for example be carried out in a solvent or solvent mixture in which all reactants are soluble. For example, the protein may be allowed to react directly with the polymer conjugating reagent in an aqueous reaction medium. This reaction medium may also be buffered, depending on the pH requirements of the nucleophile. The optimum pH for the reaction will generally be at least 4.5, typically between about 5.0 and about 8.5, preferably about 6.0 to 7.5. The optimal reaction conditions will of course depend upon the specific reactants employed.

Reaction temperatures between 3-40°C are generally suitable when using an aqueous reaction medium. Reactions conducted in organic media (for example THF, ethyl acetate, acetone) are typically conducted at temperatures up to ambient. In one preferred embodiment, the reaction is carried out in aqueous buffer which may contain a proportion of organic solvent, for example up to 20% by volume of organic solvent, typically from 5 to 20 % by volume of organic solvent.

The protein can be effectively conjugated using a stoichiometric equivalent or a slight excess of conjugating reagent. However, it is also possible to conduct the conjugation reaction with an excess stoichiometry of conjugating reagent, and this may be desirable for some proteins. The excess reagent can easily be removed, for example by ion exchange chromatography or HPLC, during subsequent purification of the conjugate.

Of course, it is possible for more than one conjugating reagent to be conjugated to a protein, where the protein contains sufficient suitable attachment points. For example, in a protein which contains two different disulfide bonds, or in a protein which contains one disulfide bond and also carries a polyhistidine tag, it is possible to conjugate two molecules of the reagent per molecule of protein, and such conjugates form part of the present invention.

**The linker**

The linker which connects the therapeutic, diagnostic or labelling agent to the protein or peptide bonding portion in the conjugates disclosed herein or to the functional grouping in conjugating reagents disclosed herein must include one or more PEG portions as described above. It may also contain any other desired groups, particularly any of the conventional groups commonly found in this field.

Subsection (i). It is disclosed herein that the linker between the payload and the grouping of formula F'IP, and particularly that portion of the linker immediately adjacent the grouping of formula F'/F, may include an alkylene group (preferably a $C_{1-10}$ alkylene group), or an optionally-substituted aryl or heteroaryl group, any of which may be terminated or interrupted by one or more oxygen atoms, sulfur atoms, $-NR^a$ groups (in which $R^a$ represents a hydrogen atom or an alkyl (preferably $C_{1-6}$alkyl), aryl (preferably phenyl), or alkyl-aryl (preferably $C_{1-6}$alkyl-phenyl) group), keto groups, -O-CO- groups, -CO-O- groups, -O-CO-O, $-O-CO-NR^a-$, -NR-CO-O-, $-CO-NR^a-$ and/or $-NR^a.CO-$ groups. Suitable aryl groups include phenyl and naphthyl groups, while suitable heteroaryl groups include pyridine, pyrrole, furan, pyran, imidazole, pyrazole, oxazole, pyridazine, pyrimidine and purine. Especially suitable linking groups are heteroaryl or, especially, aryl groups, especially phenyl groups. These may be adjacent a further portion of the linking group which is, or contains, a $-NR^a.CO-$ or $-CO.NR^a-$ group, for example an -NH.CO- or -CO.NH- group. Here and elsewhere throughout this Specification, where a group $R^a$ is present, this is preferably a $C_{1-4}$alkyl, especially a methyl group or, especially, a hydrogen atom.

Substituents which may be present on an optionally substituted aryl, especially phenyl, or heteroaryl group include for example one or more of the same or different substituents selected from alkyl (preferably $C_{1-4}$alkyl, especially methyl, optionally substituted by OH or $CO_2H$), -CN, $-NO_2$, $-CO_2R^a$, -COH, $-CH_2OH$, $-COR^a$, $-OR^a$, $-OCOR^a$, $-OCO_2R^a$, $-SR^a$, $-SOR^a$, $-SO_2R^a$, $-NHCOR^a$, $-NR^aCOR^a$, $-NHCO_2R^a$, $-NR^a.CO_2R^a$, -NO, -NHOH, $-NR^a.OH$, $-C=N-NHCOR^a$, $-C=N-NR^a.COR^a$, $-N^+R^a_3$, $-N^+H_3$, $-N^+HR^a_2$, $-N^+H_2R^a$, halogen, for example fluorine or chlorine, $-C\equiv CR^a$, $-C=CR^a_2$ and $-C=CHR^a$, in which each $R^a$ independently represents a hydrogen atom or an alkyl (preferably $C_{1-6}$alkyl), aryl (preferably phenyl), or alkyl-aryl (preferably $C_{1-6}$alkyl-phenyl) group. The presence of electron withdrawing substituents is especially disclosed. Disclosed substituents include for example CN, $NO_2$, $-OR^a$, $-OCOR^a$, $-SR^a$, $-NHCOR^a$, $-NR^a.COR^a$, -NHOH and $-NR^a.COR^a$.

It is disclosed that the linker includes for example one of the above groups adjacent the grouping F'/F. Especially

preferred are conjugates and conjugating reagents which include the grouping:

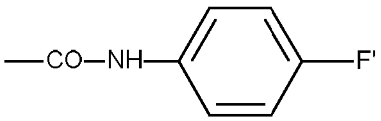

(Va) or

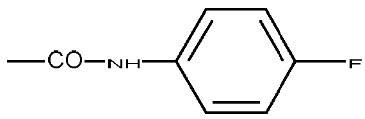

(VIa)

or, especially:

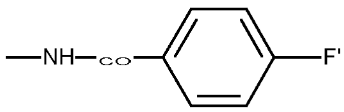

(Vb) or

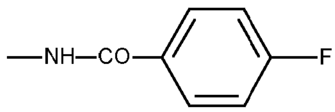

(VIb).

In the above formulae, it is disclosed that F' has the formula Ia or Ib above, and preferably F has the formula IIa, II'a, IIb or II'b above.

Subsection (ii). It is disclosed herein that the linker may contain a degradable group, i.e. it may contain a group which breaks under physiological conditions, separating the payload from the protein to which it is, or will be, bonded. Alternatively, it may be a linker that is not cleavable under physiological conditions. Where a linker breaks under physiological conditions, it is preferably cleavable under intracellular conditions. Where the target is intracellular, it is disclosed that the linker is substantially insensitive to extracellular conditions (i.e. so that delivery to the intracellular target of a sufficient dose of the therapeutic agent is not prohibited).

Where the linker contains a degradable group, this is generally sensitive to hydrolytic conditions, for example it may be a group which degrades at certain pH values (e.g. acidic conditions). Hydrolytic/acidic conditions may for example be found in endosomes or lysosomes. Examples of groups susceptible to hydrolysis under acidic conditions include hydrazones, semicarbazones, thiosemicarbazones, cis-acotinic amides, orthoesters and ketals. Examples of groups susceptible to hydrolytic conditions include:

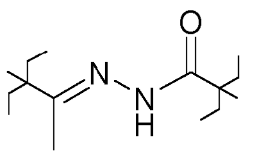

and

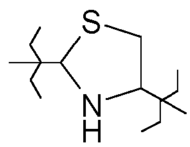

.

In a preferred embodiment, the linker includes

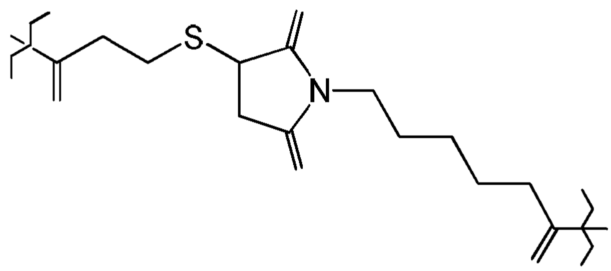

.

For example, it may include:

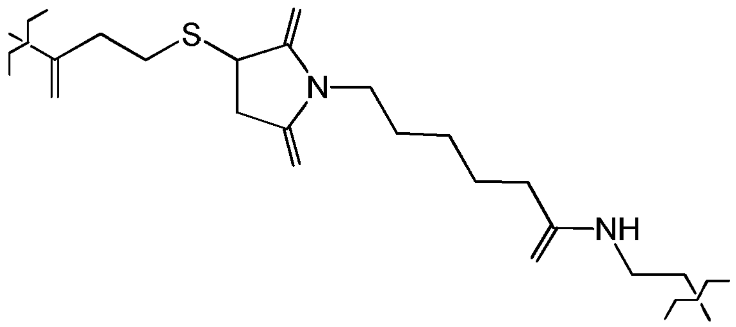

.

The linker may also be susceptible to degradation under reducing conditions. For example, it may contain a disulfide group that is cleavable on exposure to biological reducing agents, such as thiols. Examples of disulfide groups include:

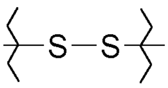

and

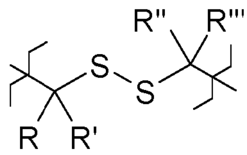

in which R, R', R" and R‴ are each independently hydrogen or $C_{1-4}$alkyl. In a disclosed embodiment the linker includes

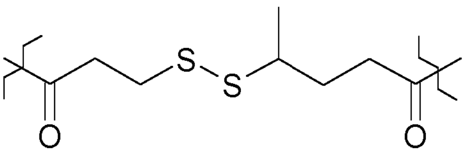

or

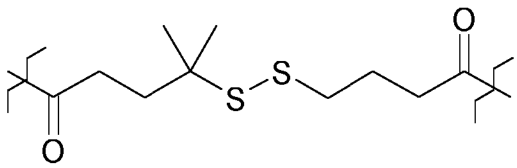

.

For example, it may include

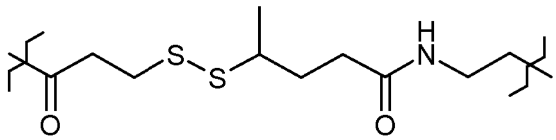

or

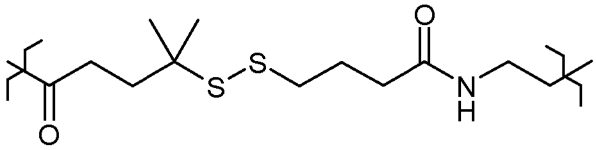

.

The linker may also contain a group which is susceptible to enzymatic degradation, for example it may be susceptible to cleavage by a protease (e.g. a lysosomal or endosomal protease) or peptidase. For example, it may contain a peptidyl group comprising at least one, for example at least two, or at least three amino acid residues (e.g. Phc-Lcu, Gly-Phc-Lcu-Gly, Val-Ala, Val-Cit, Phe-Lys). For example, it may include an amino acid chain having from 1 to 5, for example 2 to 4, amino acids. Another example of a group susceptible to enzymatic degradation is:

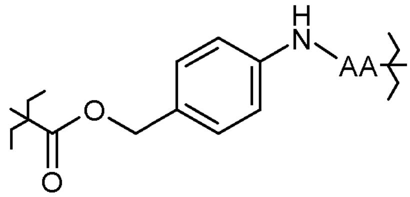

,

wherein AA represents a protease-specific amino acid sequence, such as Val-Cit. In a preferred embodiment, the linker includes:

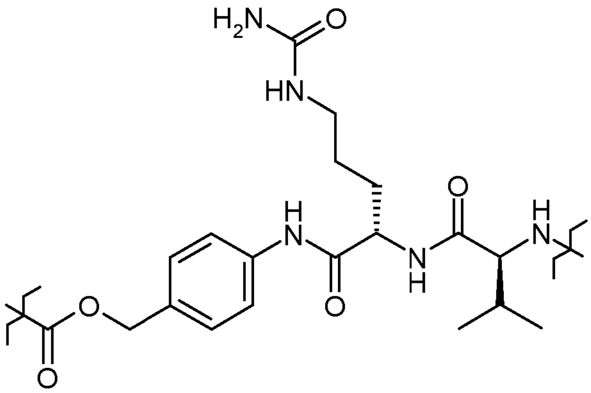

For example, it may include

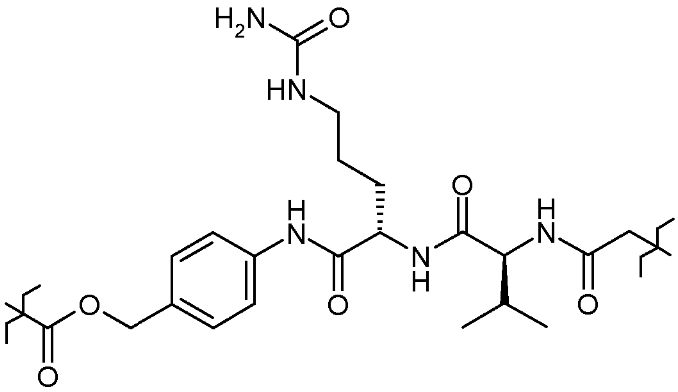

.

The linker may carry a single payload D, or more than one group D. Multiple groups D may be incorporated by the use of a branching linker, which may for example incorporate an aspartate or glutamate or similar residue. This introduces a branching element of formula:

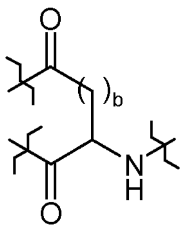

(XIIa)

where b is 1, 2 or 3, b=1 being aspartate and b=2 being glutamate, and b=3 representing one preferred embodiment. Each of the acyl moieties in the above formula may be coupled to a group D. The branching group above may incorporate a $-CO.CH_2-$ group, thus:

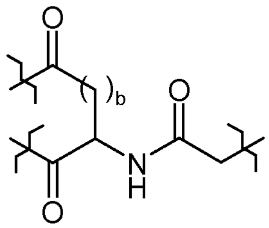

(XIIb)

If desired, the aspartate or glutamate or similar residue may be coupled to further aspartate and/or glutamate and/or similar residues, for example:

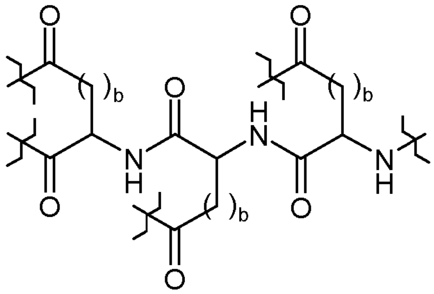

(XIIc) or

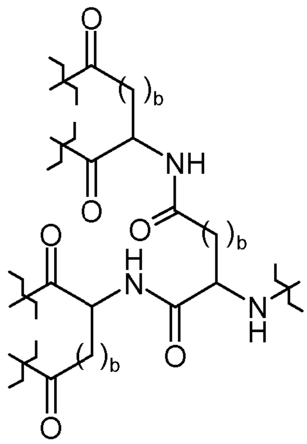

(XIId)

and so on.

As will be apparent, many alternative configurations for the linker between the grouping F/F' and the payload are possible. One preferred configuration may be represented schematically as follows:

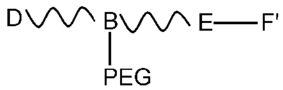

(VII)

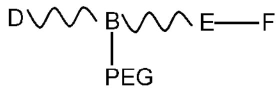

(VIII)

in which E represents one of the groups mentioned in subsection (i) above, and B represents one of the groups mentioned in this subsection (ii).

A specific, particularly preferred construction is shown below:

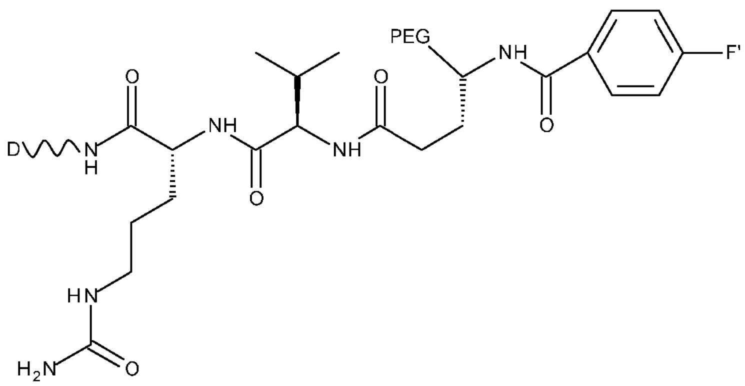

(VIIa)

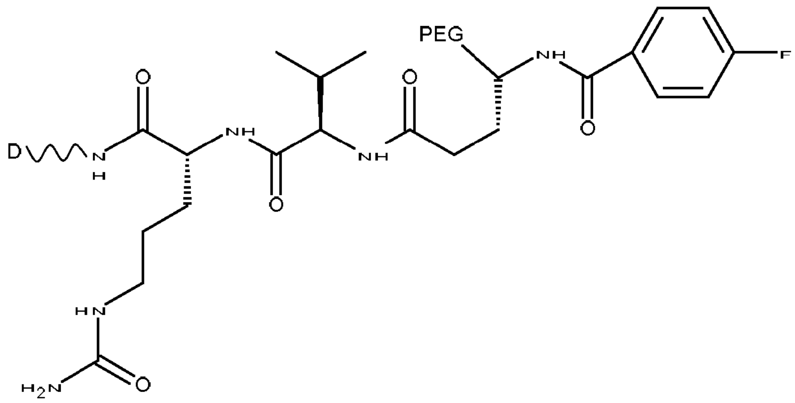

(VIIIa)

in which F' and F have the meanings given above.

Subsection (iii). The linker which connects the therapeutic, diagnostic or labelling agent to the protein or peptide bonding portion in the conjugates disclosed herein or to the functional grouping in the conjugating reagents disclosed herein may contain additional PEG in addition to the pendant PEG chain which has a terminal end group of formula $-CH_2CH_2OR$. It may for example contain PEG in the backbone of the linker, shown schematically thus:

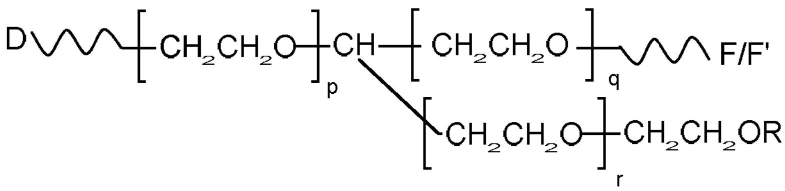

(IX)

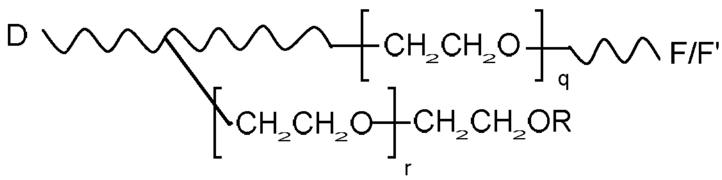

(X)

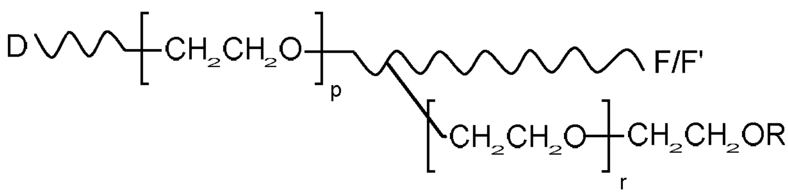

(XI)

In these formulae, p, q and r represent the number of PEG units present in the various PEG chains present in the linker of the conjugate or the reagent. For clarity, the PEG units are shown as straight-chain units, but it will be understood that any of the units may include branched chains.

Subsecton (iv). The linker which connects the therapeutic, diagnostic or labelling agent to the protein or peptide bonding portion in the conjugates disclosed herein or to the functional grouping in the conjugating reagents disclosed herein may contain two or more pendant PEG chains. This may be illustrated schematically for two pendant PEG chains thus:

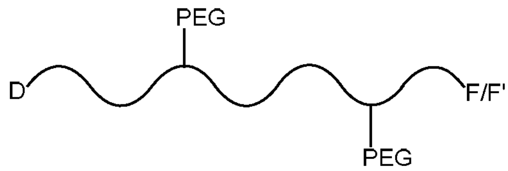

and obviously more than two pendant PEG chains may similarly be present. The linker may or may not contain additional PEG in addition to the pendant PEG chains, as described in subsection (iii) above.

Multiple pendant PEG chains may be incorporated into the linker using any suitable method. A pendant PEG chain may for example be introduced by reaction with any reactive grouping present in any of the linker portions discussed above. Branching groups of the formulae (XIIa-d) as described above may be used. For example, in one specific embodiment, two pendant PEG portions may be incorporated by use of a structure (XIIa):

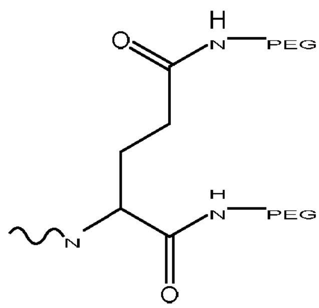

(XIIe)

Alternatively, branching may be introduced by use of a polyol functionality, for example:

$$\sim CH_s[(CH_2)_tO]_{3-s}\sim$$

in which s is 0, 1 or 2, and t is 1 to 4. For example, in one specific embodiment, three pendant PEG portions may be incorporated by use of a structure:

$$\sim C[CH_2O\text{-}(CH_2)_2\text{-}CO\text{-}NH\text{-}PEG]_3.$$

## Brief description of the drawings

Figures 1 and 2 show the results of Example 6.
Figure 3 shows the results of Example 7.
Figures 4a, 4b and 4c show the results of Example 14.
Figures 5a and 5b show the results of Example 15.
Figures 6a, 6b, 6c and 6d show the results of Example 16.
Figures 7a, 7b and 7c show the results of Example 17.
Figures 8a and 8b show the results of Example 18.
Figures 9a and 9b show the results of Example 19.
Figure 10 shows the results of Example 23.
Figure 11 shows the results of Example 25.

The following Examples illustrate the invention.

Example 1: Synthesis of conjugation reagent **1** comprising an auristatin cytotoxic payload

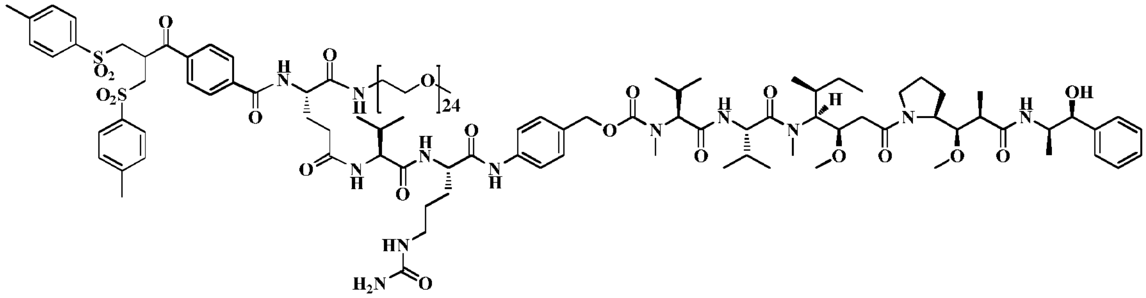

**1**

Step 1: Synthesis of compound **2**.

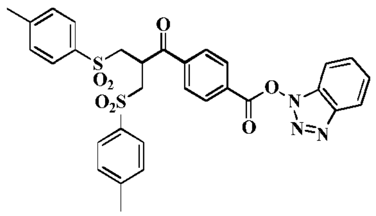

**2**

A solution of 4-[2,2-bis[(p-tolylsulfonyl)-methyl]acetyl]benzoic acid (1.0 g, Nature Protocols, 2006, 1(54), 2241-2252) was added to N-hydroxybenzotriazole hydrate (306 mg) in anhydrous THF (10 mL) under a nitrogen atmosphere. The resulting solution was cooled to 0 °C and diisopropylcarbodiimide (310 $\mu$L) was added dropwise. The reaction mixture was stirred for 20 min at 0 °C before being warmed to room temperature. Additional THF (10 mL) was added to the reaction mixture after 1 h. After 18 h, the formed precipitate was filtered and washed with cold THF (2 $\times$ 5 mL) before being dried *in vacuo.* The solid was stirred with MeOH (10 mL) for 1 h at room temperature, collected by filtration and washed sequentially with MeOH (2 $\times$ 5 mL) and $Et_2O$ (5 mL). The solid was then dried *in vacuo* to give bis-tolylsulfonyl-propanoyl-benzoic HOBt ester compound **2** as a white solid (1.1 g, 88%). m/z $[M+H]^+$ (618, 100%).

Step 2: Synthesis of compound **3**.

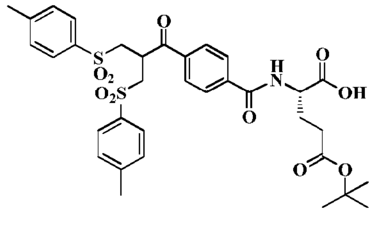

**3**

To a stirred suspension of (S)-Glu-5-(OtBu) (198 mg) in anhydrous DMF (20 mL) under a nitrogen atmosphere was added N-methylmorpholine (NMM) (107 $\mu$L). The reaction mixture was cooled to 0 °C before compound **2** (603 mg) was added. The resulting suspension was stirred at 0 °C for 1 h, after which the reaction mixture was allowed to warm to room temperature. After 19 h, the resulting solution was concentrated *in vacuo* and purified by reverse phase column C18-column chromatography eluting with buffer A (v/v): water:5% acetonitrile:0.1% formic acid and buffer B (v/v): acetonitrile:0.1% formic acid (100:0 v/v to 0:100 v/v). The organic solvent was removed *in vacuo* and the aqueous solvent was removed by lyophilisation to give the bis-tolylsulfonyl-propanoyl-benzamide-L-Glu-[O$^t$Bu]-[OH] compound **3** as a white solid (198 mg, 67%). $^1$H NMR (400 MHz, $CDCl_3$) $\delta$ 7.98 (1H, d), 7.86 (2H), 7.71 - 7.65 (6H, m), 7.36 (4H, d), 4.68 (1H, ddd), 4.34 (1H, q), 3.62 (2H, ddd), 3.50 (2H, ddd), 2.69 (1H ddd), 2.55 - 2.45 (1H, m), 2.48 (6H, s), 2.34-2.16 (2H,

m), 1.46 (9H, s); m/z $[2M+H]^+$ (1371,74%), $[2M-^tBu]^+$ (1315, 70%), $[M-^tBu]^+$ (630, 100%).

Step 3: Synthesis of compound **4**.

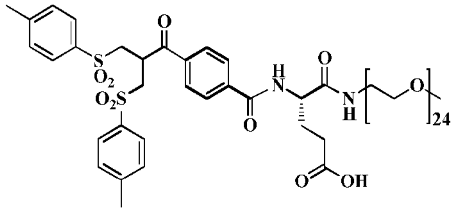

**4**

Compound **3** (50 mg) and (benzotriazol-1-yloxy)tris-(dimethylamino) phosphonium hexafluorophosphate (BOP) (40 mg) were dissolved in anhydrous DMF (3 mL), cooled to 0 °C and added to a solution of $NH_2$-PEG(24u)-OMe (99 mg) and NMM (10 μL) in anhydrous DMF (2 mL).The reaction mixture stirred at 0 °C and after 4 h, additional amounts of BOP (10 mg) and NMM (2.5 μL) were added to the reaction mixture and incubated for a further 15 min., before being stored at -20 °C for 18 h. The resultant reaction mixture was concentrated *in vacuo* and purified by reverse phase column C18-column chromatography, eluting with buffer A (v/v): water:5% acetonitrile:0.1% formic acid and buffer B (v/v): acetonitrile:0.1% formic acid (100:0 v/v to 0:100 v/v). The organic solvent was removed *in vacuo* and the aqueous solvent removed by lyophilisation to give bis-tolylsulfonyl-propanoyl-benzamide-L-Glu-[$O^t$Bu]-[PEG(24u)-OMe] as a colourless oil (128 mg, 100%). m/z $[M+H]^+$ (1757, 100%), $[M+2H]^{2+}$ (879, 100%). Bis-tolylsulfonyl-propanoyl-benzamide-L-Glu-[$O^t$-Bu]-[PEG(24u)-OMe] (126.5 mg) was dissolved in formic acid (2.5 mL) and stirred under a nitrogen atmosphere at room temperature. After 20 h, the reaction mixture was concentrated *in vacuo* and dried under high vacuum for 18 h to give bis-tolylsulfonyl-propanoyl-benzamide-L-Glu-[OH]-[PEG(24u)-OMe] compound **4** as a colourless oil (122 mg, assumed quantitative yield). m/z $[M+Na]^+$ (1723, 15%), $[M+H]^+$ (1700, 100%). This material was used without any further purification.

Step 4: Synthesis of reagent **1**.

A solution of compound **4** (13.0 mg), HATU (4.1 mg), val-cit-PAB-MMAE TFA salt (9.0 mg) in DMF (1.0 mL) under an argon atmosphere was cooled to 0 °C. To this was added NMM (2.0 μL). After 1 h, an additional amount of HATU (4.1 mg) and NMM (2 μL) was added, and after a further 1.5 h the solution was stored at -20 °C for 72 h. The reaction solution was concentrated *in vacuo, dissolved* in acetonitrile (1.0 ml) and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent was removed by lyophilisation to give bis-tolylsulfonyl-propanoyl-benzamide-Glu-[NH-PEG(24u)-OMe]-[val-cit-PAB-MMAE] reagent **1** as a thick clear colourless oil (11.4 mg, 56%). m/z $[M+H]^+$ (2805, 20%), $[M+2H]^{2+}$ (1403, 75%), $[M+3H]^{3+}$ (936, 100%).

**Example 2**: Synthesis of conjugation reagent **5** comprising a maytansinoid cytotoxic payload

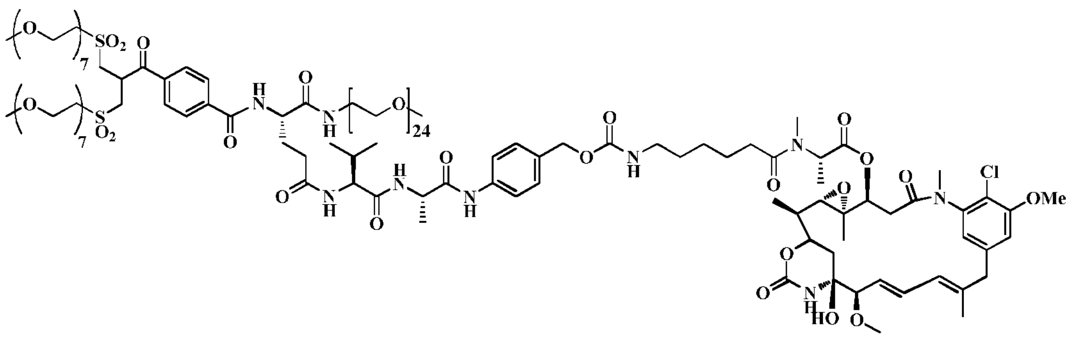

5

Step 1: Synthesis of compound 6.

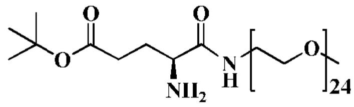

6

A solution of Fmoc-L-Glu-(OtBu)-OH (36 mg) in DMF (2 mL) was cooled to 0 °C under an argon atmosphere and (benzotriazol-a-yloxy)tris-(dimethylamino)phosphonium hexafluorophosphate BOP (41 mg) was added, followed by $NH_2$-PEG(24u)-OMe (100 mg) and N,N-diisopropylethylamine (19 $\mu$L). The solution was allowed to warm to room temperature and after 22 h the volatiles were removed *in vacuo*. The resulting residue was dissolved in dichloromethane (1 mL) and purified by normal phase column chromatography eluting with dichloromethane methanol (100:0 v/v to 80:20 v/v). The organic solvent was removed *in vacuo* to give Fmoc-L-Glu-[$O^tBu$]-[PEG(24u)-OMe] as a colourless oil (84 mg, 67%). Piperidine (49 $\mu$L) was added to a solution of compound Fmoc-L-Glu-[$O^tBu$]-[PEG(24u)-OMe] (74 mg) in DMF (2 mL) under an argon atmosphere and the resulting solution stirred at room temperature for 22 h, after which the volatiles were removed *in vacuo*. The resulting residue was triturated with hexane (3 $\times$ 0.7 mL). The organic solvent was decanted each time and the resulting residue dried *in vacuo* to give the L-Glu-[OtBu]-[PEG(24u)-OMe] compound **6** as a white solid (61 mg, 97%). m/z $[M+H]^+$ (1097, 10%), $[M+2H]^{2+}$ (1035, 100%).

Step 2: Synthesis of compound **7**.

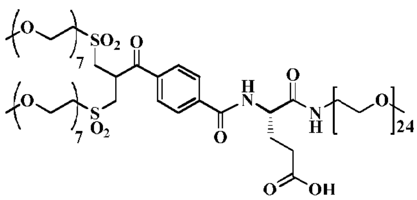

7

A solution of compound **6** (26.6 mg) in DMF (550 $\mu$L) was cooled to 0 °C under an argon atmosphere to which HATU (10.5 mg) was added and the solution stirred for 0.5 h at 0 °C. To this was added a solution of 4-[2,2-bis[alpha-methoxy-omega-sulfonyl hepta(ethylene glycol)]acetyl]benzoic acid (32 mg, prepared in an analogous way to 4-[2,2-bis[(p-tolylsulfonyl)-methyl]acetyl]benzoic acid in Nature Protocols, 2006, 1(54), 2241-2252, but using alpha-methoxy-omega-mercapto hepta(ethylene glycol) instead of 4-methylbenzenethiol) in DMF (550 $\mu$L). The resulting solution was stirred for 5 min at 0 °C before addition of NMM (2.9 $\mu$L) and HATU (10.5 mg). The reaction solution was allowed to stir at 0 °C for 2 h before being warmed to room temperature and stirred for a further 3.5 h. After this time the volatiles were removed *in vacuo*. The resulting residue was dissolved in water and acetonitrile (v/v; 1/1, 1.2 ml), and purified by reverse phase C18-column chromatography eluting with buffer A (v/v): water:5% acetonitrile:0.1% formic acid and buffer B (v/v):

acetonitrile:0.1% formic acid (100:0 v/v to 0:100 v/v). The organic solvent was removed *in vacuo* and the aqueous solvent removed by lyophilisation to give bis-mPEG(7u)sulfone-propanoyl-benzamide-L-Glu-[OtBu]-[PEG(24u)-OMe] as a colourless oil (30.5 mg, 55%). $^1$H NMR (400 MHz, MeOH-$\delta_4$) 8.19 (2H, d), 8.04 (2H, d), 4.83 - 4.71 (1H, m), 4.58 (1H, dd, ), 3.92 - 3.83 (6H, m), 3.78 - 3.56 (140H, m), 3.57-3.51 (6H, m), 3.40 (4H, dd), 3.36 (3H, s), 3.35 (6H, s), 2.41 (2H, t), 2.24 - 2.13 (1H, m), 2.10 - 1.98 (1H, m), 1.45 (9H, s); m/z $[M+Na]^+$ (2243, 50%), $[M+H]^+$ (2221, 40%), $[M+Na+2H]^{3+}$ (747,100%). A solution of bis-mPEG(7u)sulfone-propanoyl-benzamide-L-Glu-[OtBu]-[PEG(24u)-OMe] (30 mg) in dichloromethane (2 mL) under an argon atmosphere was cooled to 0 °C to which trifluoroacetic acid (500 $\mu$L) was added and the resulting solution stirred for 1.5 h. The reaction mixture was allowed to warm to room temperature and stirred for a further 1 h. After this time the volatiles were removed *in vacuo.* The resulting residue was dissolved in water and acetonitrile (v/v; 1/1, 0.6 mL), and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give the bis-mPEG(7u)sulfone-propanoyl-benzamide-L-Glu-[OH]-[PEG(24u)-OMe] compound **7** as a colourless oil (20 mg, 68%). $^1$H NMR (400 MHz, MeOH-$\delta_4$) 8.19 (2H, d), 8.04 (2H, d), 4.81 - 4.72 (1H, m), 4.59 (1H, dd), 3.92 - 3.84 (6H, m), 3.67 - 3.50 (146H, m), 3.40 (4H, dd), 3.36 (3H, s), 3.35 (6H, s), 2.48 (2H, t), 2.26 - 2.15 (1H, m), 2.15 - 2.03 (1H, m); m/z $[M+H]^+$ (2165, 55%), $[M+2H]^{2+}$ (1083, 60%), $[M+2H+Na]^{3+}$ (729, 100%).

Step 3: Synthesis of reagent **5**

A solution of compound **7** (15.0 mg) in DMF (600 $\mu$L) was cooled to 0 °C under an argon atmosphere. HATU (2.9 mg) was added and the solution stirred for 0.5 h at 0 °C. To this was added a solution of val-ala-PAB-AHX-DM1 (9.2 mg) and NMM (0.8 $\mu$L) in DMF (600 $\mu$L), which had been stirred at room temperature for 0.5 h. After 5 min, an additional amount of HATU (2.9 mg) and NMM (0.8 $\mu$L) was added and the reaction mixture stirred at 0 °C. After 3 h, an additional amount of HATU (0.7 mg) was added and the reaction mixture stirred at 0 °C. After a further 2 h, the reaction was stored at -20 °C for 16 h. The reaction solution was concentrated *in vacuo* and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give the bis-mPEG(7u)sulfone-propanoyl-benzamide-L-Glu-[val-ala-PAB-AHX-DM1]-[PEG(24u)-OMe] compound **5** as a thick clear colourless oil (14.3 mg, 64%). $^1$H NMR (600 MHz, MeOH $\delta_4$) (selected characteristic signals) 5.69 (1H, dd,), 6.59 (1H, dd), 6.68 (1H, s), 6.69 (1H, d), 7.10 (1H, s), 7.28 (2H, d), 7.57 (2H, d), 8.01 (2H, d), 8.16 (2H, d); m/z $[M\text{-}AHX\text{-}DM1]^+$ (2422, 40%).

Example 3: Synthesis of a conjugation reagent **8** comprising 7 repeat unit polymeric leaving groups and a maytansinoid cytotoxic payload

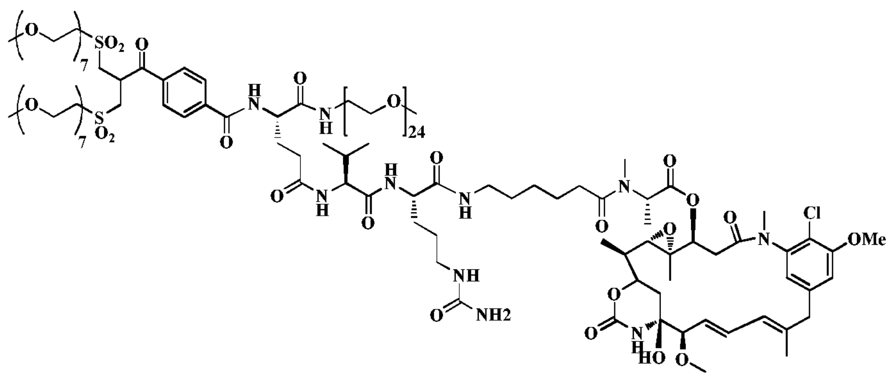

**8**

A solution of compound **7** (12.4 mg) in DMF (500 $\mu$L) was cooled to 0 °C under an argon atmosphere. HATU (2.4 mg) was added and the solution stirred for 0.5 h at 0 °C. To this was added a solution of val-cit-AHX-DM1 made in an analogous way to compound **10A** (6.4 mg) and NMM (0.7 $\mu$L) in DMF (500 $\mu$L), which had been stirred at room temperature for 0.5 h. After 5 min, an additional amount of HATU (1.2 mg) and NMM (0.4 $\mu$L) was added and the reaction mixture stirred at room temperature. After 2 h, an additional amount of HATU (1.2 mg) and NMM (0.4 $\mu$L) was added and the reaction mixture stirred at room temperature. After a further 1 h, the reaction solution was concentrated *in vacuo*

and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give bis-mPEG(7u)sulfone-propanoyl-benzamide-L-Glu-[val-cit-AHX-DM1]-[PEG(24u)-OMe] **8** as a thick clear colourless oil (9.6 mg, 53%). m/z $[M-H_2O]^+$ (3148, 8%), $[M-H_2O]^{2+}$ (1575, 40%), $[M-H_2O]^{3+}$ (1050, 100%), 1036 $[M-NHCO-H_2O]^{3+}$.

**Example 4**: Synthesis of a conjugation reagent **9** comprising an auristatin cytotoxic payload

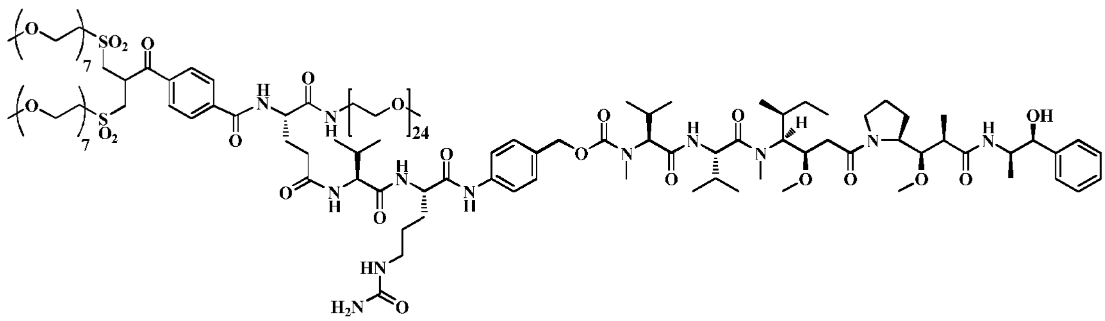

**9**

Reagent **9** was synthesised in analogous way to reagent **8** of Example 3 from compound **7** and val-cit-PAB-MMAE TFA salt. Bis-mPEG(7u)sulfone-propanoyl-benzamide-L-Glu-[val-cit-PAB-MMAE]-[PEG(24u)-OMe] **9** was isolated as a colourless oil. m/z $[M+H]^+$ (3270, 12%), $[M+2H]^{2+}$ (1636, 50%), $[M+3H]^{3+}$ (1091, 100%).

**Example 5**. Preparation of antibody drug conjugates

Antibody drug conjugates were prepared by methods analogous to those described in WO2014064423 and WO2014064424. Briefly, antibody (trastuzumab or brentuximab) was reduced using tris(2-carboxyethyl)phosphine at 40 °C for 1 h. Conjugation of the antibody with 1.5 molar equivalents of reagent (i.e., **1**, **5**, **8**, **9**) per inter-chain disulfide bond was then performed by dissolving reagents to a final concentration of 1.6 mM in either acetonitrile or DMF. The antibody solution was diluted to 4.21 mg/mL with 20 mM sodium phosphate buffer, 150 mM NaCl, 20 mM EDTA, pH 7.5. Reagents were added to antibody and the final antibody concentration in the reaction was adjusted to 4 mg/mL with 20 mM sodium phosphate buffer, 150 mM NaCl, 20 mM EDTA, pH 7.5. Each solution was mixed gently and incubated at 22 °C. Antibody drug conjugate product was purified by hydrophobic interaction chromatography for each conjugate.

**Example 6**: *In vitro* cytotoxicity comparison of brentuximab drug conjugate **10** prepared from reagent **9** with the brentuximab drug conjugate **11** produced using reagent bis-tolylsulfonyl-propanoyl-benzamide-PEG(24u)-val-cit-PAB-MMAE, **12** using the method described in WO2014064423.

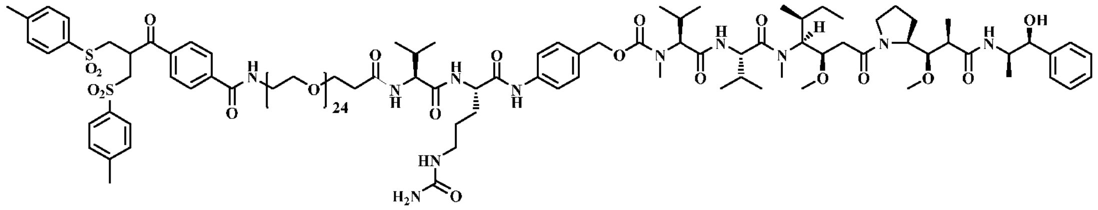

**12**

Purified brentuximab drug conjugates **10** and **11,** with a drug to antibody ratio (DAR) of four as described in Example 6, were evaluated in vitro by measuring the inhibitory effect on cell growth of (CD30)-positive cell line Karpas 299 using the method described below.

Loss of tumour cell viability following treatment with cytotoxic drugs or ADCs in vitro can be measured by

growing cell lines in the presence of increasing concentrations of drugs or ADCs and quantifying the loss of proliferation or metabolic activity using CellTiter Glo® Luminescence reagent (Promega Corp. Technical Bulletin TB288; Lewis Phillips G.D, Cancer Res 2008; 68:9280-9290). The protocol describes cell seeding, drug treatment and determination of the cell viability in reference to untreated cells based on ATP synthesis, which is directly related to the number of cells present in the well.

The human T cell lymphoma cell line Karpas 299 was obtained from Dr Abraham Karpas at the University of Cambridge. The cells were grown in RPMI medium (Life Technologies®), 10% fetal bovine serum, 100 u/mL Penicillin and 100 μg/mL Streptomycin.

CD30-positive Karpas 299 were counted using a Neubauer haemocytometer and adjusted to a cell density of $5\times10^4$/mL. Cells were seeded (50 μL/well) into opaque-walled 96-well plates and incubated for 24 h at 37°C and 5% $CO_2$.

Methods for cell culture were derived from product information sheet from supplier and references quoted therein, for example, Culture of Animal Cells: A Manual of Basic Technique by R. Ian Freshney 3rd edition, published by Alan R. Liss, N.Y. 1994, or 5th edition published by Wiley-Liss, N.Y. 2005. Serial dilutions of ADC or free drug (MMAE), were made in triplicate by pipetting across a 96 well plate from columns 2-11 with 2-fold dilutions using the relevant cell culture medium as a diluent. The CD30-positive Karpas 299 were treated with drug concentrations shown in Table 1. Cells were then incubated with the drug at 37 °C and 5% $CO_2$ for a further 72h.

Table 1

| Cell line | Drug/drug-conjugate | Concentration range |
|---|---|---|
| **Karpas 299** | MMAE (free drug) | 2500 pM-4.9 pM |
| **Karpas 299** | Brentuximab-drug conjugate **10**, DAR4 | 333 pM-0.65 pM |
| **Karpas 299** | Brentuximab-drug conjugate **11**, DAR4 | 333 pM-0.65 pM |

The cell viability assay was carried out using the Cell-Titer Glo® Luminescence reagent, as described by the manufacturer's instructions, (Promega Corp. Technical Bulletin TB288; Lewis Phillips G.D, Cancer Res 2008; 68:9280-9290). Incubation times, e.g. cell lysis and incubation with luminescent reagent, were extended to 3 min and 20 min respectively, for optimal luminescent signal. Luminescence was recorded using a plate reader (e.g. MD Spectramax M3 plate reader), and data subsequently analysed using a four parameter nonlinear regression model.

The results are shown in Figures 1 and 2, which illustrate cell viability responses to treatment with either antibody conjugates **10**, **11** or free drug within Karpas 299 cells. Figure 1 shows the effect of brentuximab-drug conjugate **10** (solid line) and free drug, MMAE (dashed line), on cell viability of CD30-positive Karpas 299 cell line, while Figure 2 shows the effect of brentuximab-drug conjugate **11** (solid line) and free drug, MMAE (dashed line), on cell viability of CD30-positive Karpas 299 cell line. Viability is expressed as % of untreated cells. The % viability (Y-axis) is plotted against the logarithm of drug concentration in pM (x-axis) to determine the IC50 values for all conjugates as well as free drug. The IC50 values are shown in Table 2.

Table 2

| Sample name | IC50 [pM] | St. Dev |
|---|---|---|
| Brentuximab-drug conjugate **10** | 13.0 | 1.1 |
| Brentuximab-drug conjugate **11** | 19.3 | 2.6 |
| MMAE | 105.3 | 3.4 |

As shown in Figures 1 and 2 and Table 2, the antibody-drug conjugates **10** and **11** are active in CD30-positive Karpas 299.

**Example 7**: In vivo xenograft study comparing brentuximab-drug conjugate **10** prepared from reagent **9** with the brentuximab-drug conjugate **11** produced using the method described in WO2014064423.

Two purified antibody drug conjugates (ADCs), **10** and **11** each with DAR = 4 were produced as described within example 6. Purity following HIC purification was greater than 95% for both conjugates.

Each conjugate was then used in xenograft studies as follows.

Healthy female severe combined immunodeficient (SCID) mice (C.B-17/Icr-*Prkdcscid*, Charles River Laboratories) with an average body weight (BW) of 20.2 g (range = 16.5 g to 23.2 g) on Day 1 of the study were used. The animals were maintained in SPF health status according to the FELASA guidelines in housing rooms under controlled environmental conditions. Animal enclosures were designed to provide sterile and adequate space with bedding material, food and water, environmental and social enrichment.

Xenografts were initiated with Karpas 299 T-anaplastic large cell lymphoma (ALCL) cell line by subcutaneous injection in SCID mice. On the day of tumour induction, each test mouse received $10^7$ Karpas 299 cells in 200 μL of RPMI 1640 into the right flank. Tumours were measured in two dimensions using calipers, and volume was calculated using the formula:

$$Tumor\ Volume\ (mm^3) = \frac{w^2 \times l}{2}$$

where w = width and *l* = length, in mm, of the tumour.

Twelve to fourteen days after tumour implantation, designated as Day 1 of the study, the animals were sorted into groups each consisting of five or ten mice with group mean tumour volumes of 111 to 115 $mm^3$ or 148 to 162 $mm^3$. Treatment began on Day 1 in all groups. One treatment group was given intravenous injection (i.v.) on Day 1 with brentuximab-drug conjugate **11** at 1 mg/kg, and another treatment group with brentuximab-drug conjugate **10** at 1 mg/kg. PBS was given to mice in the vehicle-treated control group.

Mice were monitored individually, and each animal was euthanized when its tumour reached the endpoint volume of 2000 mm3. Treatment tolerability was assessed by body weight measurements and frequent observation for clinical signs of treatment-related side effects.

Percentage change in tumour volume was calculated for each mouse at day 7 and expressed as % mean ± standard error. All regimens were well tolerated and could be evaluated for efficacy. Percentage tumour volume change after 7 days in the group treated with brentuximab conjugated using brentuximab-drug conjugate **11** was 212 ± 43%, indicating an increase in tumour volume. In contrast, percentage tumour volume change at day 7 in the group treated with brentuximab conjugated using brentuximab-drug conjugate **10**was significantly lower (p=0.0043; student's t test) at -2 ± 6%, indicating a reduction in tumour volume and enhanced anti-tumour effect. The results are shown in Figure 3, which shows the percentage change in tumour volume for brentuximab-drug conjugates **10** and **11**. Conjugates were dosed at 1 mg/kg i.v., and tumour volume measured at day 7 post-injection. Values are expressed as % mean ± standard error.

**Example 8**: Synthesis of a disulfide bridging reagent **11A** comprising the cytotoxic payload **1A**.

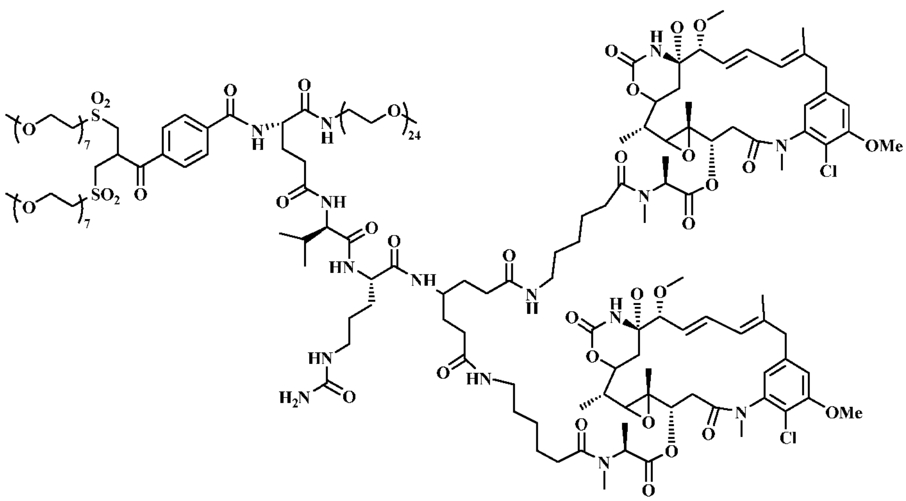

**11A**

Synthesis of cytotoxic payload **1A.**

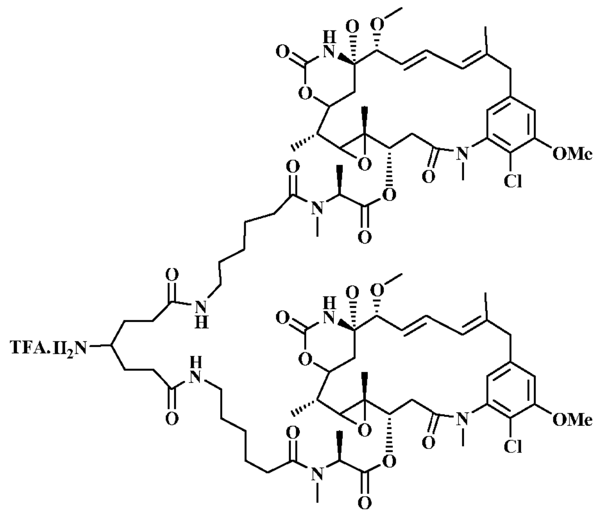

**1A**

Step 1: Synthesis of compound **2A**.

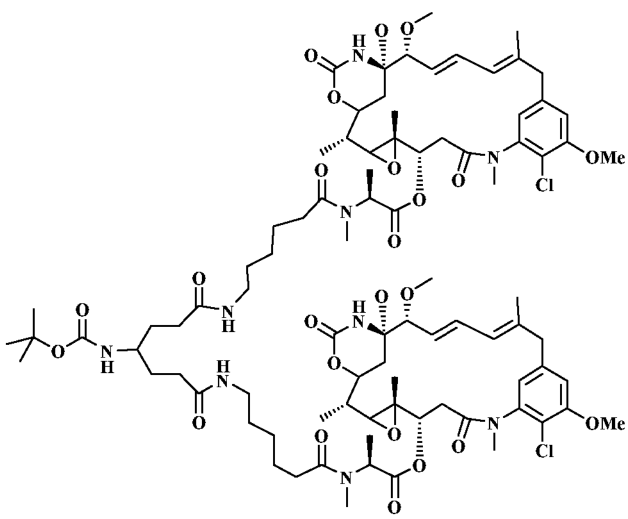

**2A**

To a stirred solution of aminohexanoic maytansine (AHX-DM1).TFA salt (29.4 mg) of formula:

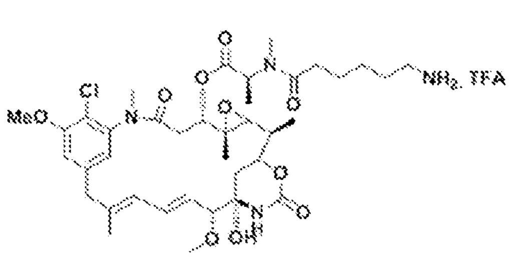

in dimethylformamide (DMF) (400 μL) was added a solution of 4-(*N*-Boc-amino)-1,6-heptanedioic acid bis-pentafluorophenyl ester (10.2 mg) in DMF (200 μL). The solution was cooled to 0 °C before addition of *N,N*-diisopropylethylamine (DIPEA) (13.5 μL). The solution was allowed to warm to room temperature and stirred for 18.5 h. The reaction solution was purified by reverse phase C18-column chromatography eluting with buffer A (v/v): water:5% acetonitrile:0.05%

trifluoroacetic acid and buffer B (v/v): acetonitrile:0.05% trifluoroacetic acid (100:0 v/v to 0:100 v/v). The solvent was removed by lyophilisation to give 4-(*N*-boc-amino)-1,6-heptanediamide bis-AHX-DM1 compound **2A** (assumed quantitative yield, 29.7 mg) as a white solid m/z $[M+2H-2(H_2O)-NHCO]^{2+}$ 844 (100%), $[M+H]^+$ 1767.

Step 2: Synthesis of cytotoxic payload **1A.**

Compound **2** (assumed quantitative yield, 29.7 mg) was dissolved in formic acid (700 μL) and the solution stirred at room temperature for 1.5 h. Volatiles were removed *in vacuo* and the residue converted to the trifluroacetic acid salt by dissolving in a buffer A:buffer B 50:50 v/v% mixture (1.5 mL, buffer A (v/v): water:5% acetonitrile:0.05% trifluoroacetic acid and buffer B (v/v): acetonitrile:0.05% trifluoroacetic acid). The solution was stirred at room temperature for 5 min before the solvent was removed by lyophilisation. The process was repeated to give 4-(amino)-1,6-heptanediamide bis-AHX-DM1 cytotoxic payload **1A** as an off-white solid (18.0 mg, 60% over 2 steps) m/z $[M+2H-2(H_2O)-NHCO)]^{2+}$ 794 (100%), $[M+H]^+$ 1667.

Step 3: Synthesis of compound **8A.**

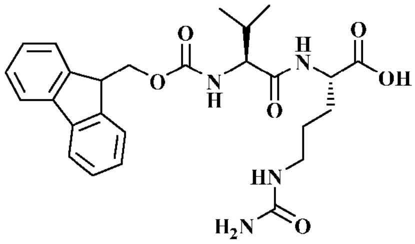

**8A**

Compound **8A** was synthesised following the procedure described in patent (EP 0 624 377 A2) to give a white solid with spectroscopic data in agreement with that previously reported.

Step 4: Synthesis of compound **9A**.

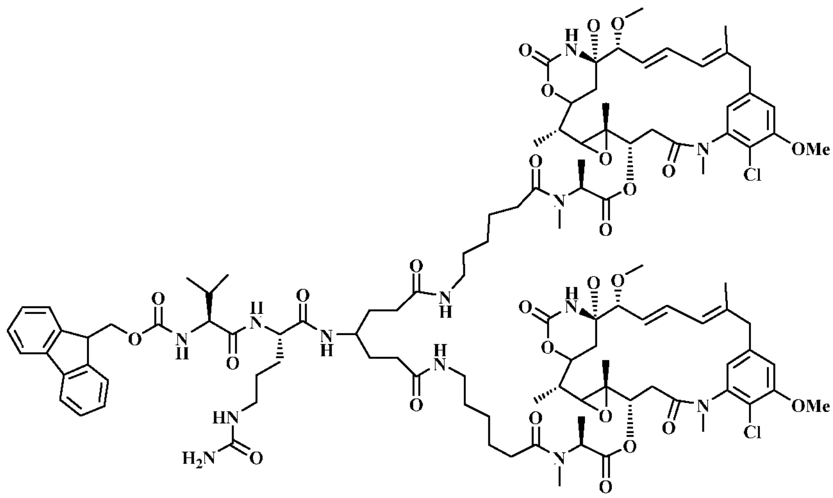

**9A**

Stock solutions of compound **8A** (20.0 mg) in DMF (500 μL) and HATU (40.0 mg) in DMF (400 μL) were prepared. To a stirred solution of compound **1A** (14.0 mg) in DMF (700 μL) was added aliquots of compound **8A** stock solution (126.9 μL) and HATU stock solution (77.8 μL). The reaction solution was cooled to 0 °C before the addition of DIPEA (4.11 μL). The solution was stirred at 0 °C for 50 min before further aliquots of compound **8A** stock solution (126.9 μL), HATU stock solution (77.8 μL) and DIPEA (4.11 μL) were added. The solution was stirred for 40 min at 0

°C. The reaction solution was purified by reverse phase C18-column chromatography eluting with buffer A (v/v): water:0.05% trifluoroacetic acid and buffer B (v/v): acetonitrile:0.05% trifluoroacetic acid (100:0 v/v to 0:100 v/v). The solvent was removed by lyophilisation to give 4-(Fmoc-val-cit-amido)-1,6-heptanediamide bis-AHX-DM1 compound **9A** (assumed quantitative yield, 16.9 mg) as an off-white solid m/z $[M+2H-2(H_2O)]^{2+}$ 1055 (100%).

Step 5: Synthesis of compound **10A.**

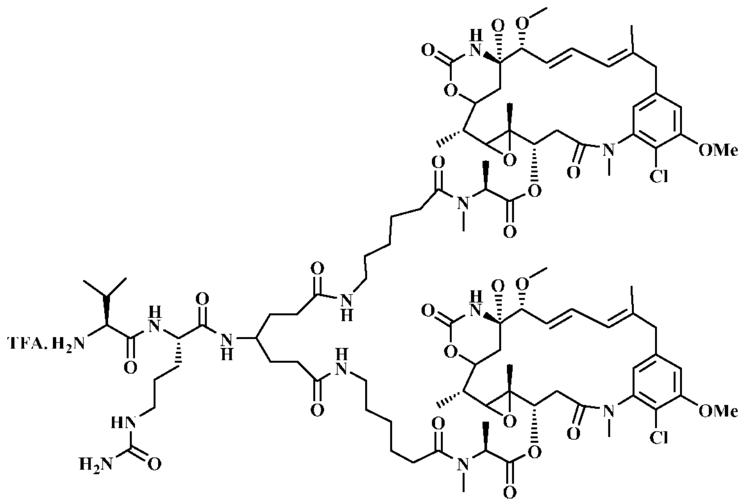

**10A**

To a stirred solution of compound **9A** (assumed quantitative yield, 16.9 mg) in DMF (500 μL) was added piperidine (3.04 μL). The reaction solution was stirred at room temperature for 1.5 h before purification by reverse phase C18-column chromatography eluting with buffer A (v/v): water:0.05% trifluoroacetic acid and buffer B (v/v): acetonitrile:0.05% trifluoroacetic acid (100:0 v/v to 0:100 v/v). The solvent was removed by lyophilisation to give 4-(val-cit-amido)-1,6-heptanediamide bis-AHX-DM1 compound **10A** as an off-white solid (8.8 mg, 55% over 2 steps) m/z $[M+2H]^{2+}$ 962 (100%).

Step 6: Synthesis of compound **12A**.

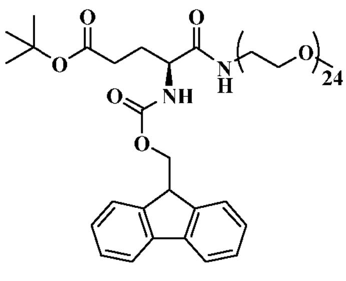

**12A**

A solution of Fmoc-L-Glu-(OtBu)-OH (36 mg) in DMF (2 mL) under an argon atmosphere was cooled to 0 °C and (benzotriazol-1-yloxy)tris-(dimethylamino) phosphonium hexafluorophosphate (BOP) (41 mg) was added followed by $NH_2$-PEG(24u)-OMe (100 mg) and DIPEA (19 μL). The solution was allowed to warm to room temperature and after 22 h the volatiles were removed *in vacuo.* The resulting residue was dissolved in dichloromethane (1 mL) and purified by normal phase column chromatography eluting with dichloromethane:methanol (100:0 v/v to 80:20 v/v). The organic solvent was removed *in vacuo* to give the Fmoc-Glu-(OtBu)-NH-PEG(24u)-OMe compound **12A** as a colourless oil (84 mg, 67%) m/z $[M+H]^+$ (1097, 10%), $[M+2H]^{2+}$ (1035,100%).

Step 7: Synthesis of compound **13A.**

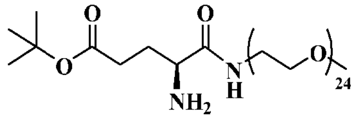

13A

To a solution of compound **12A** (74 mg) in DMF (2 mL) under an argon atmosphere was added piperidine (49 μL) and the resulting solution was stirred at room temperature. After 22 h, the volatiles were removed *in vacuo* and the resulting residue was triturated with hexane (3 × 0.7 mL). The organic solvent was decanted each time and resulting residue dried *in vacuo* to give the Glu-(OtBu)-NH-PEG(24u)-OMe compound **13A** as a solid (61 mg, 97%) m/z $[M+H]^+$ (1097, 10%), $[M+2H]^{2+}$ (1035,100%).

Step 8: Synthesis of compound **4A**

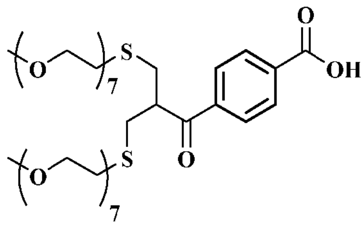

4A

To a stirred solution of 4-[2,2-bis[(p-tolylsulfonyl)-methyl]acetyl]benzoic acid (1.50 g, Nature Protocols, 2006, 1(54), 2241-2252) in DMF (70 mL) was added alpha-methoxy-omega-mercapto hepta(ethylene glycol) (3.20 g) and triethylamine (2.50 mL). The resulting reaction mixture was stirred under an inert nitrogen atmosphere at room temperature. After 19 h, volatiles were removed *in vacuo.* The resulting residue was dissolved in water (2.4 mL), and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give 4-[2,2-bis[alpha-methoxy-omega-thio-hepta(ethylene glycol)]acetyl]-benzoic acid compound **4A** as a thick clear colourless oil (1.77 g, 66%) m/z $[M+H]^+$ 901.

Step 9: Synthesis of compound **5A**.

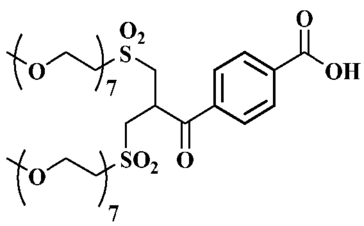

5A

To a stirred solution of **4A** (1.32 g) in methanol:water (18 mL, 9:1 v/v) at room temperature was added Oxone© (2.70 g). After 2.5 h, the volatiles were removed *in vacuo* and water was azeotropically removed with acetonitrile (2 × 15 mL). The resulting residue was dissolved in dichloromethane (3 × 10 mL), filtered through a column of magnesium

sulphate and washed with dichloromethane (2 × 7 mL). The eluent and washings were combined and the volatiles were removed *in vacuo* to give a thick clear pale yellow oil (1.29 g, 92%). A portion of the residue (700 mg) was dissolved in water:acetonitrile (1.50 mL, 3:1 v/v), and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give 4-[2,2-bis[alpha-methoxy-omega-sulfonyl hepta(ethylene glycol)]acetyl]benzoic acid reagent **5A** as a thick clear colourless oil (524 mg, 68%) m/z $[M+H]^+$ 965.

Step 10: Synthesis of compound **14A.**

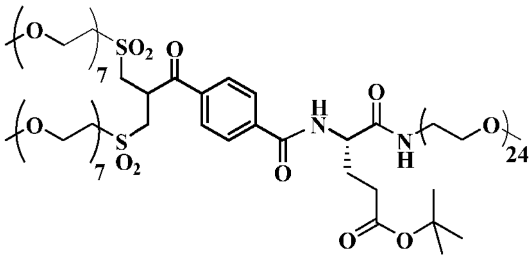

**14A**

A solution of compound **5A** (26.6 mg) in DMF (550 μL) was cooled to 0 °C under an argon atmosphere when HATU (10.5 mg) was added and the solution was stirred for 0.5 h at 0 °C. To this was added a solution of **13A** (32 mg) in DMF (550 μL) and the resulting solution was stirred for 5 min at 0 °C before addition of NMM (2.9 μL) and HATU (10.5 mg). The reaction solution was allowed to stir at 0 °C for 2 h before being warmed to room temperature and stirred for a further 3.5 h. After this time the volatiles were removed *in vacuo*,the resulting residue dissolved in water and acetonitrile (v/v; 1/1, 1.2 mL), and purified by reverse phase C18-column chromatography eluting with buffer A (v/v): water:5% acetonitrile:0.1% formic acid and buffer B (v/v): acetonitrile:0.1% formic acid (100:0 v/v to 0:100 v/v). The organic solvent was removed *in vacuo* and the aqueous solvent removed by lyophilisation to give the bis-mPEG(7u)sulfone-propanoyl-benzamide-Glu-(OtBu)-NH-PEG(24u)-OMe compound **14A** as a colourless oil (30.5 mg, 55%) m/z $[M+Na]^+$ (2243, 50%), $[M+H]^+$ (2221, 40%), $[M+Na+2H]^{3+}$ (747,100%).

Step 11: Synthesis of compound **15A.**

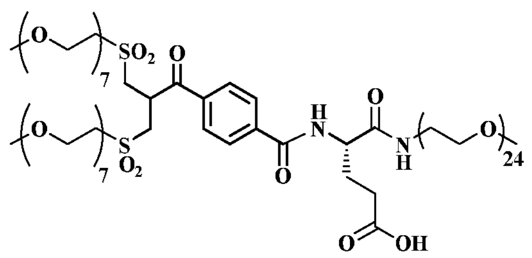

**15A**

A solution of compound **14A** (30 mg) in dichloromethane (2 mL) under an argon atmosphere was cooled to 0 °C after which trifluoroacetic acid (500 μL) was added and the resulting solution stirred for 1.5 h. The reaction mixture was allowed to warm to room temperature and stirred for a further 1 h, after which time the volatiles were removed *in vacuo.* The resulting residue was dissolved in water and acetonitrile (v/v; 1/1, 0.6 mL), and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give the bis-mPEG(7u)sulfone-propanoyl-benzamide-Glu-NH-PEG(24u)-OMe compound **15A** as a colourless oil (20 mg, 68%) m/z $[M+H]^+$ (2165, 55%), $[M+2H]^{2+}$ (1083, 60%), $[M+2H+Na]^{3+}$ (729, 100%).

Step 12: Synthesis of reagent **11A**.

Stock solutions of HATU (10 mg) in DMF (200 μL) and NMM (5.83 μL) in DMF (94.2 μL) were prepared. Compound **15A** (5.4 mg) was dissolved in a solution of compound **10A** (3.6 mg) in DMF (153.7 μL) with stirring. To the stirred solution was added an aliquot of HATU stock solution (40 μL). The solution was cooled to 0 °C before an aliquot of NMM stock solution (10 μL) was added. After 50 min, further aliquots of HATU stock solution (6.67 μL) and NMM stock solution (1.67 μL) were added. The reaction solution was stirred at 0 °C for a further 30 min and purified directly by reverse phase C18-column chromatography eluting with buffer A (v/v): water:0.05% trifluoroacetic acid and buffer B (v/v): acetonitrile:0.05% trifluoroacetic acid (100:0 v/v to 0:100 v/v). The solvent was removed by lyophilisation to give reagent **11A** as an off-white solid (3.8 mg, 53%) m/z $[M+4H-(H_2O)-NHCO]^{4+}$ 1003 (100%), $[M+3H-2(H_2O)-NHCO]^{3+}$ 1331, $[M+2H-2(H_2O)]^{2+}$ 2017.

**Example 9**: Synthesis of a disulfide bridging reagent **16A** comprising the cytotoxic payload **1A**.

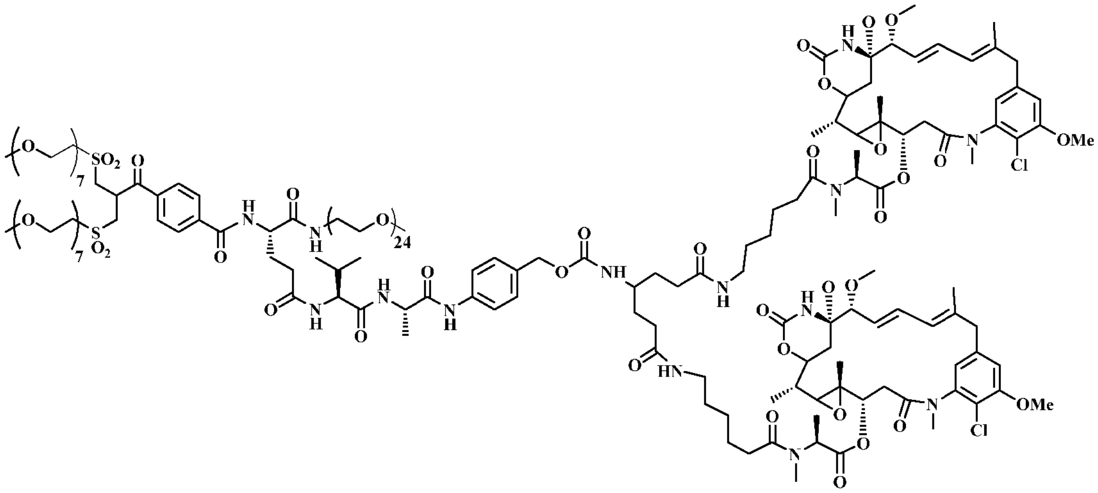

**16A**

Step 1: Synthesis of compound **17A.**

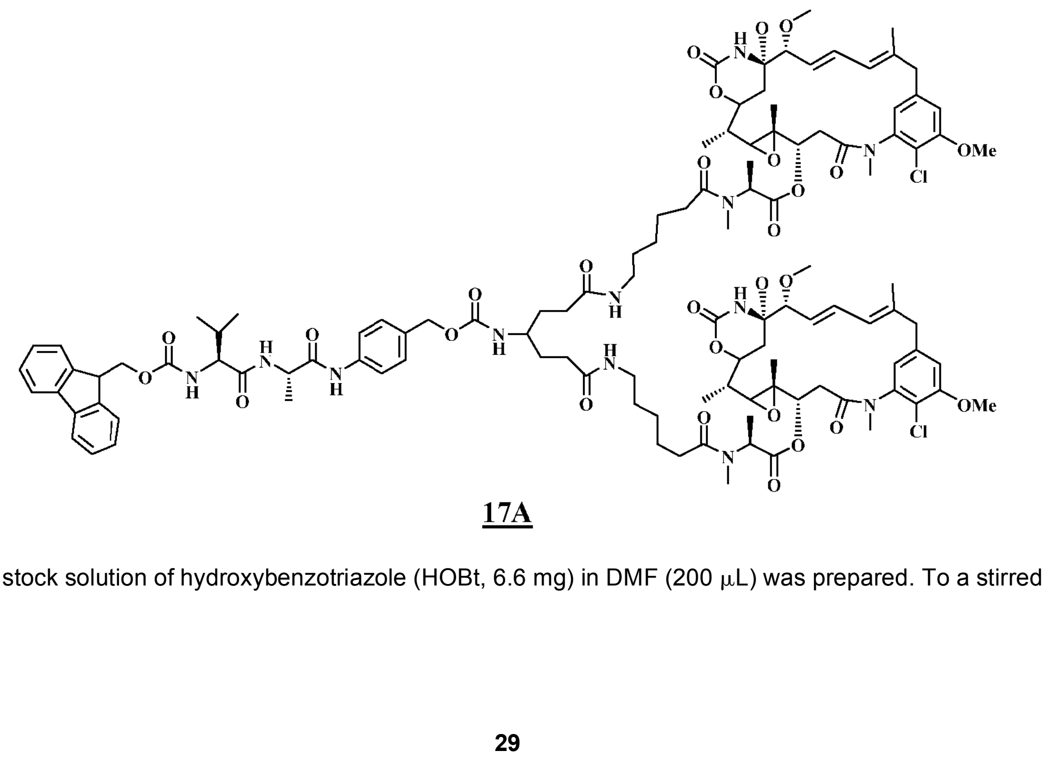

**17A**

A stock solution of hydroxybenzotriazole (HOBt, 6.6 mg) in DMF (200 μL) was prepared. To a stirred solution

of cytotoxic payload **1A** (10 mg) in DMF (500 μL) was added Fmoc-val-ala-PAB-PNP (3.7 mg) and an aliquot of HOBt stock solution (2 μL). The reaction solution was cooled to 0 °C before DIPEA (2.14 μL) was added. The reaction solution was then stirred at room temperature for 18 h before purification by reverse phase C18-column chromatography, eluting with buffer A (v/v): water:0.05% trifluoroacetic acid and buffer B (v/v): acetonitrile:0.05% trifluoroacetic acid (100:0 v/v to 0:100 v/v). The solvent was removed by lyophilisation to give Fmoc-val-ala-PAB-amido-1,6-heptanediamide bis-AHX-DM1 reagent **17A.**

Step 2: Synthesis of compound **18A.**

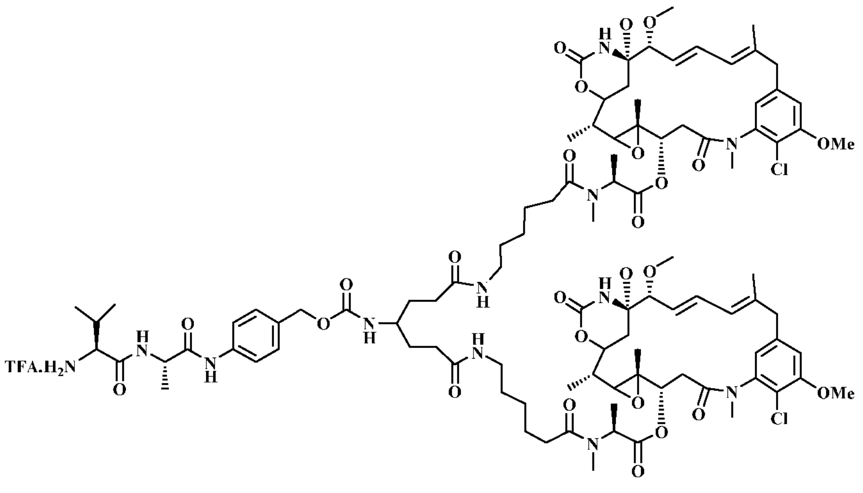

**18A**

The bis-maytansinoid compound amine-val-ala-PAB-amido-1,6-heptanediamide bis-AHX-DM1 **18A** was synthesised in an analogous way to that described for compound **10A**, using compound **17A** instead of compound **9A.**

Step 3: Synthesis of reagent **16A**.

The bis-maytansinoid reagent bis-mPEG(7u)sulfone-propanoyl-benzamide-Glu-[NH-PEG(24u)-OMe]-[val-ala-PAB-amido-1,6-heptanediamide bis-AHX-DM1] **16A** was synthesised in an analogous way to that described for reagent **11A,** using compound **18A** instead of compound **10A**.

**Example 10**: Synthesis of conjugation reagent **13** comprising an auristatin cytotoxic payload

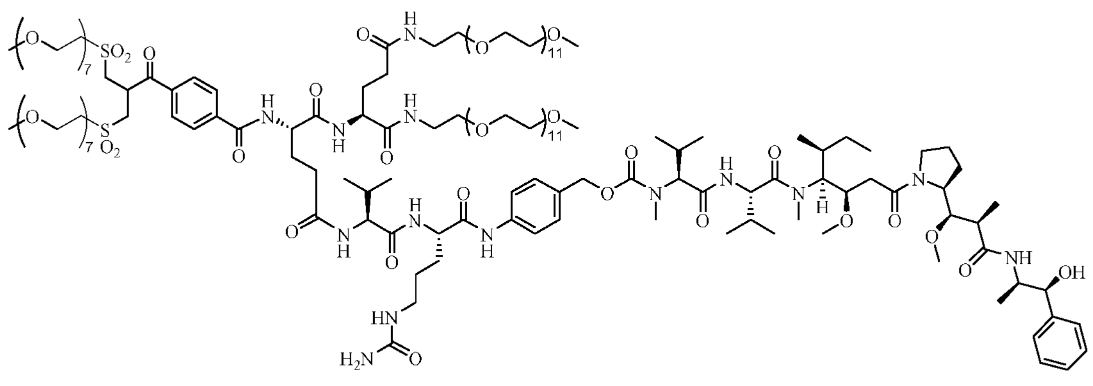

**13**

Step 1: Synthesis of compound **14**.

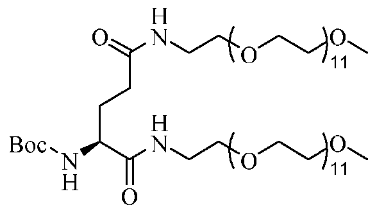

**14**

Boc-L-Glu (134.9 mg) and (benzotriazol-1-yloxy)tris-(dimethylamino) phosphonium hexafluorophosphate (BOP) (724 mg) were dissolved in anhydrous DMF (4 mL) and were stirred at 0 °C under a nitrogen atmosphere for 1.25 h. This solution was then added to a solution of $H_2N$-PEG(12u)-Me (685 mg) and NMM (179.8 μL) in DMF (3 mL). The solution was then stirred under $N_2$ for 4 h. The solution was then stirred 0-4 °C under a nitrogen atmosphere for 4.5 h. Further BOP (241 mg) and NMM (60 μL) were added, reaction mixture left for 24 h at 4 °C. The volatiles were removed *in vacuo* and the resulting residue was purified by reverse phase C18-flash chromatography eluting with eluting with buffer A (v/v): water: 5% acetonitrile:0.1% formic acid and buffer B (v/v): acetonitrile:0.1% formic acid (100:0 v/v to 65:35 v/v). The organic solvent was removed *in vacuo* and the aqueous solvent was removed by lyophilisation. The material was repurified by normal phase flash chromatography eluting with ethyl acetate:methanol (100:0 v/v to 0:100 v/v). The organic solvent was removed *in vacuo* and the aqueous solvent was removed by lyophilisation to give Boc-Glu-[PEG(12u)-Me]$_2$ compound **14** as a colourless oil (450 mg). m/z $[M+H]^+$ (1331, 100%), $[M+2H]^{2+}$ (665, 100%).

Step 2: Synthesis of compound **15**.

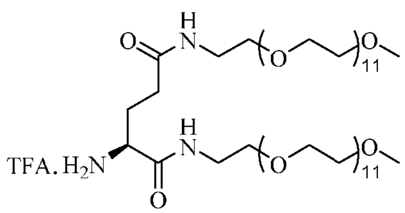

**15**

Compound **14** (450 mg) was dissolved in DCM (25 mL) to which was added TFA (2.5mL). The solution stirred at room temperature for 5 h. After which the volatiles were removed *in vacuo.* The resulting residue was purified by reverse phase C18-flash chromatography eluting with buffer A (v/v): water:5% acetonitrile:0.05% trifluoroacetic acid and buffer B (v/v): acetonitrile:0.05% trifluoroacetic acid (100:0 v/v to 60:40 v/v). The organic solvent was removed *in vacuo* and the aqueous solvent was removed by lyophilisation to give Glu-[HN-PEG(12u)-Me]$_2$ TFA compound **15** as a clear colourless gum (320 mg) m/z $[M+Na]^{1+}$ (1253.0, 10%) $[M+H]^{2+}$ (616.8, 100%)

Step 3: Synthesis of compound **16**

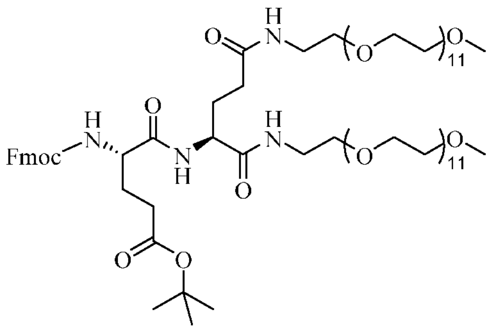

**16**

To a stirred solution of Fmoc-L-Glu-(OtBu)-OH (36.6 mg) in anhydrous DMF (2 mL) was added HATU (37.30 mg). The reaction mixture was stirred at 0 °C under a nitrogen atmosphere for 1 h and then added to a solution of compound **15** (103.5 mg) and NMM (19.2 μL) in DMF (1 mL). Additional DMF (1 mL) was added. The stirred reaction was left to warm to room temperature over 5 h. The volatiles were removed *in vacuo.* The resulting pale yellow oil was purified by reverse phase C18-flash chromatography eluting with buffer A (v/v): water: 5% acetonitrile:0.1% formic acid and buffer B (v/v): acetonitrile:0.1% formic acid (100:0 v/v to 50:50 v/v). The organic solvent was removed *in vacuo* and the aqueous solvent was removed by lyophilisation to give Fmoc-L-Glu-($O^tBu$)-Glu-[HN-PEG(12u)-Me]$_2$ compound **16** (173 mg) as a white paste. m/z $[M+1]^+$ (1638, 100%) & $[M+Na]^+$ (1660, 57%).

Step 4: Synthesis of compound **17**

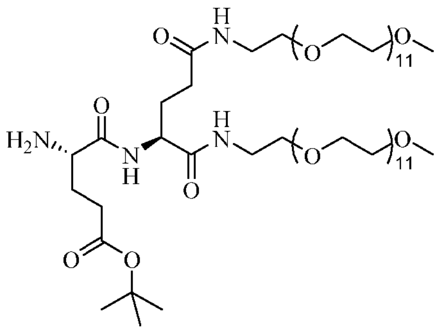

**17**

To a stirred solution of compound **16** (173 mg) in anhydrous DMF (3.2 mL) was added piperidine (104.4 μL). The solution was stirred at room temperature under argon for 1.5 h. The volatiles were removed *in vacuo* and the residue triturated repeatedly with hexane. The product was dried *in vacuo* to give L-Glu-($O^tBu$)-L-Glu-[HN-PEG(12u)-Me]$_2$ compound **17** (152 mg) as a clear colourless oil. m/z $[M+H]^{1+}$ (1416.7, 85%), $[M+2H]^{2+}$ (708.5, 100%), $[M+Na]^{1+}$ (1438.7, 30%)

Step 5: Synthesis of compound **18**

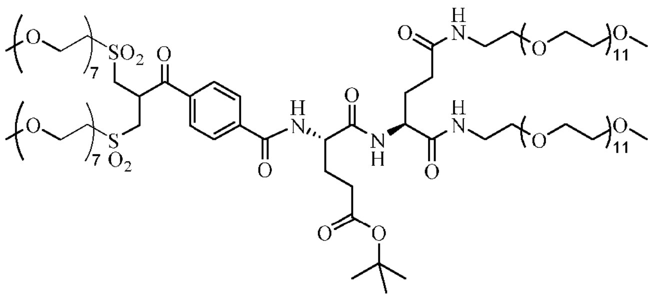

**18**

To a stirred solution of 4-[2,2-bis[alpha-methoxy-omega-sulfonyl hepta(ethylene glycol)]acetyl]benzoic acid (114 mg) in anhydrous DMF (3 mL) was added HATU (51.4 mg). Reaction mixture was stirred at 0 °C for 0.5 h then added to a solution of L-Glu($O^tBu$)-Glu-[HN-PEG(12u)-OMe]$_2$. (152.0 mg) in DMF (2 mL) and washed in with further DMF (1 mL), followed by NMM (14.8 µL). The reaction mixture was stirred at 0-15 °C for 3.5 h after which the volatiles were removed *in vacuo.* The resulting residue was purified by reverse phase C18-flash chromatography eluting with buffer A (v/v): water:5% acetonitrile:0.1% formic acid and buffer B (v/v): acetonitrile:0.1% formic acid (100:0 v/v to 55:45 v/v). The organic solvent was removed *in vacuo* and the aqueous solvent was removed by lyophilisation to give Bis-mPEG(7u)sulfone-propanoyl-benzamide -L-Glu-($O^tBu$)-Glu-[HN-PEG(12u)-Me]$_2$ compound **18** (160.6 mg) as a clear colourless oil. m/z $[M+H]^{1+}$ (2366.7, 100%), $[M+2H]^{2+}$ (1184.0, 80%) $[M+H_2O]^{3+}$ (795.5, 100%).

Step 6: Synthesis of compound **19**

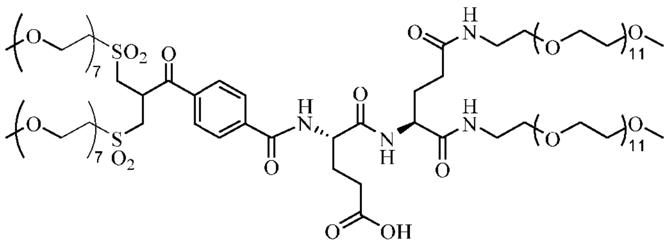

**19**

To the stirred solution of compound **18** (58 mg) in anhydrous DCM (6 mL) was added TFA (6.0 mL). Reaction mixture was stirred at room temperature for 2 h. after which the volatiles were removed *in vacuo,* dissolved in water (25 mL) and lyophilised to give Bis-mPEG(7u)sulfone-propanoyl-benzamide-L-Glu-(OH)-Glu-[HN-PEG(12u)-OMe]$_2$ compound **19** (160.6 mg) as a clear colourless oil. m/z $[M+H]^{1+}$ (2306.8, 90%), $[M+2H]^{2+}$ (1153.0, 100%).

Step 7: Synthesis of reagent **13**

Reagent **13** was synthesised in analogous way to reagent **8** of Example 3 from compound **7** and val-cit-PAB-MMAE TFA salt. Bis-mPEG(7u)sulfone-propanoyl-benzamide-L-Glu-(val-cit-PAB-MMAE)-Glu-[HN-PEG(12u)-Me]$_2$ **13** was isolated as a colourless oil (69%). m/z $[M+H]^{1+}$ (3410.4, 90%), $[M+2H]^{2+}$ (1706.2, 60%), $[M+3H]^{3+}$ (1137.2, 85%), $[M+4H]^{4+}$ (852.8, 70%).

Example 11: Synthesis of conjugation reagent **20** comprising an auristatin cytotoxic payload

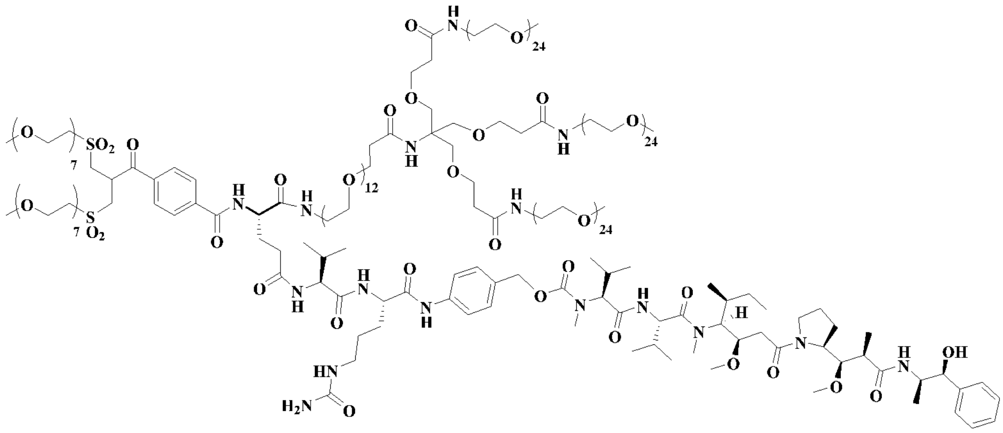

**20**

Reagent **20** was synthesised in analogous way to reagent **8** of Example 3 using compound **20B** instead of compound **7** and val-cit-PAB-MMAE TFA salt.

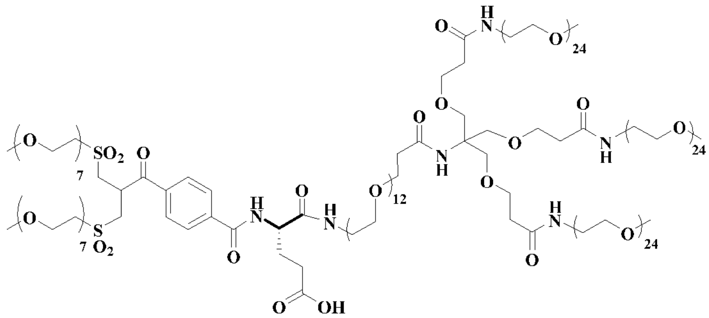

**20B**

Compound **20B** was made in an analogous way to compound **7** in Example 3, using $H_2N$-PEG(12u)-tri(m-dPEG(24u) instead of $H_2N$-PEG(24u). Bis-mPEG(7u)sulfone-propanoyl-benzamide-L-Glu-[val-cit-PAB-MMAE]-[PEG(12u)-tri(m-dPEG(24u))] **20** was isolated as a colourless oil. m/z $[M+2H]^{2+}$ (3166, 20%), $[M+3H]^{3+}$ (2111, 50%), $[M+4H]^{4+}$ (1583, 100%).

**Example 12**: Synthesis of conjugation reagent **21** comprising an auristatin cytotoxic payload

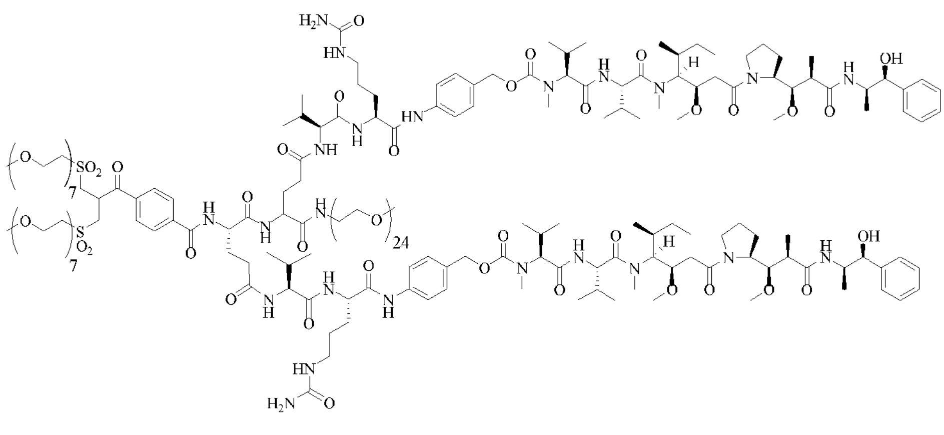

**21**

Step 1: Synthesis of compound **22.**

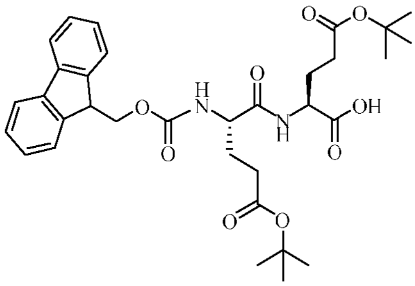

**22**

To a stirred solution of Fmoc-L-Glu-(O$^t$Bu)-OH (2000 mg) in anhydrous DMF (18 mL) was added HOBt (666 mg) and DIC (768 μL). The reaction mixture was stirred at 0 °C for 10 min and then 2.5 h at room temperature. H-L-Glu-(O$^t$Bu)-OH (1194 mg) and DIPEA (2464 μL) were added and the reaction mixture was stirred for 18 h at room temperature. The reaction mixture was diluted with water (100 mL) and acidified to pH 2.0 by adding diluted HCl. The aqueous layer was extracted with EtOAc (3 × 100 mL), and the organic phases combined and washed with water (2 × 50 mL) and saturated brine solution (1 × 50 mL). The EtOAc layer was dried over $Na_2SO_4$ for 2 h and then concentrated on a rotary evaporator. The product was isolated by reverse phase C18-flash chromatography eluting with buffer A (v/v): water: 5% acetonitrile: 0.1% formic acid and buffer B (v/v): acetonitrile: 0.1% formic acid (100:0 v/v to 80:20 v/v). The organic solvent was removed *in vacuo* and the aqueous solvent was removed by lyophilisation to give compound Fmoc-L-Glu-(OtBu)-L-Glu-(OtBu)-OH **22** (875 mg) as a white solid. m/z $[M+H]^{1+}$ (610.8, 85%), $[M+Na]^{1+}$ (633.1, 55%), $[2M+Na]^{+}$ (1243.2, 55%).

Step 2: Synthesis of compound **23**.

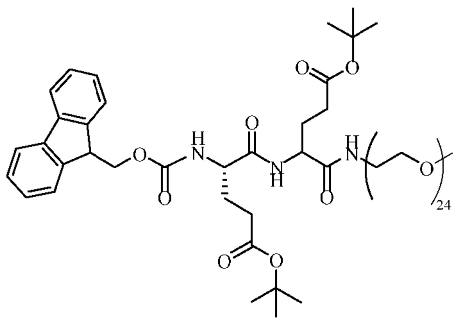

**23**

To a stirred solution of Fmoc-L-Glu-(OtBu)-L-Glu-(OtBu)-OH (510 mg) and $NH_2$-PEG(24u)-OMe (1000 mg) in anhydrous DMF (5 mL) was added and N,N-diisopropylethylamine (43.8 μL) and HATU (47.6 mg). The reaction mixture was stirred at 0 °C for 10 min and then 16 h at room temperature. The solution was concentrated *in vacuo* to 2 mL and the residue was purified by reverse phase C18-flash chromatography eluting with buffer A (v/v): water:5% acetonitrile:0.1% formic acid and buffer B (v/v): acetonitrile:0.1% formic acid (100:0 v/v to 83:17 v/v). The organic solvent was removed *in vacuo* and the aqueous solvent was removed by lyophilisation to give Fmoc-Glu-(OtBu)-Glu-(OtBu)-PEG(24u)-OMe compound **23** 644 mg) as a white paste. m/z $[M+H]^{1+}$ (1681.0, 40%), $[M+Na]^{1+}$ (1704.0, 30%) and $[M+2H]^{2+}$ (841.4, 55%).

Step 3: Synthesis of compound **24**.

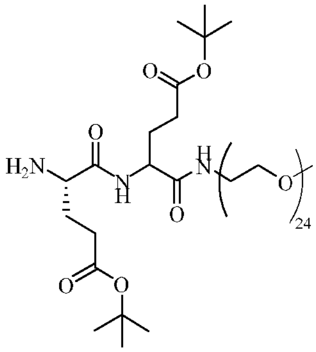

**24**

To a stirred solution of Fmoc-Glu-(OtBu)-Glu-(OtBu)-PEG(24u)-OMe (193 mg) in anhydrous DMF (900 μL) was added piperidine (34 μL) and the reaction mixture was stirred 1 h at room temperature. The solution was concentrated *in vacuo* to dryness and the residue triturated with $Et_2O$ (2 x 2.5 mL). The product was dried *in vacuo* to give H-L-Glu-(OtBu)-Glu-(OtBu)-PEG(24u)-OMe compound **24** (166 mg) as an off-white solid.

Step 4: Synthesis of compound **25**.

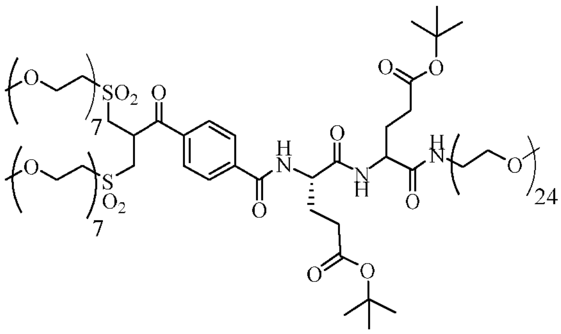

**25**

Reagent **25** was synthesised in analogous way to reagent **18** of Example 8 from compound **24** and 4-[2,2-bis[alpha-methoxy-omega-sulfonyl hepta(ethylene glycol)]acetyl]benzoic acid. Bis-mPEG(7u)sulfone-propanoyl-benzamide-Glu-(OtBu)-Glu-(OtBu)-PEG(24u)-OMe **25** was isolated as a colourless oil. m/z $[M+H]^{1+}$ (2407.2, 25%), $[M+Na]^{1+}$ (2429.4, 70%).

Step 5: Synthesis of compound **26**.

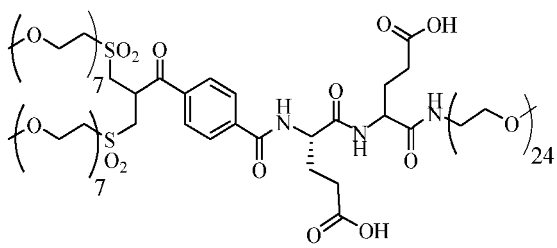

**26**

Reagent **26** was synthesised in analogous way to reagent **19** of Example 8 from compound **25.** Bis-mPEG(7u)sulfone-propanoyl-benzamide-Glu-(OH)-Glu-(OH)-PEG(24u)-OMe **26** was isolated as a colourless oil. m/z $[M+H]^{1+}$ (2294.2, 20%), $[M+Na]^{1+}$ (2317.4, 10%) and $[M+2Na]^{2+}$ (1217.4, 100%).

Step 6: Synthesis of reagent **21.**

To a stirred solution of compound **26** (28.1 mg), val-cit-PAB-MMAE TFA salt (30.6 mg) and HATU (13.9 mg) in anhydrous DMF (1.5 mL) was added N-methylmorpholine (6.7 μL) and the reaction mixture was stirred at 0 °C for 5 h. The solution was diluted with water (1 mL) and purified by reverse phase C18-flash chromatography eluting with buffer A (v/v): water:5% acetonitrile:0.1% TFA and buffer B (v/v): acetonitrile:0.1% TFA (100:0 v/v to 60:40 v/v). The organic solvent was removed *in vacuo* and the aqueous solvent was removed by lyophilisation to give bis-mPEG(7u)sulfone-propanoyl-benzamide-bis-[Glu-(val-cit-PAB-MMAE)]-PEG(24u)-OMe compound **21** (36.1 mg) as a white solid. m/z $[M+2H]^{2+}$ (2252.7, 20%), $[M+3H]^{3+}$ (1501.6.7, 40%) and $[M+4H]4^{+}$ (1126.6, 100%).

**Example 13.** Analysis of antibody drug conjugates (ADCs) by *in vitro* cell viability assay

The *in vitro* efficacy of the antibody conjugates and free payloads prepared in Example 5 were determined by measuring the inhibitory effect on cell growth of target over-expressing cancer cell lines.

Loss of tumour cell viability following treatment with ADCs or free payloads *in vitro* can be measured by growing cell lines in the presence of increasing concentrations of compounds and quantifying the loss of proliferation or metabolic activity using Cell-Titer Glo® Luminescent reagent (Promega). The protocol describes cell seeding, drug treatment and determination of the cell viability in reference to untreated cells based on ATP synthesis, which is directly correlated to the number of cells present in the well.

The characteristics of the cell line as well as the seeding density for the assay are described in Table 3 below.

Adherent JIMT-1 cells were detached with TrypLE and resuspended in complete medium. Cells were counted using disposable Neubauer counting chambers and cell density adjusted as detailed in Table 3 below. Cells were seeded (adherent cells at 100 μL/well and Karpas-299 at 50 μL/well) into Tissue Culture treated opaque-walled 96-well white plates and incubated for 24 hrs at 37°C and 5% $CO_2$.

Table 3

| Cell line | Target | Growth Medium | Seeding density |
|---|---|---|---|
| **JIMT-1** | $Her2^{low}$ | DMEM medium (Life Technologies®), 10% fetal bovine serum, 100 U/mL Penicillin and 100 μg/mL Streptomycin | 0.3 x $10^4$ cells per well |

Eight point serial dilutions of compounds were prepared in the relevant culture medium. The titration range was adjusted for each compound / cell line combination. In the case of adherent cells, the medium from the plate containing the cells was removed and replaced by 100 μL/well of the 1x serially diluted compounds.

The cell viability assay was carried out using the Cell-Titer Glo® Luminescent reagent (Promega), as described by the manufacturer.

Luminescence was recorded using a Molecular Devices SpectramaxM3 plate reader and data subsequently analysed using GraphPad Prism four parameter non-linear regression model.

Viability was expressed as % of untreated cells and calculated using the following formula:

$$\% Viability = 100 \times \frac{Luminescence_{Sample} - Luminescenc_{\ No\ cell\ Control}}{Luminescence_{Untreated} - Luminescence_{No\ cell\ Control}}$$

The % viability was plotted against the logarithm of drug concentration to extrapolate the $IC_{50}$ values for all conjugates.

Example 14: Karpas-299 mouse xenograft studies comparing Brentuximab-drug conjugate **10** with Brentuximab-drug conjugate **46** (comparative), Trastuzumab-drug conjugate **32** (negative control), and Adcetris® (comparative).

In this example, Trastuzumab-drug conjugate **32** was used as a negative control. Trastuzumab binds the target HER-2 which is not present, or present at very low levels, on Karpas-299 cells. Thus Trastuzumab-drug conjugate **32** is not specifically targeted at these cells and should only have a non-specific cytotoxic effect. Conversely, Brentuximab-drug conjugate **10** recognises the cell surface marker CD30, which is expressed upon Karpas-299 cells, targeting the

ADC specifically to these cells resulting in a specific cytotoxic effect.

Brentuximab drug conjugates **10** and **46** were prepared from conjugation reagents **9** and **45** respectively by the method described in Example 5. Trastuzumab drug conjugate **32** was prepared from conjugation reagent **1** by the method described in Example 5.

Healthy female CB17 SCID mice (CB17/lcr-Prkdcscid/Crl) with an average body weight of 19.7 g were used for cell inoculation. 24 to 72 hours prior to tumour cell injection, the mice were γ-irradiated (1.44 Gy, $^{60}$Co). The animals were maintained in SPF health status according to the FELASA guidelines in housing rooms under controlled environmental conditions.

Tumours were induced by subcutaneous injection of $10^7$ Karpas-299 cells (T-anaplastic large cell lymphoma, ALCL) in 200 μL of RPMI 1640 into the right flank. Tumours were measured twice a week with calipers, and the volume was estimated using the formula:

$$Tumor\ Volume\ (mm^3) = \frac{width^2 \times length}{2}$$

Fourteen days after tumour implantation, the animals were randomised into groups of five mice using Vivo manager® software (152.9 $mm^3$ mean tumor volume) and treatment was initiated (Day 0). All test substances were injected via the tail vein (i.v., bolus). Four 0.4 mg/kg doses of ADC were given every 4 days (Q4Dx4) and PBS was used for the vehicle group (Q4Dx4).

Mice viability and behaviour were recorded every day. Body weights were measured twice a week. The animals were euthanized when a humane endpoint was reached (e.g. 2,000 $mm^3$ tumour volume) or after a maximum of 6 weeks post-dosing.

The mean tumour volumes ± standard error are represented in Figures 4a to 4d for each treatment group. All compounds were well tolerated. Results show that in addition to an initial reduction in tumour volume, conjugate **10** displayed a greater and more prolonged inhibition of tumour growth than conjugate **46** or Adcetris®, whereas the negative control, **32**, had no discernible effect over the vehicle (no drug) control.

**Example 15:** JIMT-1 mouse xenograft studies comparing Trastuzumab-drug conjugate **27** to Kadcyla® (comparative).

Trastuzumab-drug conjugate **27** was prepared from reagent **9** by the method described in Example 5.

Healthy female NMRI nude mice (RjOrl:NMRI-*Foxn1$^{nu}$/Foxn1$^{nu}$*) aged 6 weeks at arrival were used for cell inoculation. The animals were maintained in SPF health status according to the FELASA guidelines in housing rooms under controlled environmental conditions.

Tumours were induced by subcutaneous injection of $5\times10^6$ JIMT-1 cells (breast carcinoma) in 200 μL of cell suspension in PBS into the right flank. Matrigel (40 μL Matrigel per 200 μL cell suspension) was added shortly before inoculation of tumour cells. Tumours were measured twice a week with calipers, and the volume was estimated using the formula:

$$Tumor\ Volume\ (mm^3) = \frac{width^2 \times length}{2}$$

When the tumour volumes reached a mean tumour volume of approximately 150 $mm^3$, the animals were randomised into groups of ten mice (128 $mm^3$ mean tumor volume) and treatment was initiated (Day 0). All test substances were injected via the tail vein (i.v., bolus). A single 5 mg/kg dose of ADC was given in 10 mL/kg and PBS was used for the vehicle group.

Mice viability and behaviour were recorded every day. Body weights were measured twice a week. The animals were euthanized when a humane endpoint was reached (e.g. calculated tumour weight of > 10% body weight, animal body weight loss of >20% compared to the body weight at group distribution, ulceration of tumours, lack of mobility, general signs of pain), or at a pre-determined study end date.

The mean tumour volume ± standard error is represented in Figures 5a and 5b for each group. Both compounds were well tolerated. Results show that conjugate **27** showed a complete reduction in tumour volume, showing greater activity than the commercial product, Kadcyla®, which had little effect over the vehicle control.

**Example 16:** Karpas-299 mouse xenograft study comparing Brentuximab-drug conjugates **10**, **28** and **29** to Adcetris® (comparative).

Conjugates **28** and **29** were prepared from conjugation reagent **21** from Example 12 and conjugation reagent **13** from Example 10, respectively. Conjugations were carried out as described in Example 5.

Healthy female CB17-SCID mice (CBySmn.CB17-Prkdcscid/J, Charles River Laboratories) with an average body weight of 18.9 g were used for cell inoculation (Day 0). 24 to 72 hours prior to tumour cell injection, the mice were γ-irradiated (1.44 Gy, $^{60}Co$). The animals were maintained in SPF health status according to the FELASA guidelines in housing rooms under controlled environmental conditions.

Tumours were induced by subcutaneous injection of $10^7$ Karpas-299 cells (T-anaplastic large cell lymphoma, ALCL) in 200 μL of RPMI 1640 into the right flank. Tumours were measured twice a week with calipers, and the volume was estimated using the formula:

$$Tumor\ Volume\ (mm^3) = \frac{width^2 \times length}{2}$$

Fifteen days after tumour implantation (Day 15), the animals were randomised into groups of eight mice using Vivo manager® software (169 $mm^3$ mean tumor volume) and treatment was initiated. The animals from the vehicle group received a single intravenous (i.v.) injection of PBS. The treated groups were dosed with a single i.v. injection of ADC at 1 mg/kg.

Treatment tolerability was assessed by bi-weekly body weight measurement and daily observation for clinical signs of treatment-related side effects. Mice were euthanized when a humane endpoint was reached (e.g. 1,600 $mm^3$ tumour volume) or after a maximum of 6 weeks post-dosing.

The mean tumour volume ± standard error is represented in Figures 6a, 6b, 6c and 6d for each group. All compounds were well tolerated. Results show that all conjugates displayed greater activity than Adcetris®, with conjugates **10** and **29** showing a complete reduction in tumour volume for the duration of the study.

**Example 17:** Karpas-299 mouse xenograft studies comparing Brentuximab-drug conjugates **10** and **30** to Adcetris® (comparative).

Conjugate **30** was prepared from conjugation reagent **20** from Example 11. Conjugations were carried out as described in Example 5.

This *in vivo* study was performed in a similar manner to that described in Example 16.

The mean body weight of the animals at the time of tumour induction (Day 0) was 19.9 g. Randomisation and treatment occurred at Day 15, when the mean tumour volume had reached 205 $mm^3$. The animals from the vehicle group received a single intravenous (i.v.) injection of PBS. The treated groups were dosed with a single i.v. injection of ADC at 1 mg/kg.

The mean tumour volume ± standard error is represented in Figures 7a, 7b and 7c for each group. All compounds were well tolerated. Results show that both conjugates **10** and **30** display greater tumour reducing activity than Adcetris®, with **10** displaying complete tumour reduction for the duration of the study.

Example 18: JIMT-1 mouse xenograft studies comparing Trastuzumab-drug conjugate **31** to Kadcyla® (comparative).

Conjugate **31** was prepared using conjugation reagent **5** from Example 2. Conjugations were carried out as described in Example 5.

The study was performed in a similar manner to that described in Example 15.

When the tumour volumes reached a mean tumour volume of approximately 150 $mm^3$, the animals were randomised into groups of ten mice (150 $mm^3$ mean tumor volume) and treatment was initiated (Day 0). All test substances were injected via the tail vein (i.v., bolus). A single dose of either 10 mg/kg or 30 mg/kg of ADC was given in 10 mL/kg and PBS was used for the vehicle group.

The mean tumour volume ± standard error is represented in Figures 8a and 8b for each group. All compounds were well tolerated. Results show that conjugate **31** reduces tumour volume, displaying greater activity than Kadcyla® at both doses.

**Example 19:** Pharmacokinetic analysis of ADCs possessing a pendant PEG group, a PEG group in series (i.e. non-pendant PEG group) and Adcetris®.

Brentuximab conjugate **10** was used in this study as the ADC with a pendant PEG group. Brentuximab conjugate **11** was used as a comparator with a non-pendant PEG group.

**In vivo pharmacokinetics in rats.** Sprague-Dawley rats (3 rats per group) with an average weight of 200 g were treated with either **10**, **11** or Adcetris®, via the tail vein (IV, bolus) at a single dose of 7 mg/kg. Serial sampling of 100 μL blood was performed before dosing and subsequently at 30 min, 24 h, 48 h, 7 d, 14 d and 35 d post-dosing respectively. Plasma was prepared from fresh blood samples and frozen at -80 °C until analysis.

**Total (anti-CD30 antibody) ELISA.** Total brentuximab antibody content from plasma samples was quantified using ELISA technology. Briefly, Maxisorp™ 96 well plates (Nunc/Fisher) were coated with recombinant human CD30, (Sino Biological Inc, 2.5 μg/mL in diluent) and incubated at 4 °C overnight. Plasma samples were diluted to ensure that the unknown ADCs fell within the linear range of the sigmoidal standard calibration curve. 100 μL of the diluted plasma sample was then transferred onto the CD30 coated plates and incubated for 3 h. After incubation, plates were washed 3x (200 μL/well) with PBS containing 0.05% Tween-20 (PBST) using a plate washer. HRP conjugated goat anti-human IgG, (Promega) was added to the plate and the samples were incubated for 1 hour at room temperature on a shaker at 350 rpm. The plate was then washed 3x with PBST and once with wash buffer using a plate washer. 100 μL of pre-warmed TMB was added and incubated for 7 minutes. The assay was stopped with 100 μL of 0.5 M $H_2SO_4$ and read at UV λ = 430 nm using the plate reader. Data were plotted and evaluated in GraphPad Prism 5 and Microsoft Excel.

**CD30 affinity capture for average DAR determination** Affinity capture was performed using streptavidin coated magnetic beads (Dynabeads-Streptavidin T1, Life Technologies). CD30 (Recombinant human CD30, Sino Biological Inc.) was biotinylated and immobilized on beads through streptavidin-biotin binding and finally blocked using skimmed milk peptides. 500 μL of the plasma sample in PBS was added to the CD30-coated beads and incubated overnight at 4 °C and finally washed using PBS. Captured antibodies were eluted using acidic elution buffer for 5 minutes at 4 °C. The eluate was subsequently neutralized to pH 7 using sodium acetate buffer, pH 8. Eluted samples were further mixed with HIC loading buffer and analysed using hydrophobic interaction chromatography with UV detection (HIC-UV).

**Hydrophobic interaction chromatography for average DAR determination.** Affinity captured ADCs were analysed using hydrophobic interaction chromatography in order to determine the average drug to antibody ratio (DAR). The method consisted of a linear gradient from 100 % buffer A (50 mM sodium phosphate pH 7.0, 1.5 M ammonium sulfate) to 100 % buffer B (50 mM sodium phosphate pH 7.0, 20% isopropanol) in 30 min using a TOSOH TSK gel Butyl-NPR HIC separation column with detection at 280 nm.

Figures 9 a and 9b show total antibody (μg/mL) and average DAR values for ADCs **10, 11** and Adcetris® in rats over a period of 850 h. The results show that conjugate **10** has the lowest rate of drug loss from the conjugate and the lowest rate of clearance from the circulation over this period. Conjugate **11** and Adcetris® (comparatives) show a much faster rate of drug dissociation and are cleared more quickly.

**Example 20:** Synthesis of conjugation reagent **33** (comparative) comprising an auristatin cytotoxic payload.

Conjugation reagent **33,** which contains a maleimide functional grouping, was synthesised as described within WO2015057699.

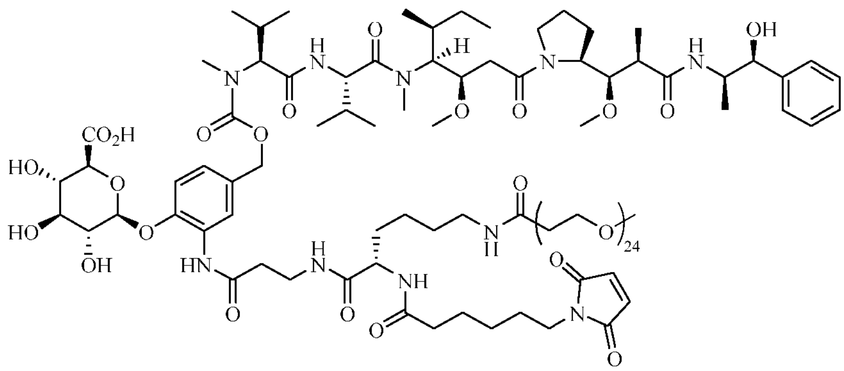

**33**

**Example 21:** Synthesis of conjugation reagent **35** comprising cytotoxic payload **36**.

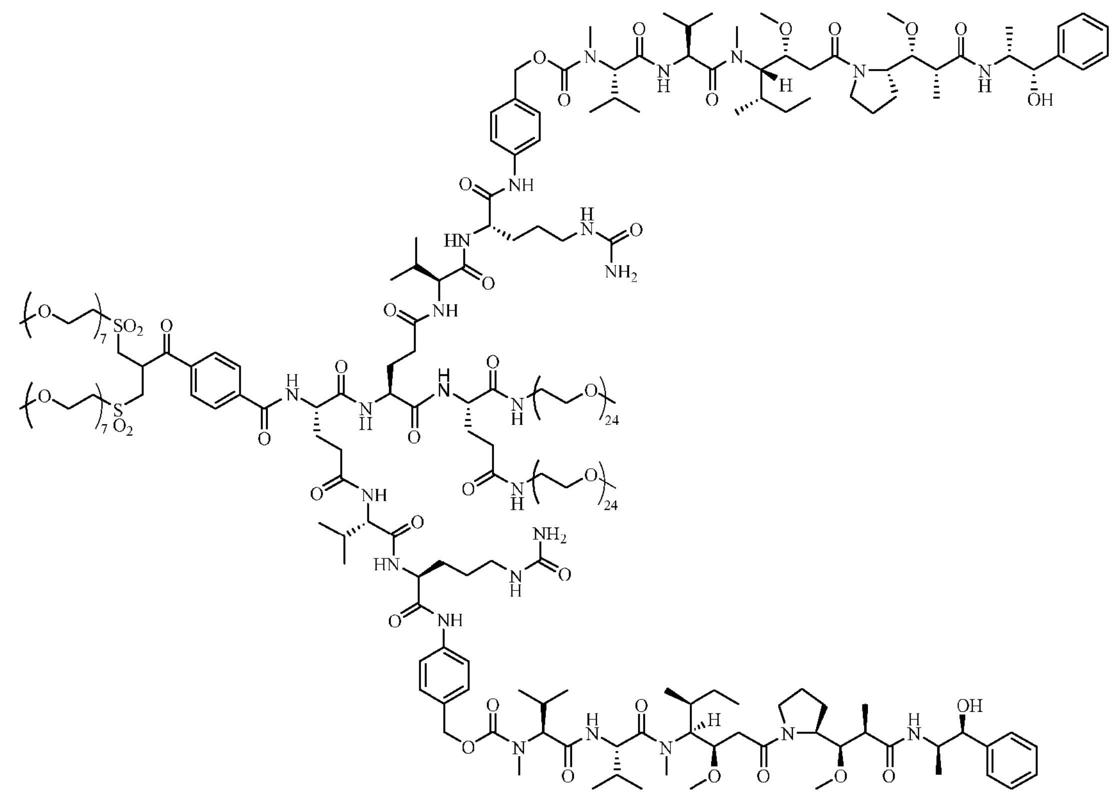

**35**

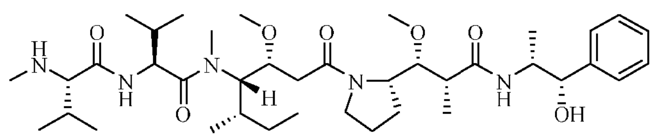

**36**

Step 1: Synthesis of compound **38.**

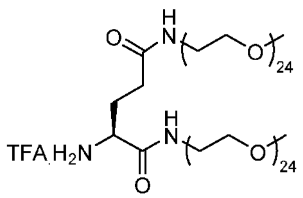

**38**

To a stirred solution of Boc-L-Glu(OH)-OH (51.6 mg) in anhydrous DMF (6 mL) was added BOP (277 mg). The solution was stirred at 0 °C for 20 min before MeO-PEG(24)-$NH_2$ (500 mg) was added followed by NMM (68.9 μL). After 4 h, additional amounts of BOP (92 mg) and NMM (23.0 μL) were added. After a further 2.5 h, the reaction mixture was stored at -20 °C for 18 h before being concentrated *in vacuo* and purified by reverse phase column C18-column chromatography, eluting with buffer A (v/v): water:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give a white solid (373 mg). Formic acid (6 mL) was added to the solid and the resulting mixture stirred under an inert atmosphere for 60 min before being concentrated *in vacuo.* The residue was dissolved in 95% water:5% acetonitrile:0.05% trifluoroacetic acid (~6 mL) and lyophilisation overnight to give TFA·$H_2N$-Glu(PEG(24)-OMe)-PEG(24)-OMe, compound **38,** as an off-white solid (330 mg). m/z $[M+2H]^{2+}$ (1144, 5%), $[M+3H]^{3+}$ (763, 35%), $[M+4H]^{+}$ (573, 100%).

Step 2: Synthesis of compound **39.**

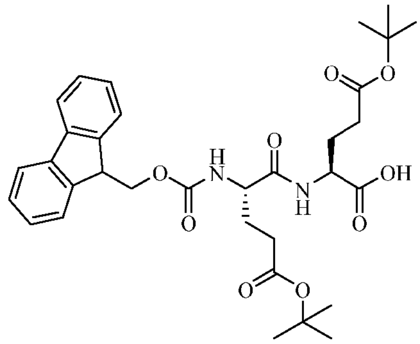

**39**

To a stirred solution of Fmoc-Glu(OtBu)-OH (2.00 g) in anhydrous DMF (18 mL) at 0 °C was added HOBt (666 mg) and DIC (768 μL). The reaction mixture was allowed to warm to RT and after 2 h, Glu(O$^t$Bu)-OH (1.19 g) and DIPEA (2.46 mL) were added. After stirring for 20 h, the reaction mixture was concentrated *in vacuo* and purified by reverse phase column C18-column chromatography, eluting with buffer A (v/v): water:0.05% trifluoroacetic acid and buffer B (v/v): acetonitrile:0.05% trifluoroacetic acid (100:0 v/v to 0:100 v/v)The organic solvent was removed *in vacuo* and the aqueous solvent removed by lyophilisation to give Fmoc-(L-Glu(O$^t$Bu))$_2$-OH, compound **39**, as a white solid (1.03 g). m/z [2M+H]$^+$ (1221, 15%), [M+H]$^+$ (611, 60%), [M-$^t$Bu+H]$^+$ (554, 65%), [M-2$^t$Bu+H]$^+$ (499, 100%).

Step 3: Synthesis of compound 40.

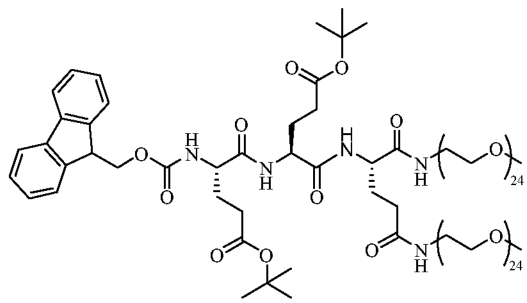

**40**

To a stirred solution of compound **38** (330 mg) in anhydrous DMF (10 mL) was added compound **39** (100 mg). At 0 °C, HATU (156 mg) and NMM (45.3 μL) were then added and the resulting solution stirred for 5 min before further addition of NMM (2.9 μL) and HATU (10.5 mg). The reaction solution was allowed to stir for another 20 min before being warmed to room temperature whereupon stirring was continued for a further 4 h. After this time, additional amounts of HATU (51.1 mg) and NMM (15.1 μL) were added. After a further 1.5 h, the mixture was stored at -20 °C for 18 h before being purified by reverse phase C18-column chromatography, eluting with buffer A (v/v): water:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give Fmoc-(L-Glu(O$^t$Bu))$_2$-L-Glu(PEG(24)-OMe)-PEG(24)-OMe, compound **40**, as a white solid (193 mg). m/z [M+3H]$^{3+}$ (961, 20%), [M-$^t$Bu+4H]$^{4+}$ (707, 100%), [M-2$^t$Bu+4H]$^{4+}$ (693, 85%), [M+5H]$^{5+}$ (577, 75%).

Step 4: Synthesis of compound **41.**

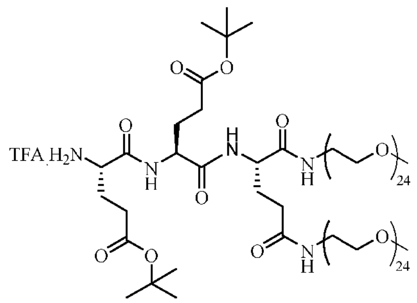

**41**

To a stirred solution of **41** (193 mg) in anhydrous DMF (1.5 mL) was added piperidine (19.8μL). After 90 min, a further amount of piperidine (13.2 μL) was added and the reaction stirred for another 90 min before being stored at -20 °C for 18 h. The solvent was removed under high vacuum and the resulting residue triturated in hexane. The residue was further dried under high vacuum for 30 min before being dissolved in a 50:50 mixture of buffer A:buffer B (2 mL, buffer A (v/v): water:0.05% trifluoroacetic acid and buffer B (v/v): acetonitrile:0.05% trifluoroacetic acid) and lyophilised overnight to give TFA·$H_2N$-(L-Glu($O^tBu$))$_2$-L-Glu(PEG(24)-OMe)-PEG(24)-OMe, compound **41,** as a pale blue solid (186 mg). m/z $[M+3H]^{3+}$ (887, 20%), [M+4H]4$^+$ (666, 100%), $[M+5H]^{5+}$ (533, 30%).

Step 5: Synthesis of compound **42.**

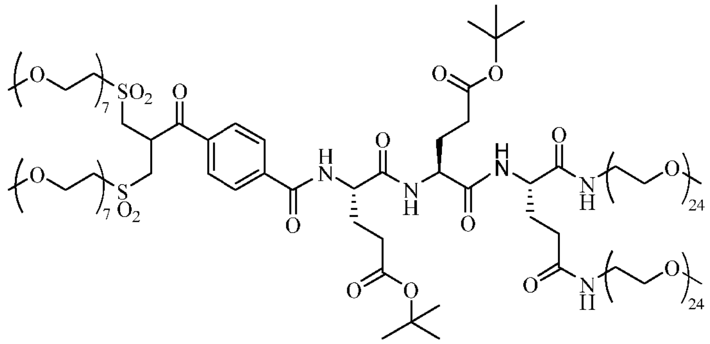

**42**

To a stirred solution of 4-[2,2-bis[alpha-methoxy-omega-sulfonyl hepta(ethylene glycol)]acetyl]benzoic acid (71.0 mg) in anhydrous DMF (1.5 mL) was added to HATU (27.9 mg). The mixture was cooled to 0 °C and stirred under an inert atmosphere for 30 min. A solution of **41** (186 mg) in anhydrous DMF (2.5 mL) was added, followed by HATU (22.9 mg) and NMM (14.7 μL), and the mixture allowed to warm to RT.

After 3 h, additional amounts of 4-[2,2-bis[alpha-methoxy-omega-sulfonyl hepta(ethylene glycol)]acetyl]benzoic acid (18.1 mg), HATU (50.8 mg) and NMM (15.1 μL) were added. After a further 1.5 h, further amounts of 4-[2,2-bis[alpha-methoxy-omega-sulfonyl hepta(ethylene glycol)]acetyl]benzoic acid (9.04 mg), HATU (50.8 mg) and NMM (15.1 μL) were added. The reaction mixture was stirred for a further 8 h and purified twice by reverse phase C18-column chromatography, eluting with buffer A (v/v): water:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give 42, as a pale yellow oil (assumed quantitative), m/z [M+4H]4$^+$ (902, 60%), $[M\text{-}^tBu+4H]^{4+}$ (888, 60%), $[M\text{-}2^tBu+4H]^{4+}$ (874, 45%), $[M\text{-}^tBu+5H]^{5+}$ (711, 100%).

Step 6: Synthesis of compound **43.**

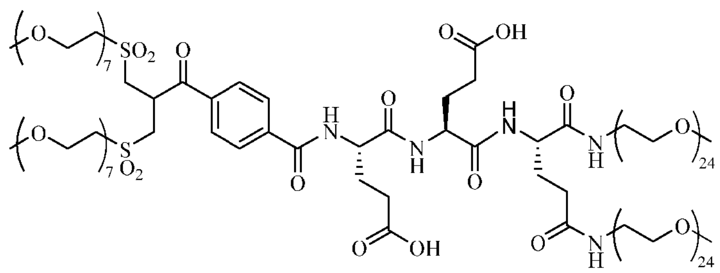

**43**

Formic acid (2 mL) was added to **42** under an inert atmosphere. The reaction mixture was stirred for 60 min before being concentrated *in vacuo.* The material was purified by reverse phase C18-column chromatography, eluting with buffer A (v/v): water:0.05% trifluoroacetic acid and buffer B (v/v): acetonitrile:0.05% trifluoroacetic acid (100:0 v/v to 0:100 v/v).

The organic solvent was removed *in vacuo* and the aqueous solvent removed by lyophilisation to give **43,** as a colourless oil (28.1 mg). m/z $[M+3H]^{3+}$ (1165, 5%), [M+4H]$4^{+}$ (874, 65%), $[M+5H]^{5+}$ (699, 100%).

Step 7: Synthesis of reagent **35**.

To a stirred solution of **43** (15.0 mg) in anhydrous DMF (270 μL) was added HATU (4.08 mg). The mixture was cooled to 0 °C and stirred under an inert atmosphere for 20 min. A solution of val-Cit-PAB-MMAE (12.2 mg) in anhydrous DMF (300 μL) was added, followed by HATU (2.45 mg) and NMM (1.89 μL), and the mixture allowed to warm to RT. After 4 h 20 min, additional amounts of HATU (3.3 mg) and NMM (0.94 μL) were added. The reaction mixture was stirred for a further 2 h before being stored at -20 °C for 18 h. Upon warming to RT, HATU (3.26 mg) and NMM (0.94 μL) were added to the stirred solution. After a 4.5 h, additional amounts of HATU (1.63 mg) and NMM (0.472 μL) were added and the reaction allowed to stir for a further 2.5 h before being stored at -20 °C for 18 h. The material was purified by reverse phase C18-column chromatography, eluting with buffer A (v/v): water:0.05% trifluoroacetic acid and buffer B (v/v): acetonitrile:0.05% trifluoroacetic acid (100:0 v/v to 0:100 v/v). The organic solvent was removed *in vacuo* and the aqueous solvent removed by lyophilisation to give **35,** as a white solid (13.8 mg). m/z $[M+4H]^{4+}$ (1426, 5%), $[M+5H]^{5+}$ (1141, 70%), $[M+6H]^{6+}$ (951, 100%), $[M+7H]^{7+}$ (815, 20%).

**Example 22:** Conjugation of reagents **33** (comparative) and **35** to Brentuximab to give Antibody-Drug Conjugates (ADCs) **34** (comparative) and **44.**

Conjugation reagent **33** was conjugated to Brentuximab, giving rise to ADC **34** using the methods described within WO2015057699, US7090843, Lyon et al., (2015) Nature Biotechnology, 33(7) p733-736 and Lyon et al., (2012), Methods in Enzymology, Volume 502, p123-137. Briefly, Brentuximab in 20 mM sodium phosphate buffer, pH 6.5 (150 mM NaCl, 20 mM EDTA) was reduced with TCEP (6 eq.) at 40 °C for 1 h. Conjugation of the reduced antibody with 2.0 molar equivalents of reagent **33** per free thiol was then performed). Reagent **33** was added to the mAb to give a final antibody concentration of 4 mg/mL. The solution was mixed gently and incubated at 22 °C for 2 h. After 2 h additional reagent **33** (0.2 molar equivalents) was added and the mixture was incubated for a further 1 h at 22 °C. Excess reagent **33** was quenched with N-acetyl-L-cysteine (20 eq. over reagent) and the crude reaction was purified using a 1 mL ToyoPearl Phenyl-650S HIC column equilibrated with 50 mM sodium phosphate, pH 7 (2 M NaCl). The ADC was eluted from the column with a gradient of 50 mM sodium phosphate, pH 7 (20% isopropanol). Fractions containing ADC were pooled and concentrated (Vivaspin20, 10 kDa PES membrane) to give an average DAR 8 product. The concentrated sample was buffer exchanged into DPBS, pH 7.1-7.5 and sterile filtered.

ADC **34** was difficult to purify and characterise due to the heterogeneity of the reaction products (number of DAR variants), leading to poor resolution of the indivdual DAR species by preparative HIC. Results showed that the final reaction product contained significant quantities of DAR species both greater than and less than DAR 8. Purifying the DAR8 species completely from these higher and lower than DAR8 species would result in significantly lower yields of DAR8 in the final product.

Conjugation reagent **35** was conjugated to Brentuximab, giving rise to ADC **44** using the conjugation procedure described in Example 5.

**Example 23:** Karpas-299 mouse xenograft study comparing Brentuximab-drug conjugates **34** and **44.**

This *in vivo* study was performed in a similar manner to that described in Example 16. The mean body weight of the animals at the time of tumour induction (Day 0) was 18.8 g. The number of animals per treatment group was 5. Randomisation and treatment occurred at Day 17, when the mean tumour volume had reached 167 $mm^3$. The animals from the vehicle group received a single intravenous (i.v.) injection of PBS. The treated groups were dosed with a single i.v. injection of ADC at 0.5 mg/kg. All compounds were well tolerated.

Figure 10 shows the % change in tumour volume for both ADCs **34** and **44** at Day 28. 100% was defined as the tumour volume at the first day of dosing (Day 17).

**Example 24:** Comparison of pendant PEG conjugates **34** (comparative) and **44** by thermal stress test.

ADC samples **34** and **44** were each prepared at 0.5 mg/mL by dilution with DPBS pH 7.1-7.5.

The two ADC samples were incubated at 65 °C for 30 minutes followed by incubation in an ice bath for 5 min before Size Exclusion Chromatography (SEC). SEC was performed using a TOSOH Bioscience TSK gel Super SW 3000 column. UV absorbance at 280 nm was monitored during an isocratic elution with a 2 M Potassium phosphate buffer, pH 6.8 (0.2 M potassium chloride and 15% isopropanol).

Tables 4a and 4b below show the conformations of ADCs **34** and **44** before and after thermal stress test, as measured by the area under the curve of each peak by Abs 280 nm, following SEC.

The results in Tables 4a and 4b show that ADC **44** remains in a non-aggregated state to a much greater extent than ADC **34** following thermal stress test. In addition, the results also show that **34** dissociates into lighter molecular weight components to a greater extent than conjugate **44**.

Table 4a

| ADC conformation before thermal stress test (% of total ADC) | **34** | **44** |
|---|---|---|
| Non-aggregated | 97.4 | 100 |
| Aggregated | 1.8 | 0 |
| Dissociated | 0.7 | 0 |

Table 4b

| ADC conformation after thermal stress test (% of total ADC) | **34** | **44** |
|---|---|---|
| Non-aggregated | 11.1 | 69.4 |
| Aggregated | 71 | 27.7 |
| Dissociated | 17.9 | 1.2 |

**Example 25:** Comparison of average DARs for pendant PEG conjugates **34** and **44** following incubation within human serum.

ADCs **34** and **44** were diluted to 0.1 mg/mL in human serum, 88 % (v/v) serum content. Each solution was immediately sub-aliquoted into 4 x 0.5 mL low-bind Eppendorf tubes. Two of the Eppendorf tubes, corresponding to the '0' time points were immediately transferred to the -80 °C freezer, whereas the rest of the samples were incubated at 37 °C for 6 days. After 6 days the appropriate samples were removed from the freezer and incubator for purification by affinity capture (CD30-coated magnetic beads), followed by analysis using hydrophobic interaction chromatography (HIC). CD30 affinity capture and HIC for average DAR determination were carried out as described in Example 19.

Figure 11 shows that after 6 days at 37 °C in human serum, conjugate **34** has lost much of its cytotoxic payload, whereas conjugate **44** remains largely unchanged, as indicated by the reduction in average DAR value of the sample. This indicates that the conjugate of the invention would have improved stability *in vivo.*

**Example 26:** Synthesis of conjugation reagent **45** (comparative) comprising an auristatin cytotoxic payload

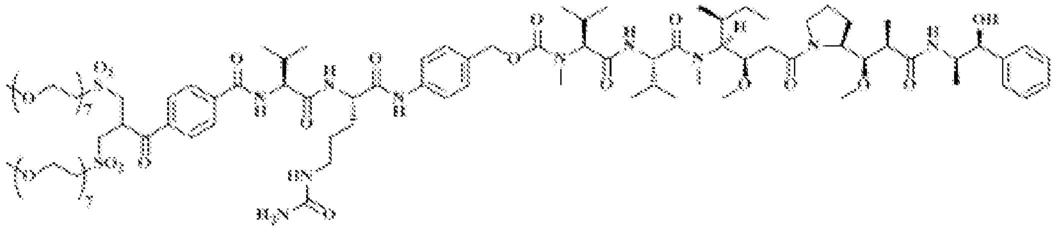

**45**

To the TFA salt of val-cit-PAB-MMAE salt having the structure below:

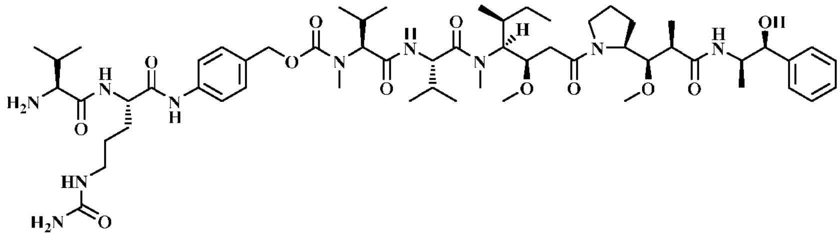

(25.0 mg) was added a solution of reagent **5A** (15.6 mg) in DMF (1.5 mL) and stirred under an inert nitrogen atmosphere at room temperature for 5 min. The mixture was cooled to 0 °C and aliquots of HATU (6.1 mg) and NMM (1.8 μL) were added every 20 min for a total of 5 additions. After 1.5 h, the reaction mixture was warmed to room temperature. After 2 h, volatiles were removed *in vacuo.* The resulting residue was dissolved in water and acetonitrile (v/v; 1/1, 0.6 mL), and purified by reverse phase C18-column chromatography eluting with buffer A (v/v): water:5% acetonitrile:0.05% trifluoroacetic acid and buffer B (v/v): acetonitrile:0.05% trifluoroacetic acid (100:0 v/v to 0:100 v/v). The organic solvent was removed *in vacuo* and the aqueous solvent was removed by lyophilisation to give bis-mPEG(7u)sulfone-propanoyl-benzamide-val-cit-PAB-MMAE reagent **45** as a white powder (22.4 mg, 68%) m/z [M+2H$^{2+}$] 1035.

## Claims

1. A conjugate of a protein or peptide with a therapeutic agent, said conjugate containing a protein or peptide bonding portion and having a linker which connects the therapeutic agent to the protein or peptide bonding portion, said linker including a polyethylene glycol portion; in which said protein or peptide bonding portion has the general formula:

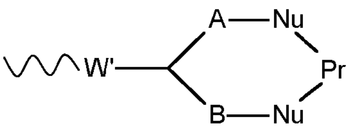

(I)

in which:

Pr represents said protein or peptide;
each Nu represents a sulfur atom or an amine group provided by a cysteine, lysine or histidine residue present in the protein or peptide Pr; or each Nu represents an imidazole group present in a polyhistidine tag attached to the protein or peptide Pr;
each of A and B independently represents a $C_{1-4}$alkylene or alkenylene chain; and
W' represents a keto group or a CH.OH group;
said polyethylene glycol portion is or includes a pendant polyethylene glycol chain which has a terminal end group of formula $-CH_2CH_2OR$, in which R represents a hydrogen atom, an alkyl group, or an optionally substituted aryl group, and wherein said pendant polyethylene glycol chain has a number average molecular weight of up to 75,000 glmole;
the linker includes the grouping:

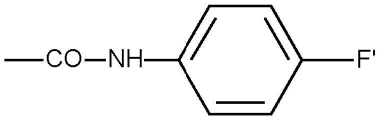

(Va) or

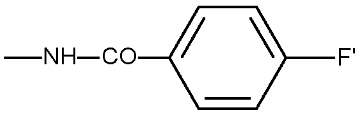

(Vb)

in which F' represents said protein or peptide bonding portion of formula I; and the protein or peptide is a receptor or ligand binding protein, an antibody or antibody fragment.

2. A conjugate as claimed in claim 1, in which R represents a hydrogen atom or a $C_{1-4}$alkyl group.

3. A conjugate as claimed in either claim 1 or claim 2, in which said pendant polyethylene glycol chain contains from 2 to 50 polyethylene glycol units.

4. A conjugate as claimed in any one of the preceding claims, in which each Nu represents a sulfur atom present in a cysteine residue in the protein or peptide Pr.

5. A conjugate as claimed in any one of the preceding claims, wherein the therapeutic agent is a cytotoxic agent or a toxin.

6. A conjugate as claimed in any one of the preceding claims, in which the protein is an antibody or antibody fragment.

7. A conjugate as claimed in any one of the preceding claims, in which said protein or peptide bonding portion has the formula

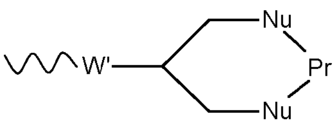

(Ia).

8. A conjugate as claimed in any preceding claims, wherein the linker includes a group which is susceptible to enzymatic degradation.

9. A conjugate as claimed in claim 8, wherein the group susceptible to enzymatic degradation is:

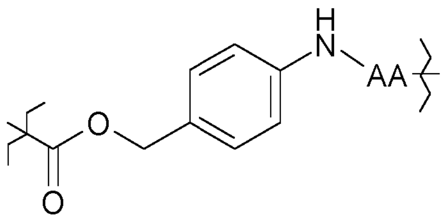

in which AA represents a protease-specific amino acid sequence.

10. A conjugate as claimed in claim 9, wherein the amino acid sequence comprises 1 to 5 amino acids.

11. A conjugating reagent capable of reacting with a protein or peptide, and including a therapeutic agent, said reagent having a linker which connects the therapeutic agent to a functional grouping, said linker including a polyethylene glycol portion; said functional grouping having the formula:

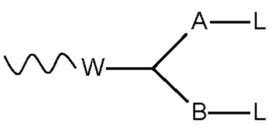

(II) or

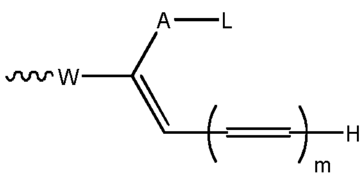

(II')

in which:

W represents a keto group or a CH.OH group;
each of A and B independently represents a $C_{1-4}$alkylene or alkenylene chain;
each L independently represents a leaving group;
said polyethylene glycol portion is or includes a pendant polyethylene glycol chain which has a terminal end group of formula $-CH_2CH_2OR$, in which R represents a hydrogen atom, an alkyl group, or an optionally substituted aryl group, and wherein said pendant polyethylene glycol chain has a number average molecular weight of up to 75,000 glmole;
the linker includes the grouping:

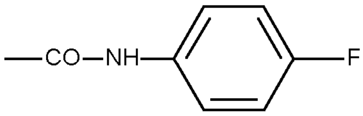
(VIa) or
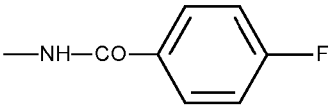
(VIb)

in which F represents the functional grouping of formula II or II'.

**12.** A conjugating reagent as claimed in claim 11, in which R represents a hydrogen atom or a $C_{1-4}$alkyl group.

**13.** A conjugating reagent as claimed in either claim 11 or claim 12, in which said pendant polyethylene glycol chain contains from 2 to 50 polyethylene glycol units.

**14.** A conjugating reagent as claimed in any one of claims 11 to 13, wherein the therapeutic agent is a cytotoxic agent or a toxin.

**15.** A conjugating reagent as claimed in any one of claims 11 to 14, in which said functional grouping has the formula

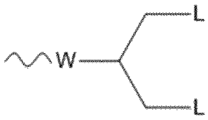
(IIa) or
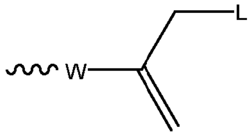
(II'a)

**16.** A conjugating reagent as claimed in any one of claims 11 to 15, in which W represents a keto group.

**17.** A conjugating reagent as claimed in any one of claims 11 to 17, in which each L represents -SP, -OP, $-SO_2P$, $-OSO_2P$, $-N^+PR^2R^3$, halogen, or -OØ, in which P represents a hydrogen atom or an alkyl, aryl, or alkyl-aryl group, or is a group which includes a portion $-(CH_2CH_2O)_n-$ in which n is a number of two or more, and each of $R^2$ and $R^3$ independently represents a hydrogen atom, a $C_{1-4}$alkyl group, or a group P, and Ø represents a substituted aryl group containing at least one electron withdrawing substituent.

**18.** A conjugating reagent as claimed in any one of claims 11 to 17, in which the linker includes a group which is susceptible to enzymatic degradation.

**19.** A conjugating reagent as claimed in claim 18, wherein the group susceptible to enzymatic degradation is:

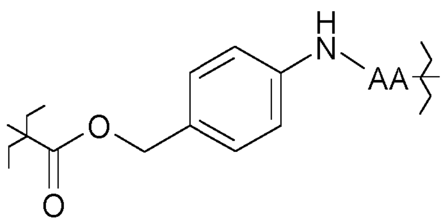

in which AA represents a protease-specific amino acid sequence.

20. A process for the preparation of a conjugate as claimed in any one of claims 1 to 10, which comprises reacting a conjugation reagent as claimed in any one of claims 11 to 19 with a protein or a peptide.

**Patentansprüche**

1. Protein- oder Peptidkonjugat mit einem therapeutischen Wirkstoff, wobei das Konjugat einen Protein- oder Peptidbindungsteil enthält und einen Linker hat, der den therapeutischen Wirkstoff mit dem Protein- oder Peptidbindungsteil verbindet, wobei der Linker einen Polyethylenglycolteil enthält; wobei der Protein- oder Peptidbindungsteil die allgemeine Formel

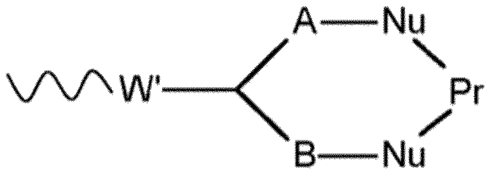

(I)

hat, in welcher
Pr das Protein oder Peptid repräsentiert;
jedes Nu ein Schwefelatom oder eine Amingruppe, die von einem Cystein-, einem Lysin- oder einem Histidinrückstand, der in dem Protein oder Peptid Pr vorhanden ist, bereitgestellt wird, repräsentiert; oder jedes Nu eine Imidazolgruppe, die in einem Polyhistidintag, der an das Protein oder Peptid Pr gebunden ist, vorhanden ist, repräsentiert; jedes A und B unabhängig ein $C_{1-4}$-Alkylen oder eine Alkylenkette repräsentieren; und
W' eine Ketogruppe oder eine CHOH-Gruppe repräsentiert;
wobei der Polyethylenglycolteil eine hängende Polyethylenglycolkette ist oder enthält, die eine terminale Endgruppe der Formel $-CH_2CH_2OR$ hat, in der R ein Wasserstoffatom, eine Alkylgruppe, oder eine optional substituierte Arylgruppe repräsentiert, und wobei die hängende Polyethylenglycolkette ein zahlengemitteltes Molekulargewicht von bis zu 75 000 g/mol hat;
wobei der Linker die Gruppierung

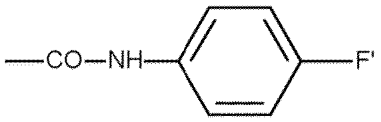

(Va) oder

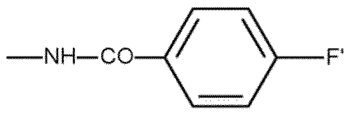

(Vb)

enthält, in welcher F' den Protein- oder Peptidbindungsteil nach Formel I repräsentiert; und das Protein oder Peptid ein Rezeptor- oder Ligandbindeprotein, ein Antikörper oder ein Antikörperfragment ist.

2. Konjugat nach Anspruch 1, wobei R ein Wasserstoffatom oder eine $C_{1-4}$-Alkylgruppe repräsentiert.

3. Konjugat nach Anspruch 1 oder 2, wobei die hängende Polyethylenglycolkette von 2 bis 50 Polyethylenglycoleinheiten enthält.

4. Konjugat nach einem der vorhergehenden Ansprüche, wobei jedes Nu ein Schwefelatom, das in einem Cysteinrückstand in dem Protein oder Peptid Pr vorhanden ist, repräsentiert.

5. Konjugat nach einem der vorhergehenden Ansprüche, wobei der therapeutische Wirkstoff ein cytotoxischer Wirkstoff oder ein Giftstoff ist.

6. Konjugat nach einem der vorhergehenden Ansprüche, wobei das Protein ein Antikörper oder Antikörperfragment ist.

7. Konjugat nach einem der vorhergehenden Ansprüche, wobei der Protein- oder Peptidbindungsteil die Formel

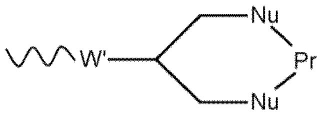

(Ia).

hat.

8. Konjugat nach einem der vorhergehenden Ansprüche, wobei der Linker eine Gruppe enthält, die enzymatisch abgebaut werden kann.

9. Konjugat nach Anspruch 8, wobei die Gruppe, die enzymatisch abgebaut werden kann,

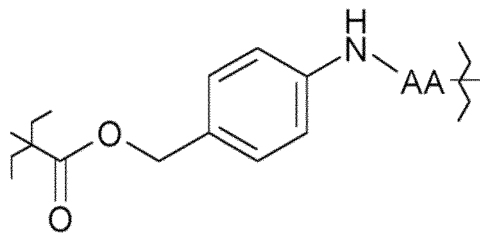

ist, wobei AA eine Protease-spezifische Aminosäuresequenz repräsentiert.

10. Konjugat nach Anspruch 9, wobei die Aminosäuresequenz 1 bis 5 Aminosäuren enthält.

11. Konjugierendes Reagenz, das mit einem Protein oder Peptid reagieren kann, und einen therapeutischen Wirkstoff enthält, wobei das Reagenz einen Linker hat, der den therapeutischen Wirkstoff mit einer funktionalen Gruppierung verbindet, wobei der Linker einen Polyethlyenglycolteil enthält; wobei die funktionale Gruppierung die Formel

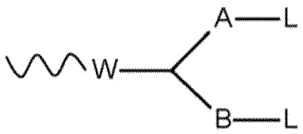

(II) oder

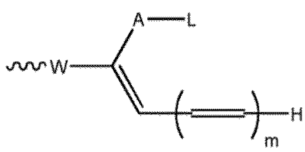

(II')

hat, wobei

W eine Ketogruppe oder eine CHOH-Gruppe repräsentiert;
jedes A und B unabhängig ein $C_{1-4}$-Alkylen oder eine Alkylenkette repräsentieren;
jedes L unabhängig eine Abgangsgruppe repräsentiert;
der Polyethylenglycolteil eine hängende Polyethylenglycolkette ist oder enthält, die eine terminale Endgruppe der Formel $CH_2CH_2OR$ hat, in der R ein Wasserstoffatom, eine Alkylgruppe, oder eine optional substituierte Arylgruppe repräsentiert, und wobei die hängende Polyethylenglycolkette ein zahlengemitteltes Molekulargewicht von bis zu 75 000 g/mol hat;
der Linker die Gruppierung

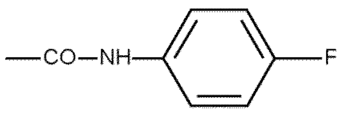

(VIa) oder

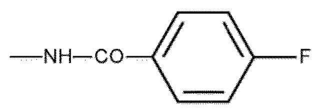

(VIb)

enthält,
wobei F die funktionelle Gruppierung nach Formel II oder II' repräsentiert.

**12.** Konjugierendes Reagenz nach Anspruch 11, wobei R ein Wasserstoffatom oder eine $C_{1-4}$-Alkylgruppe repräsentiert.

**13.** Konjugierendes Reagenz nach Anspruch 11 oder 12, wobei die hängende Polyethylenglycolkette von 2 bis 50 Polyethylenglycoleinheiten enthält.

**14.** Konjugierendes Reagenz nach einem der Ansprüche 11 bis 13, wobei der therapeutische Wirkstoff ein cytotoxischer Wirkstoff oder ein Giftstoff ist.

**15.** Konjugierendes Reagenz nach einem der Ansprüche 11 bis 14, wobei die funktionelle Gruppierung die Formel

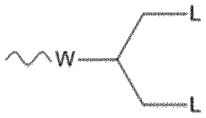
(IIa)
oder
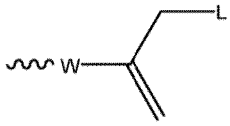
(II'a)

hat.

**16.** Konjugierendes Reagenz nach einem der Ansprüche 11 bis 15, wobei W eine Ketogruppe repräsentiert.

**17.** Konjugierendes Reagenz nach einem der Ansprüche 11 bis 17, wobei jedes L -SP, -OP, $-SO_2P$, $-OSO_2P$, $-N^+PR^2R^3$, Hallogen, oder -OØ repräsentiert, wobei P ein Wasserstoffatom oder eine Alkyl-, Aryl-, oder Alkylarylgruppe repräsentiert, oder ein Gruppe ist, die einen Teil $-(CH_2CH_2O)_n-$ enthält, wobei n eine Zahl von zwei oder mehr ist, und wobei jedes $R^2$ und $R^3$ unabhängig ein Wasserstoffatom, eine $C_{1-4}$-Alkylgruppe, oder eine P-Gruppe repräsentieren, und wobei Ø eine substituierte Arylgruppe repräsentiert, die mindestens einen Elektronen-entziehenden Substituenten enthält.

**18.** Konjugierendes Reagenz nach einem der Ansprüche 11 bis 17, wobei der Linker eine Gruppe enthält, die enzymatisch abgebaut werden kann.

**19.** Konjugierendes Reagenz nach Anspruch 18, wobei die Gruppe, die enzymatisch abgebaut werden kann,

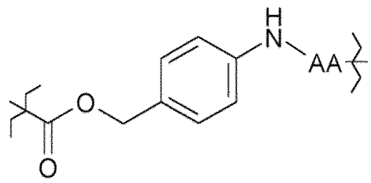

ist,
wobei AA eine Protease-spezifische Aminosäuresequenz repräsentiert.

**20.** Verfahren zur Herstellung eines Konjugats nach einem der Ansprüche 1 bis 10, das das Reagieren eines konjugierenden Reagenz nach einem der Ansprüche 11 bis 19 mit einem Protein oder Peptid enthält.

**Revendications**

**1.** Conjugué d'une protéine ou d'un peptide avec un agent thérapeutique, ledit conjugué contenant une portion de liaison protéique ou peptidique et ayant un lieur qui relie l'agent thérapeutique à la portion de liaison protéique ou peptidique, ledit lieur comportant une portion polyéthylène glycol ; dans lequel ladite portion de liaison protéique ou peptidique est de formule générale :

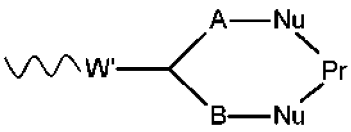

(I)

dans laquelle :

Pr représente ladite protéine ou ledit peptide ;
chaque Nu représente un atome de soufre ou un groupe amine fourni par un résidu cystéine, lysine ou histidine présent dans la protéine ou le peptide Pr ; ou chaque Nu représente un groupe imidazole présent dans une étiquette polyhistidine attachée à la protéine ou au peptide Pr ;
chacun de A et B représente indépendamment une chaîne alkylène ou alcénylène en $C_{1-4}$ ; et
W représente un groupe céto ou un groupe CH.OH ;
ladite portion polyéthylène glycol est ou comporte une chaîne polyéthylène glycol pendante qui a un groupe terminal de formule $-CH_2CH_2OR$, dans laquelle R représente un atome d'hydrogène, un groupe alkyle, ou un groupe aryle éventuellement substitué, et où ladite chaîne polyéthylène glycol pendante a un poids moléculaire moyen en nombre allant jusqu'à 75 000 g/mole ;
le lieur comporte le groupement :

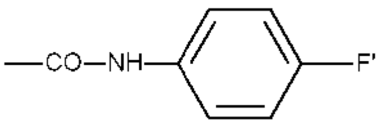

(Va) or

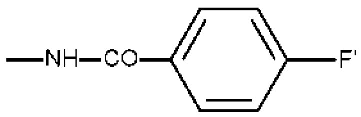

(Vb)

dans lequel F' représente ladite portion de liaison protéique ou peptidique de formule I ; et la protéine ou le peptide est une protéine de liaison à un récepteur ou à un ligand, un anticorps ou un fragment d'anticorps.

**2.** Conjugué selon la revendication 1, dans lequel R représente un atome d'hydrogène ou un groupe alkyle en $C_{1-4}$.

**3.** Conjugué selon la revendication 1 ou la revendication 2, dans lequel ladite chaîne polyéthylène glycol pendante contient 2 à 50 motifs polyéthylène glycol.

**4.** Conjugué selon l'une quelconque des revendications précédentes, dans lequel chaque Nu représente un atome de soufre présent dans un résidu cystéine dans la protéine ou le peptide Pr.

**5.** Conjugué selon l'une quelconque des revendications précédentes, où l'agent thérapeutique est un agent cytotoxique ou une toxine.

**6.** Conjugué selon l'une quelconque des revendications précédentes, dans lequel la protéine est un anticorps ou fragment d'anticorps.

**7.** Conjugué selon l'une quelconque des revendications précédentes, dans lequel ladite portion de liaison protéique ou peptidique est de formule

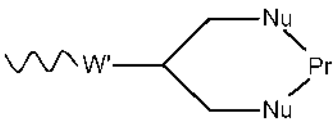

(Ia).

**8.** Conjugué selon l'une quelconque des revendications précédentes, où le lieur comporte un groupe qui est susceptible d'une dégradation enzymatique.

**9.** Conjugué selon la revendication 8, où le groupe susceptible d'une dégradation enzymatique est :

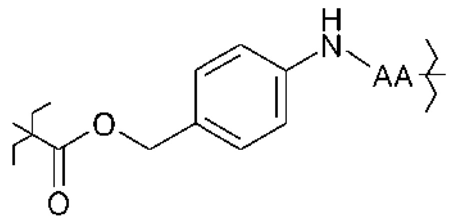

dans lequel AA représente une séquence d'acides aminés spécifique à la protéase.

**10.** Conjugué selon la revendication 9, où la séquence d'acides aminés comprend 1 à 5 acides aminés.

**11.** Réactif de conjugaison capable de réagir avec une protéine ou un peptide, et comportant un agent thérapeutique, ledit réactif ayant un lieur qui relie l'agent thérapeutique à un groupement fonctionnel, ledit lieur comportant une portion polyéthylène glycol ; ledit groupement fonctionnel étant de formule :

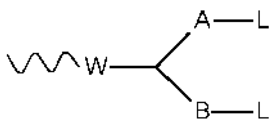

(II) or

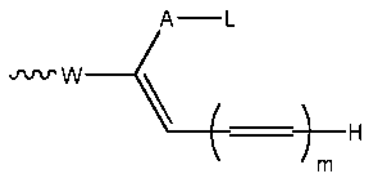

(II')

dans laquelle :

W représente un groupe céto ou un groupe CH.OH ;
chacun de A et B représente indépendamment une chaîne alkylène ou alcénylène en C1-4 ;
chaque L représente indépendamment un groupe partant ;
ladite portion polyéthylène glycol est ou comporte une chaîne polyéthylène glycol pendante qui a un groupe terminal de formule $-CH_2CH_2OR$, dans laquelle R représente un atome d'hydrogène, un groupe alkyle ou un groupe aryle éventuellement substitué, et où ladite chaîne polyéthylène glycol pendante a un poids moléculaire moyen en nombre allant jusqu'à 75 000 g/mole ;
le lieur comporte le groupement :

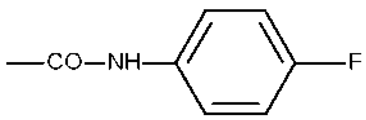

(VIa) or

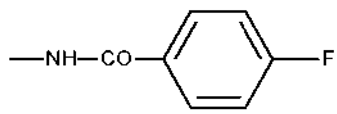

(VIb)

dans lequel F représente le groupement fonctionnel de formule II ou II'.

**12.** Réactif de conjugaison selon la revendication 11, dans lequel R représente un atome d'hydrogène ou un groupe alkyle en $C_{1-4}$.

**13.** Réactif de conjugaison selon la revendication 11 ou la revendication 12, dans lequel ladite chaîne polyéthylène glycol pendante contient 2 à 50 motifs polyéthylène glycol.

**14.** Réactif de conjugaison selon l'une quelconque des revendications 11 à 13, où l'agent thérapeutique est un agent cytotoxique ou une toxine.

**15.** Réactif de conjugaison selon l'une quelconque des revendications 11 à 14, dans lequel ledit groupement fonctionnel est de formule

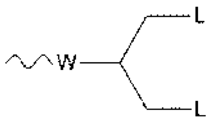

(IIa) or

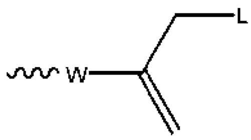

(II'a)

**16.** Réactif de conjugaison selon l'une quelconque des revendications 11 à 15, dans lequel W représente un groupe céto.

**17.** Réactif de conjugaison selon l'une quelconque des revendications 11 à 17, dans lequel chaque L représente -SP, -OP, $-SO_2P$, $-OSO_2P$, $-N^+PR^2R^3$, halogène, ou -Oϕ, dans lequel P représente un atome d'hydrogène ou un groupe alkyle, aryle, ou alkyl-aryle, ou est un groupe qui comporte une portion $-(CH_2CH_2O)_n-$ dans laquelle n est un nombre de deux ou plus, et chacun de $R^2$ et $R^3$ représente indépendamment un atome d'hydrogène, un groupe alkyle en $C_{1-4}$, ou un groupe P, et ϕ représente un groupe aryle substitué contenant au moins un substituant attracteur d'électrons.

**18.** Réactif de conjugaison selon l'une quelconque des revendications 11 à 17, dans lequel le lieur comporte un groupe susceptible d'une dégradation enzymatique.

**19.** Réactif de conjugaison selon la revendication 18, dans lequel le groupe susceptible d'une dégradation enzymatique est : dans lequel AA représente une séquence d'acides aminés spécifique à la protéase.

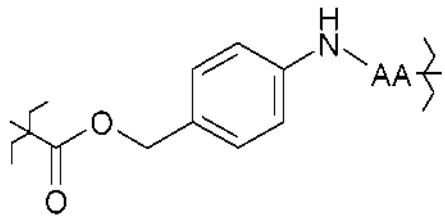

**20.** Procédé pour la préparation d'un conjugué selon l'une quelconque des revendications 1 à 10, qui comprend réaction d'un réactif de conjugaison tel que revendiqué dans l'une quelconque des revendications 11 à 19 avec une protéine ou un peptide.

Figure 1

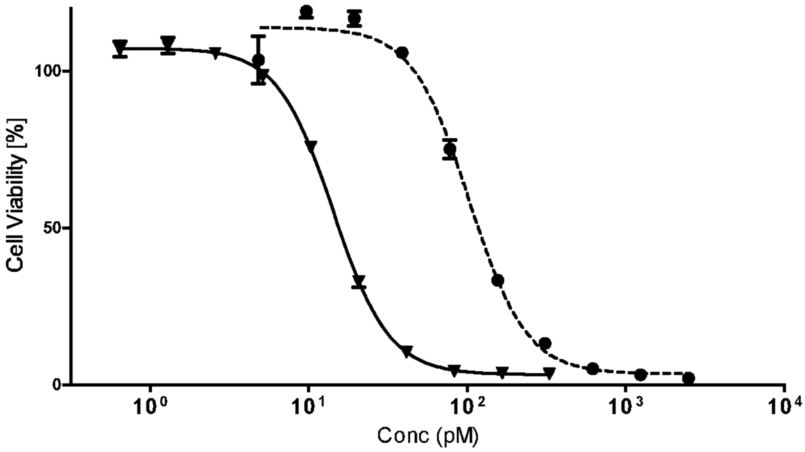


▼ Brentuximab - drug conjugate 10, DAR4
● MMAE

Figure 2

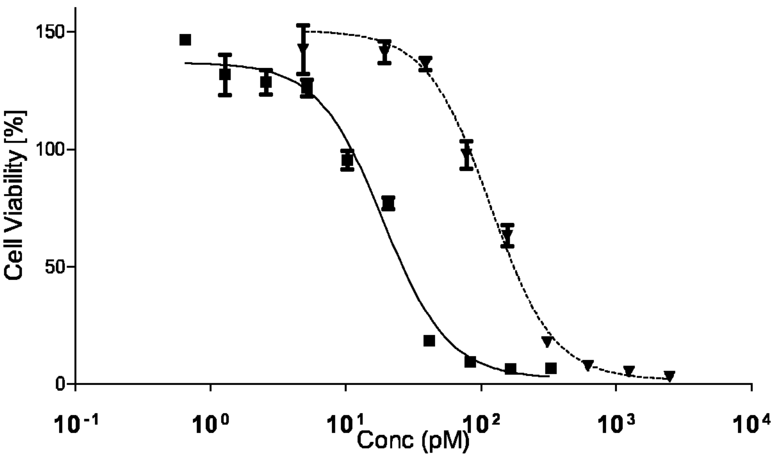


■ Brentuximab - drug conjugate 11, DAR4
▼ MMAE

Figure 3

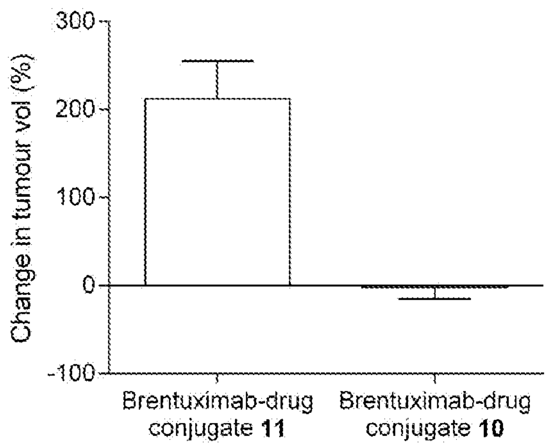


Figure 4a:

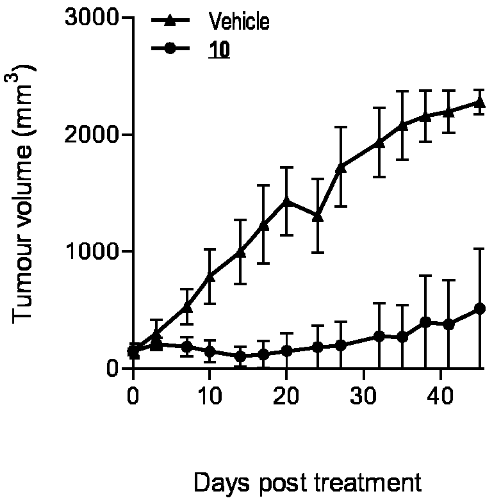

Figure 4b:

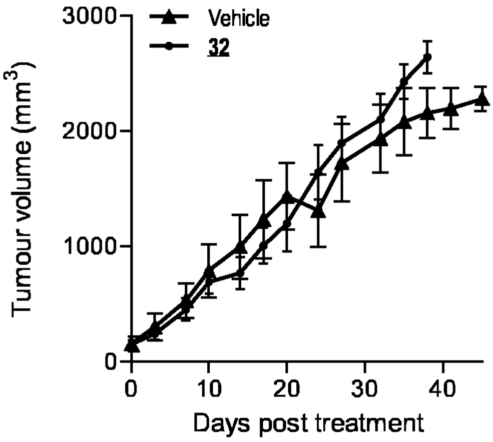


Figure 4c:

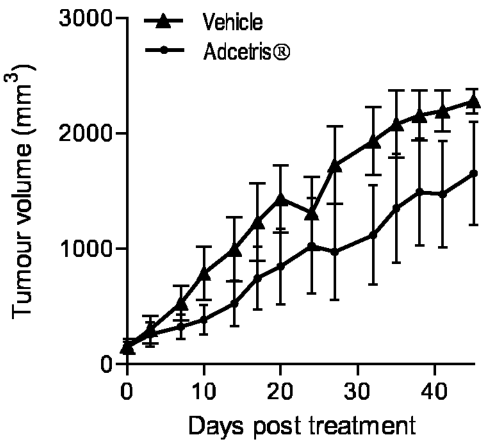

Figure 5a:

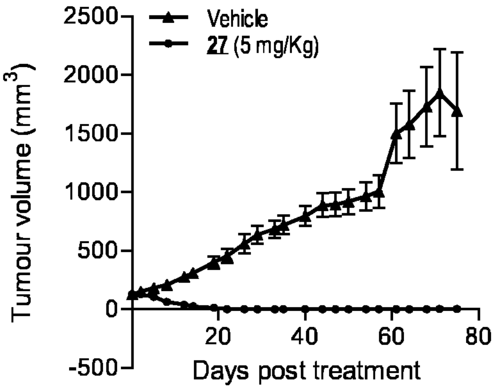


Figure 5b:

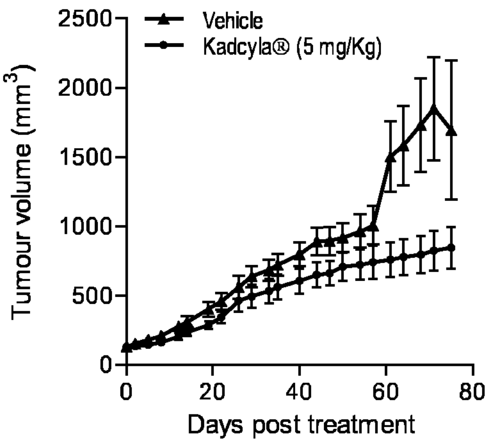

Figure 6a:

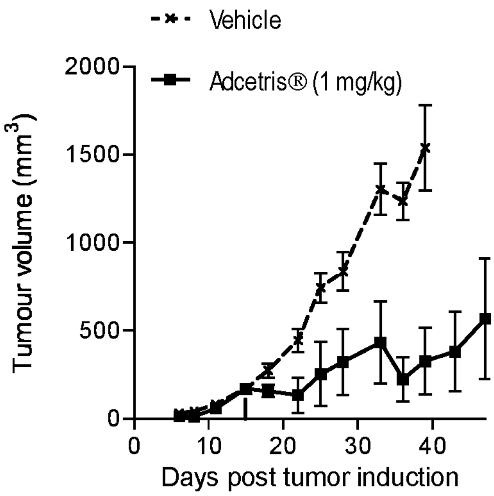


Figure 6b:

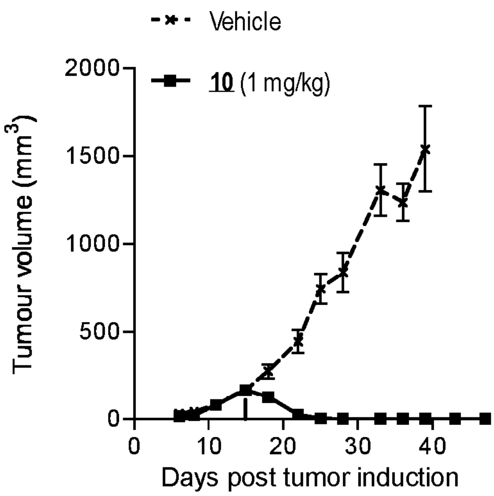

Figure 6c:

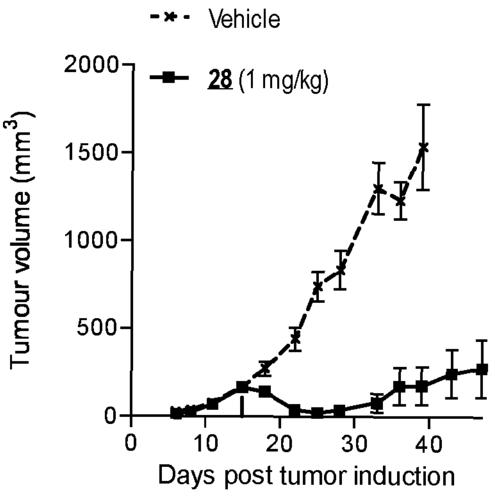


Figure 6d:

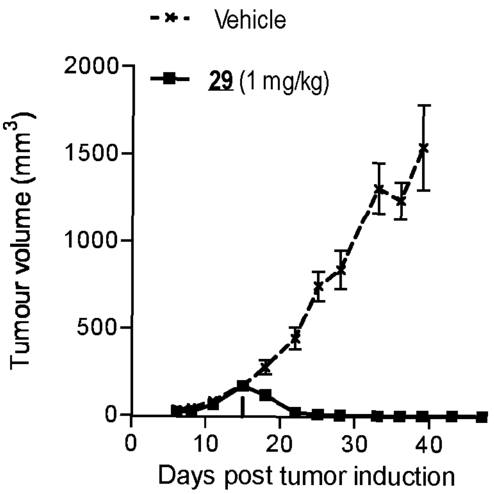

Figure 7a:

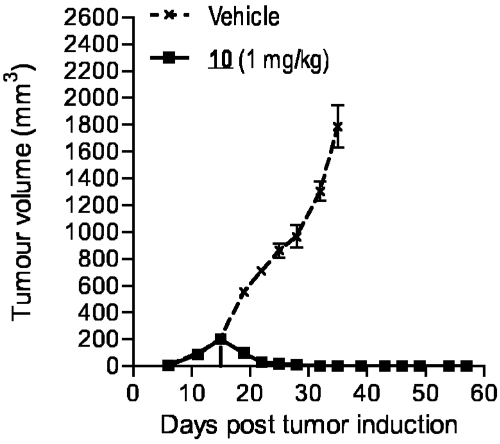


Figure 7b:

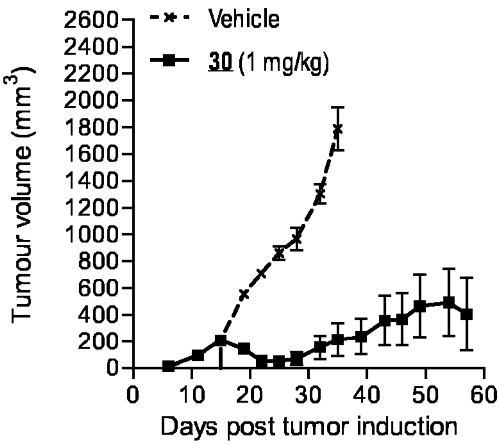

Figure 7c:

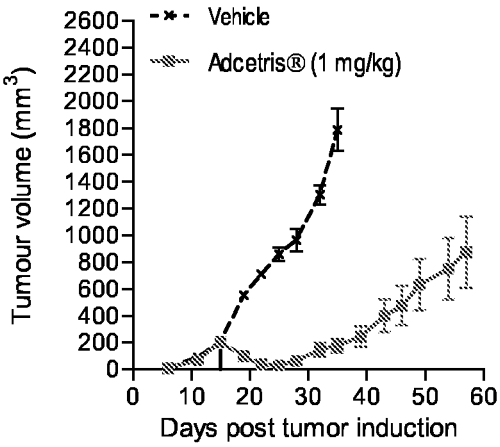


Figure 8a:

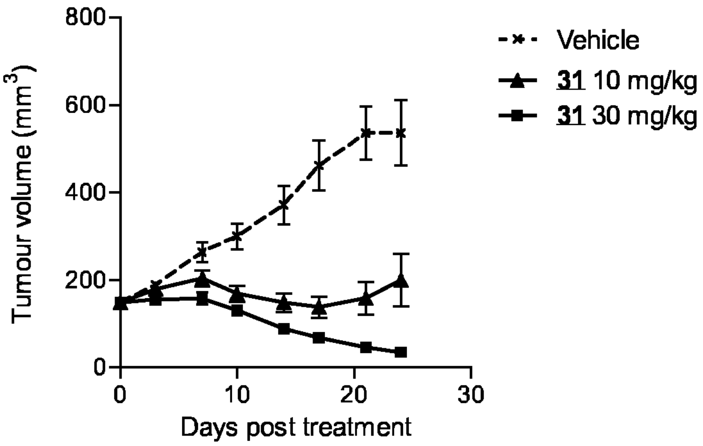

Figure 8b:

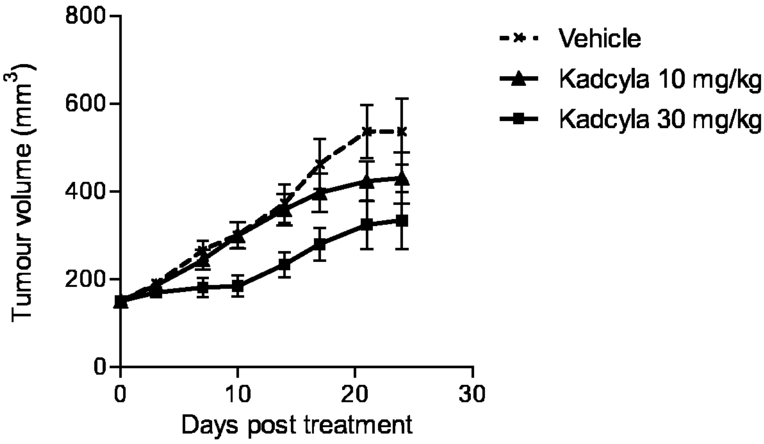


Figure 9a:

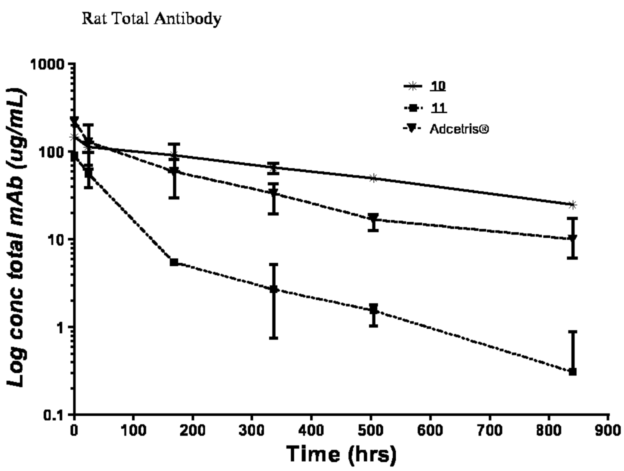

Figure 9b:

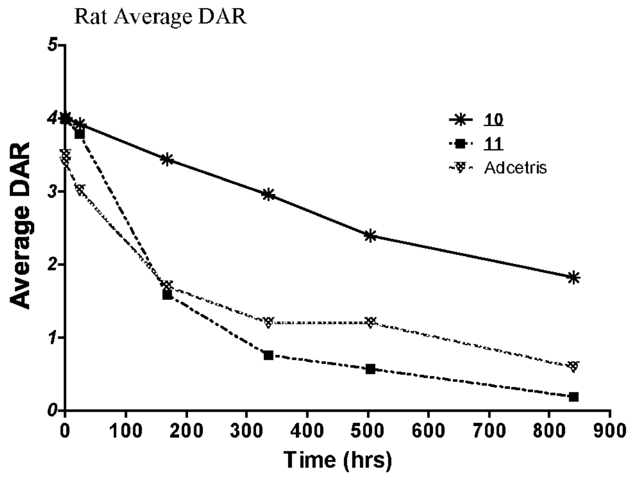


Figure 10:

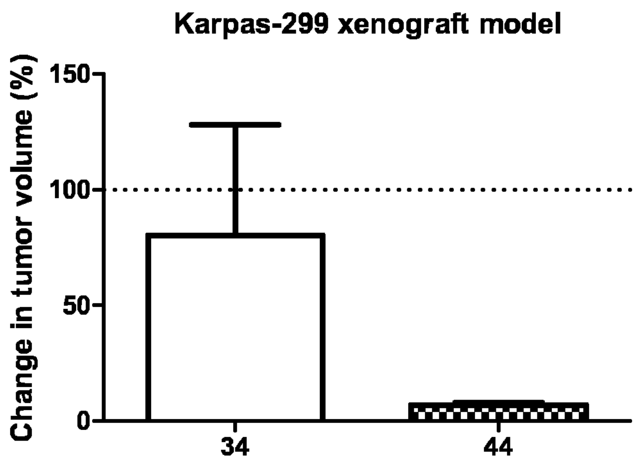

Figure 11

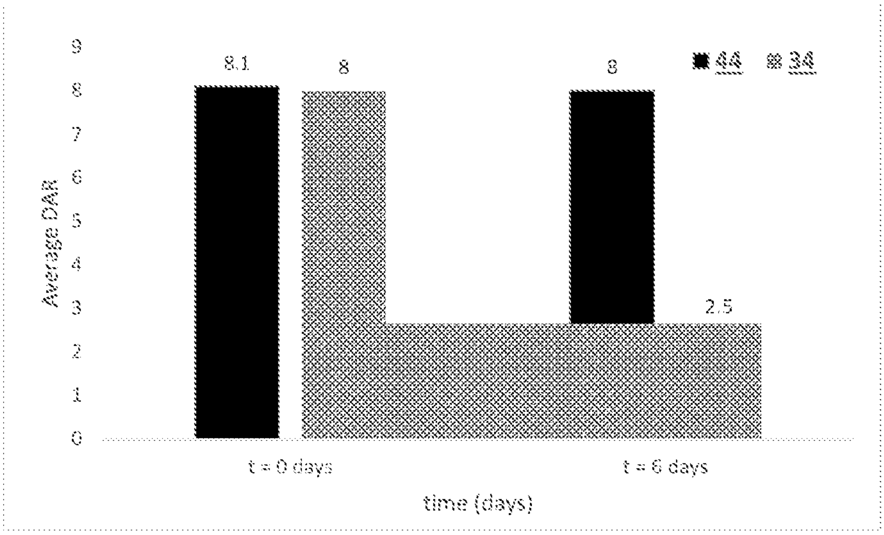
9
8
7
6
5
4
3
2
1
0
Average DAR
8.1
8
8
2.5
44
34
t = 0 days
t = 6 days
time (days)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004060965 A
- WO 2005007197 A
- WO 2009047500 A
- WO 2010000393 A
- WO 2014064423 A
- WO 2013190292 A
- WO 2013190272 A
- EP 2260873 A
- US 2013338231 A
- WO 2011077067 A
- WO 2014064424 A
- GB 1418984 A
- US 4179337 A
- GB 1418186 A
- EP 0624377 A2
- WO 2015057699 A
- US 7090843 B


### Non-patent literature cited in the description


- **LIBERATORE et al.** *Bioconj. Chem,* 1990, vol. 1, 36-50
- **DEL ROSARIO et al.** *Bioconj. Chem.,* 1990, vol. 1, 51-59
- **DREBORG et al.** *Crit. Rev. Therap. Drug Carrier Syst.,* 1990, vol. 6, 315-365
- *Nature Protocols,* 2006, vol. 1 (54), 2241-2252
- *Technical Bulletin TB288*
- **LEWIS PHILLIPS G.D.** *Cancer Res,* 2008, vol. 68, 9280-9290
- **R. IAN FRESHNEY.** Culture of Animal Cells: A Manual of Basic Technique. Alan R. Liss, 1994
- CULTURE OF ANIMAL CELLS:A MANUAL OF BASIC TECHNIGUE. Wiley-Liss, 2005
- **LYON et al.** *Nature Biotechnology,* 2015, vol. 33 (7), 733-736
- **LYON et al.** *Methods in Enzymology,* 2012, vol. 502, 123-137